# EUROPEAN PATENT SPECIFICATION

(11) **EP 4 231 964 B1**
(45) Date of publication and mention of the grant of the patent: **22.04.2026**
(21) Application number: 21807431.8
(22) Date of filing: 22.10.2021
(51) Int. Cl.: A61F 2/24

(54) **PROSTHETIC VALVE DOCKING DEVICE**
KLAPPENPROTHESENANDOCKVORRICHTUNG
DISPOSITIF D'ACCUEIL DE VALVE PROTHÉTIQUE

(30) Priority: 23.10.2020 US 202063105099 P; 10.03.2021 US 202163159130 P; 05.10.2021 US 202163252524 P
(43) Date of publication of application: 30.08.2023
(73) Proprietor: Edwards Lifesciences Corporation, Irvine, CA 92614-5688 (US)
(72) Inventor: CHAU, Jocelyn, Irvine, CA 92614 (US); NGUYEN, Tram Ngoc, Irvine, CA 92614 (US); PATEL, Darshin S., Irvine, CA 92614 (US)
(74) Representative: Eisenführ Speiser
(86) International application number: PCT/US2021/056150
(87) International publication number: WO 2022/087336

(56) References cited:
- WO-A1-2015/023579
- WO-A1-2020/247907
- US-A1- 2018 055 628
- US-A1- 2018 325 663

## Description

### CROSS-REFERENCE TO RELATED APPLICATIONS

This application claims the benefit of U.S. Provisional Application No. 63/252,524, filed October 5, 2021, U.S. Provisional Application No. 63/159,130, filed March 10, 2021, and U.S. Provisional Application No. 63/105,099, filed October 23, 2020.

### FIELD

The present disclosure concerns examples of a docking device configured to secure a prosthetic valve at a native heart valve, as well as methods of assembling such devices.

### BACKGROUND

Prosthetic valves can be used to treat cardiac valvular disorders. Native heart valves (e.g., the aortic, pulmonary, tricuspid and mitral valves) function to prevent backward flow or regurgitation, while allowing forward flow. These heart valves can be rendered less effective by congenital, inflammatory, infectious conditions, etc. Such conditions can eventually lead to serious cardiovascular compromise or death. For many years, the doctors attempted to treat such disorders with surgical repair or replacement of the valve during open heart surgery.

A transcatheter technique for introducing and implanting a prosthetic heart valve using a catheter in a manner that is less invasive than open heart surgery can reduce complications associated with open heart surgery. In this technique, a prosthetic valve can be mounted in a compressed state on the end portion of a catheter and advanced through a blood vessel of the patient until the valve reaches the implantation site. The valve at the catheter tip can then be expanded to its functional size at the site of the defective native valve, such as by inflating a balloon on which the valve is mounted or, for example, the valve can have a resilient, self-expanding stent or frame that expands the valve to its functional size when it is advanced from a delivery sheath at the distal end of the catheter. Optionally, the valve can have a balloon-expandable, self-expanding, mechanically expandable frame, and/or a frame expandable in multiple or a combination of ways.

In some instances, a transcatheter heart valve (THV) may be appropriately sized to be placed inside a particular native valve (e.g., a native aortic valve). As such, the THV may not be suitable for implantation at another native valve (e.g., a native mitral valve) and/or in a patient with a larger native valve. Additionally or alternatively, the native tissue at the implantation site may not provide sufficient structure for the THV to be secured in place relative to the native tissue. Accordingly, improvements to THVs and the associated transcatheter delivery apparatus are desirable.

US Patent application US 2018/055628 A1 relates to anchoring or docking devices configured to be positioned at a native valve of a human heart and to provide structural support for docking a prosthetic valve therein. The docking devices can have coiled structures that define an inner space in which the prosthetic valve can be held. The docking devices can have enlarged end regions with circular or non-circular shapes, for example, to facilitate implantation of the docking device or to better hold the docking device in position once deployed. The docking devices can be laser-cut tubes with locking wires to assist in better maintaining a shape of the docking device. The docking devices can include various features to promote friction, such as frictional cover layers. Such docking devices can have ends configured to more securely attach the cover layers to cores of the docking devices.

### SUMMARY

The present disclosure relates to methods and devices for treating valvular regurgitation and/or other valve issues. Specifically, the present disclosure is directed to a docking device configured to receive a prosthetic valve and the methods of assembling the docking device and implanting the docking device. The invention relates to a device as claimed in claims 1 and 15.

Certain examples of the disclosure concern a docking device for securing a prosthetic valve at a native valve. The docking device includes a coil comprising a proximal end and a distal end, a first cover surrounding at least a portion of the coil, and a guard member surrounding at least a portion of the first cover. The guard member includes an expandable member and a second cover surrounding an outer surface of the expandable member. A distal end portion of the guard member is fixedly attached to the first cover. A proximal end portion of the guard member is movable relative to the first cover and the coil. The guard member is movable between a radially compressed state and a radially expanded state.
The proximal end portion of the guard member is disposed closer to the proximal end of the coil when the guard member is in the radially compressed state than in the radially expanded state.

According to certain examples, a docking device for securing a prosthetic valve at a native valve includes a coil comprising a plurality of helical turns when deployed at the native valve, an expandable member extending radially outwardly from the coil, and a cover member surrounding an outer surface of the expandable member. The expandable member is movable between a radially-compressed/axially-elongated state and a radially-expanded/axially-foreshortened state. Both a distal end portion of the cover member and a distal end portion of the expandable member are fixedly coupled to the coil via a distal suture. The distal suture includes a plurality of knots and a plurality of wraps. A proximal end portion of the expandable member is fixedly coupled to a proximal end portion of the cover member. The proximal end portion of the expandable member and the proximal end portion of the cover member are axially movable relative to the coil.

Certain examples of the disclosure concern a cover assembly for a docking device configured to receive a prosthetic valve. The cover assembly includes a first cover configured to cover at least a portion of a helical coil of the docking device, an expandable member surrounding at least a portion of the first cover, and a second cover surrounding an outer surface of the expandable member. The expandable member is changeable between a radially expanded state and a radially compressed state. A distal end portion of the second cover is fixedly coupled to a distal end portion of the expandable member and the first cover. A proximal end portion of second cover includes a fold covering a proximal end of the expandable member. The proximal end of the expandable member is slidably movable relative to the first cover and the helical coil of the docking device.

Certain examples of the disclosure also concern an implant assembly. The implant assembly can include a radially expandable and compressible prosthetic valve, and a docking device configured to receive the prosthetic valve. The docking device can include a coil configured to surround native tissue when deployed at an implant position. The prosthetic valve can be configured to be radially expandable within the coil. The docking device can also include a guard member covering at least a portion of the coil. The guard member can be configured to reduce paravalvular leakage. The guard member can include an expandable member and a cover member surrounding an outer surface of the expandable member. The expandable member can extend radially outwardly from the coil and is movable between a radially compressed state and a radially expanded state. A distal end portion of the cover member and a distal end portion of the expandable member can be fixedly coupled to the coil via a distal suture. A proximal end portion of the expandable member can be fixedly coupled to a proximal end portion of the cover member via a proximal suture. The proximal end portion of the expandable member can be axially movable relative to the coil.

Certain examples of the disclosure also concern a method for assembling a cover assembly for a docking device configured to receive a prosthetic valve. The method can include positioning a cover member within a lumen of an expandable member, securing a proximal end portion of the cover member to a proximal end of the expandable member, removing a distal end portion of the cover member out of the expandable member through the proximal end of the expandable member, covering an outer surface of the expandable member with the cover member, securing the distal end portion of the expandable member to a tubular member of the docking device, and securing the distal end portion of the cover member to the expandable member and the tubular member. The tubular member can be configured to surround at least a portion of a docking device.

According to certain examples, a method for assembling a cover assembly for a docking device configured to receive a prosthetic valve can include securing a proximal end portion of an outer cover to a proximal end of an expandable member, inverting the outer cover from inside the expandable member to outside the expandable member, folding the outer cover at the proximal end of the expandable member, and securing a distal end portion of the expandable member and a distal end portion of the outer cover to an inner cover of the docking device.

According to certain examples, a method for assembling a docking device configured to receive a prosthetic valve can include securing a proximal end portion of a cover member to a proximal end of an expandable member, folding the proximal end portion of the cover member at the proximal end of the expandable member, covering an outer surface of the expandable member with the cover member, and securing a distal end portion of the expandable member and a distal end portion of the cover member to the docking device.

According to certain examples, a method for assembling a docking device configured to receive a prosthetic valve can include attaching a proximal end portion of a cover member to a proximal end of an expandable member, attaching a distal end portion of the cover member to a distal end portion of the expandable member, and securing the distal end portion of the cover member and the distal end portion of the expandable member to the docking device through a plurality of knots, a plurality of wraps, and one or more locking stitches. Each of the locking stitches can extend through the plurality of wraps and/or the plurality of knots.

Certain examples of the disclosure also concern a method for implanting a prosthetic valve. The method can include deploying a docking device at a native valve, wherein the docking device can include a coil and a guard member, wherein the coil deployed at the native valve can include a stabilization turn and one or more functional turns distal to the stabilization turn, wherein the guard member can cover at least a portion of the stabilization turn; and deploying the prosthetic valve within the docking device. Deploying the docking device at the native valve can include wrapping around leaflets of the native valve with the one or more functional turns of the coil and resting the stabilization turn of the coil against a native wall around the native valve. Deploying the prosthetic valve can include placing the prosthetic valve in a radially compressed state within the one or more functional turns of the coil and radially expanding the prosthetic valve to a radially expanded state, wherein radially expanding the prosthetic valve can cause radial expansion of the one or more functional turns of the coil.

According to certain examples, a docking device for securing a prosthetic valve at a native valve can include a coil configured to surround native tissue when deployed at the native valve and a tubular member surrounding at least a portion of the coil. The tubular member can include at least a first seating marker and a second seating marker. The first seating marker can be positioned proximal relative to the second seating marker. The docking device can also include a retention element surrounding at least a portion of the tubular member, and a guard member surrounding at least a portion of the retention element and configured to reduce paravalvular leakage. A proximal end of the guard member can be axially movable relative to the coil. When deployed at the native valve, the proximal end of the guard member can be positioned between the first seating marker and the second seating marker.

According to certain examples, a docking device for securing a prosthetic valve at a native valve can include a coil configured to have a helical configuration when deployed at the native valve. The coil in the helical configuration includes a stabilization turn, one or more functional turns, and an ascending portion located between the stabilization turn and the one or more functional turns. The docking device can also include at least a first seating marker and a second seating marker disposed distal to the ascending portion. The first seating marker can be positioned proximal relative to the second seating marker. The docking device can further include a guard member surrounding at least a portion of the coil and configured to reduce paravalvular leakage. A proximal end of the guard member can be axially movable relative to the coil. When deployed at the native valve, the proximal end of the guard member can be positioned between the first seating marker and the second seating marker.

According to certain examples, a docking device for securing a prosthetic valve at a native valve can include a coil configured to surround native tissue when deployed at the native valve, a radiopaque marker disposed at a predefined location on the coil, and a guard member surrounding at least a portion of the coil and configured to reduce paravalvular leakage. A proximal end of the guard member can be axially movable relative to the coil. When deployed at the native valve, the proximal end of the guard member can be positioned distal to the radiopaque marker.

According to certain examples, a cover assembly for a docking device configured to receive a prosthetic valve can include a retention element configured to surround at least a portion of a coil of the docking device, and a guard member surrounding at least a portion of the retention element and configured to reduce paravalvular leakage. A radiopaque marker can be positioned at a proximal end portion of the retention element. A proximal end of the guard member can be configured to be axially movable relative to the retention element. When deployed at a native valve, the proximal end of the guard member can be positioned distal to the radiopaque marker.

Certain examples of the disclosure also concern an implant assembly. The implant assembly can include a radially expandable and compressible prosthetic valve and a docking device configured to receive the prosthetic valve. The docking device can include a coil configured to surround native tissue when deployed at a native valve, a radiopaque marker positioned at a proximal portion of the coil, a retention element surrounding at least a portion of the coil, and a guard member surrounding at least a portion of the retention element and configured to reduce paravalvular leakage. A proximal end of the guard member can be axially movable relative to the retention element. When deployed at the native valve, the proximal end of the guard member can be positioned distally relative to the radiopaque marker.

Certain examples of the disclosure also concern a method for assembling a cover assembly for a docking device configured to receive a prosthetic valve. The method can include inserting a tubular member through a lumen of a retention element, securing a retention element to the tubular member, and securing a distal end of a guard member to a distal portion of the tubular member. At least one radiopaque marker can be disposed at a proximal portion of the tubular member. A proximal end of the guard member can be axially movable relative to the retention element. The guard member can be movable between a radially expanded state and a radially compressed state. When the guard member is in the radially expanded state, a proximal end of the guard member can be positioned distal to the radiopaque marker.

Certain examples of the disclosure also concern a method for implanting a prosthetic valve. The method can include deploying a docking device at a native valve, wherein the docking device deployed at the native valve comprises a coil and a guard member surrounding at least a portion of the coil; positioning a proximal end of the guard member distal to a predefined location on the coil; and deploying the prosthetic valve within the docking device.

Also disclosed herein is another example of docking device for securing a prosthetic valve at a native valve. The docking device can include a coil configured to surround native tissue when deployed at the native valve, a retention element surrounding at least a portion of the coil, and a guard member surrounding at least a portion of the retention element and configured to reduce paravalvular leakage. A proximal end of the guard member can be axially movable relative to the retention element. An inner surface of the guard member can be configured to frictionally engage with the retention element so that the retention element frictionally impedes the proximal end of the guard member to move axially relative to the coil.

According to certain examples, a docking device for securing a prosthetic valve at a native valve can include a coil configured to surround native tissue when deployed at the native valve and a sealing member having an inner edge coupled the coil and an outer edge that is movable between a folded position and an extended position. The outer edge in the folded position can extend along and adjacent to the coil. At least a segment of the outer edge in the extended position can be spaced apart from the coil.

According to certain examples, a docking device for securing a prosthetic valve at a native valve can include a coil configured to surround native tissue when deployed at the native valve and a skirt coupled to the coil, wherein the skirt is movable between a delivery configuration and a deployed configuration. When the skirt is in the delivery configuration, an outer edge of the skirt can extend along and adjacent the coil. When the skirt is in the deployed configuration, at least a segment of the outer edge of the sealing member can extend radially away from the coil so as to reduce paravalvular leakage.

According to certain examples, a docking device for securing a prosthetic valve at a native valve can include a coil configured to surround native tissue when deployed at the native valve and a skirt coupled to the coil. The skirt can be movable between a first configuration and a second configuration. The skirt can be folded along the coil when the skirt is in the first configuration. The skirt can be flat or substantially flat and extend radially from the coil when the skirt is in the second configuration.

Certain examples of the disclosure concern a device for reducing paravalvular leakage between a prosthetic valve received in a docking device and native tissue surrounding the prosthetic valve. The device can include a first edge fixedly attached to a coil of the docking device and a second edge that is movable between a folded position and an extended position. The second edge in the folded position can extend along and adjacent to the coil. The second edge in the extended position can radially fan out from the coil.

Certain examples of the disclosure concern a method for assembling a docking device configured to receive a prosthetic valve. The method can include attaching a skirt to a coil. The coil can be configured to surround native tissue when deployed at a native valve. An outer edge of the skirt can be movable between a folded position and an extended position. The outer edge in the folded position can extend along and adjacent to the coil. The outer edge in the extended position can radially fan out from the coil.

Certain examples of the disclosure concern a method for implanting a prosthetic valve. The method can include deploying a docking device at a native valve and deploying the prosthetic valve within the docking device. The docking device deployed at the native valve can include a coil and a skirt extending radially outwardly from the coil. The skirt can have a planar or generally planar surface that forms an oblique angle relative to a central longitudinal axis of the docking device.

Certain examples of the disclosure concern a medical assembly including a docking device described above and a radially expandable and compressible prosthetic valve configured to be received within the docking device.

Certain examples of the disclosure concern a medical assembly including a docking device described above and a delivery apparatus configured to deliver the docking device to a target implantation site of a patient.

Certain examples of the disclosure concern a medical assembly including an implantable device having a frame and a skirt coupled to a sealing segment of the frame. When the implantable device is deployed at a target implantation site, an outer edge of the skirt can radially fan out from the sealing segment of the frame, and the sealing segment of the frame can form a helical shape rotating about a central longitudinal axis of the frame such that a proximal end of the sealing segment has an offset relative to a distal end of the sealing segment along the central longitudinal axis of the frame.

Certain examples of the disclosure concern a docking device for securing a prosthetic valve at a native valve. The docking device can include a coil configured to surround native tissue when deployed at the native valve and a paravalvular leakage guard connected to the coil. The paravalvular leakage guard can be movable between a delivery configuration and a deployed configuration. When the paravalvular leakage guard is in the delivery configuration, an outer edge of the paravalvular leakage guard can extend along and adjacent the coil. When the paravalvular leakage guard is in the deployed configuration, the outer edge of the paravalvular leakage guard can form a helical shape rotating about a central longitudinal axis of the coil and at least a segment of the outer edge of paravalvular leakage guard can extend radially away from the coil.

The foregoing and other objects, features, and advantages of the disclosed technology will become more apparent from the following detailed description, which proceeds with reference to the accompanying figures.

### BRIEF DESCRIPTION OF THE DRAWINGS

FIG. 1A is a side perspective view of a docking device in a helical configuration, according to one example.
FIG. 1B is a top view of the docking device depicted in FIG. 1A.
FIG. 1C is a cross-sectional view of the docking device taken along line 1C-1C depicted in FIG. 1B, according to one example.
FIG. 1D is a cross-sectional view of the docking device taken along the same line as in FIG. 1C, except in FIG. 1D, the docking device is in a substantially straight delivery configuration.
FIG. 1E is a cross-sectional view of the docking device taken along line 1C-1C depicted in FIG. 1B, according to another example.
FIG. 1F is a cross-sectional view of the docking device taken along the same line as in FIG. 1E, except in FIG. 1F, the docking device is in a substantially straight delivery configuration.
FIG. 1G is a schematic diagram depicting the docking device in a substantially straight configuration.
FIG. 2A is a perspective view a prosthetic valve, according to one example.
FIG. 2B is a perspective view of the prosthetic valve of FIG. 2A with an outer cover, according to one example.
FIG. 3A is a perspective view of an exemplary prosthetic implant assembly comprising the docking device depicted in FIG. 1A and the prosthetic valve of FIG. 2B retained within the docking device.
FIG. 3B is a side elevation view of the prosthetic implant assembly of FIG. 3.
FIGS. 4-42 depict various detail views of the docking device, illustrating an exemplary method of attaching a paravalvular leakage guard to the docking device, according to one example.
FIGS. 43-59 illustrate an exemplary method of attaching a retention element to a tubular member to form a part of a cover assembly, according to one example.
FIG. 60 is a side view of a delivery assembly comprising a delivery apparatus and the docking device of FIG. 1A, according to one example.
FIG. 61A is a side cross-sectional view of a sleeve shaft, according to one example.
FIG. 61B is a side cross-sectional view of a pusher shaft, according to one example.
FIG. 62A is a side cross-sectional view of an assembly comprising the sleeve shaft of FIG. 61A, the pusher shaft of FIG. 61B, and a delivery sheath, wherein the sleeve shaft covers a docking device.
FIG. 62B is a side cross-sectional view of the same assembly of FIG. 62A, except the docking device is uncovered by the sleeve shaft.
FIG. 63 is a schematic cross-sectional view of a distal end portion of a delivery system, showing fluid flow through lumens within the delivery system.
FIG. 64A illustrates a perspective view of an example of a sleeve shaft covering a docking device and extending outside of a delivery sheath of a delivery system.
FIG. 64B illustrates the sleeve shaft surrounding a pusher shaft after deploying the docking device from the delivery system of FIG. 64A and removing the sleeve shaft from the docking device.
FIGS. 65-78 depict various portions of an exemplary implantation procedure in which the delivery apparatus of FIG. 60 is being used to implant the prosthetic implant assembly of FIG. 3A at a native mitral valve location using a transseptal delivery approach.
FIGS. 79A is a top perspective view of another docking device having a foldable PVL guard in a deployed configuration, according to one example.
FIG. 79B is a top perspective view of the docking device depicted in FIG. 79A.
FIG. 79C is a bottom perspective view of the docking device depicted in FIG. 79A.
FIG. 79D is a cross-sectional view of a sealing member of the docking device and depicts an example measurement of flatness of the sealing member.
FIG. 80A is a perspective view of an example sleeve shaft covering the docking device of FIG. 79A and a sealing member of the docking device being in a delivery configuration.
FIG. 80B depicts the sleeve shaft is partially removed from the docking device and a part of the sealing member is exposed and radially expanded.
FIG. 81 is a top view of a docking device, according to another example.
FIG. 82A is a top view of a docking device, according to another example.
FIG. 82B is a cross-sectional view of the docking device of FIG. 82A.
FIG. 83 is a top view of a docking device, according to another example.
FIG. 84 is an atrial side view of a docking device implanted in the mitral valve, according to one example.
FIG. 85 is an atrial side view of the docking device of FIG. 84 after a prosthetic valve is received within the docking device, according to one example.

### DETAILED DESCRIPTION

### General Considerations

It should be understood that the disclosed examples can be adapted to deliver and implant prosthetic devices in any of the native annuluses of the heart (e.g., the pulmonary, mitral, and tricuspid annuluses), and can be used with any of various delivery approaches (e.g., retrograde, antegrade, transseptal, transventricular, transatrial, etc.).

For purposes of this description, certain aspects, advantages, and novel features of the examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved. The technologies from any example can be combined with the technologies described in any one or more of the other examples. In view of the many possible examples to which the principles of the disclosed technology may be applied, it should be recognized that the illustrated examples are only preferred examples and should not be taken as limiting the scope of the disclosed technology.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the terms "coupled" and "connected" generally mean electrically, electromagnetically, and/or physically (e.g., mechanically or chemically) coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

As used herein, the term "approximately" and "about" means the listed value and any value that is within 10% of the listed value. For example, "about 1 mm" means any value between about 0.9 mm and about 1.1 mm, inclusive.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated examples. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

### Introduction to the Disclosed Technology

Disclosed herein are various systems, apparatuses, methods, etc., including anchoring or docking devices, which can be used in conjunction with expandable prosthetic valves at a native valve annulus (e.g., a native mitral and/or tricuspid valve annulus), in order to more securely implant and hold the prosthetic valve at the implant site. Anchoring/docking devices according to examples of the disclosure can, for example, provide a stable anchoring site, landing zone, or implantation zone at the implant site in which prosthetic valves can be expanded or otherwise implanted. Many of the disclosed docking devices comprise a circular or cylindrically-shaped portion, which can (for example) allow a prosthetic heart valve comprising a circular or cylindrically-shaped valve frame or stent to be expanded or otherwise implanted into native locations with naturally circular cross-sectional profiles and/or in native locations with naturally with non-circular cross sections. In addition to providing an anchoring site for the prosthetic valve, the anchoring/docking devices can be sized and shaped to cinch or draw the native valve (e.g., mitral, tricuspid, etc.) anatomy radially inwards. In this manner, one of the main causes of valve regurgitation (e.g., functional mitral regurgitation), specifically enlargement of the heart (e.g., enlargement of the left ventricle, etc.) and/or valve annulus, and consequent stretching out of the native valve (e.g., mitral, etc.) annulus, can be at least partially offset or counteracted. Some examples of the anchoring or docking devices further include features which, for example, are shaped and/or modified to better hold a position or shape of the docking device during and/or after expansion of a prosthetic valve therein. By providing such anchoring or docking devices, replacement valves can be more securely implanted and held at various valve annuluses, including at the mitral valve annulus which does not have a naturally circular cross-section.

In some instances, a docking device can comprise a paravalvular leakage (PVL) guard (also referred to herein as "a guard member"). The PVL guard can, for example, help reduce regurgitation and/or promote tissue ingrowth between the native tissue and the docking device.

The PVL guard can, in some examples, be movable between a delivery configuration and a deployed configuration. When the PVL guard is in the delivery configuration, an outer edge of the PVL guard can extend along and adjacent the coil. When the PVL guard is in the deployed configuration, the outer edge of the PVL guard can form a helical shape rotating about a central longitudinal axis of the coil and at least a segment of the outer edge of PVL guard can extend radially away from the coil.

In certain examples, the PVL guard can cover or surround a portion of a coil of the docking device. As described more fully below, such PVL guard can move from a radially compressed (and axially elongated) state to a radially expanded (and axially foreshortened) state, and a proximal end portion of the PVL guard can be axially movable relative to the coil.

In other examples, the PVL guard can be foldable along a segment of a coil of the docking device. As described more fully below, such PVL guard can have an inner edge coupled the coil and an outer edge that is movable between a folded position and an extended position. The outer edge in the folded position can extend along and adjacent to the coil, and at least a segment of the outer edge in the extended position can be spaced apart from the coil.

Exemplary methods of attaching the PVL guard to the docking device and example methods of limiting axial movement of the PVL guard are also disclosed herein.

### Exemplary Docking Devices

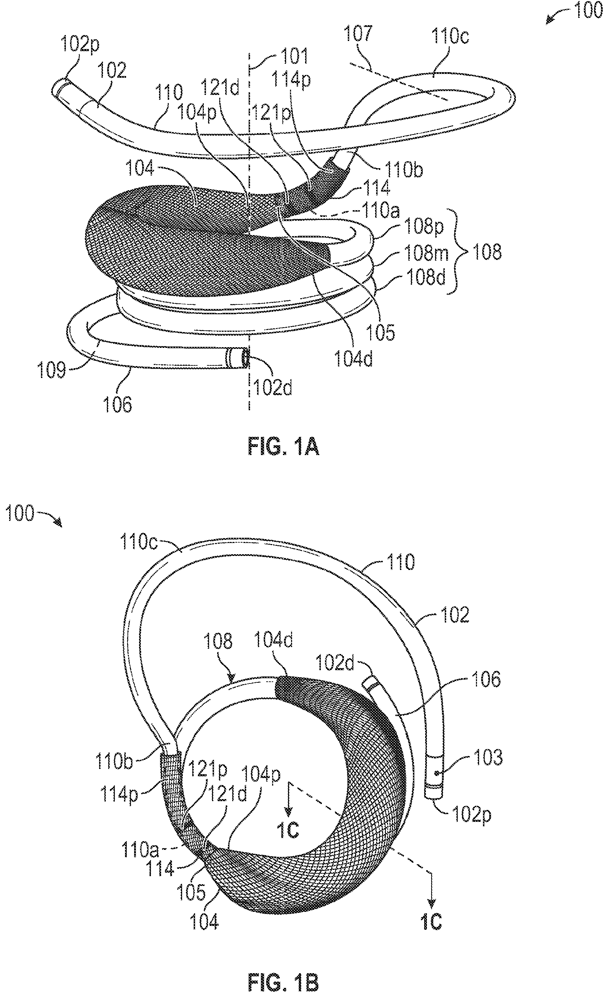

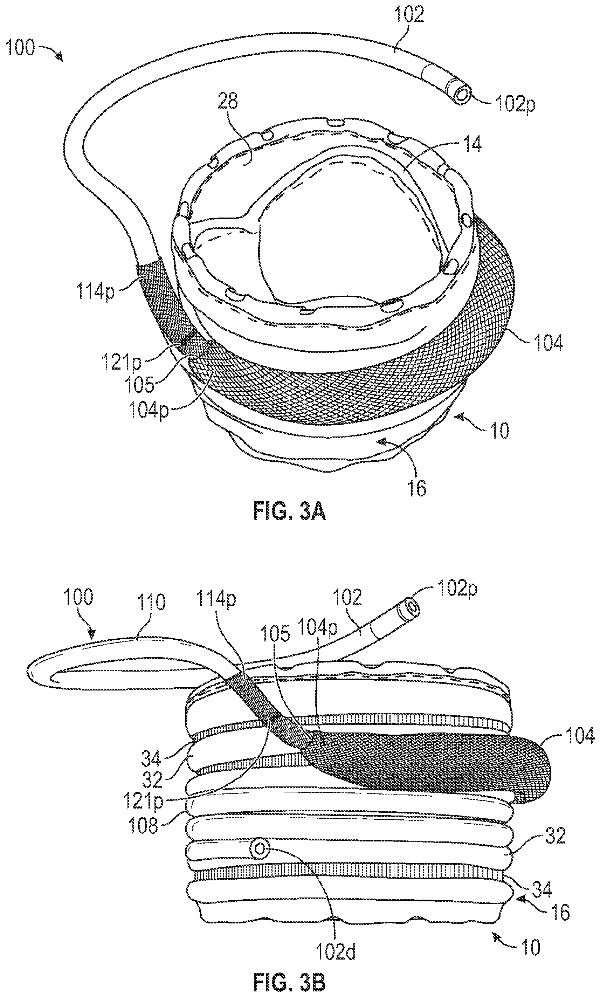

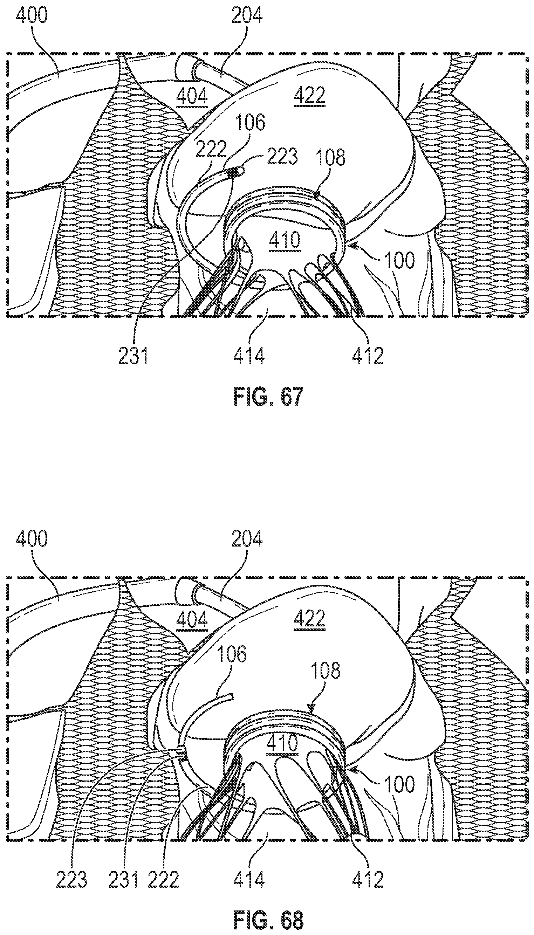

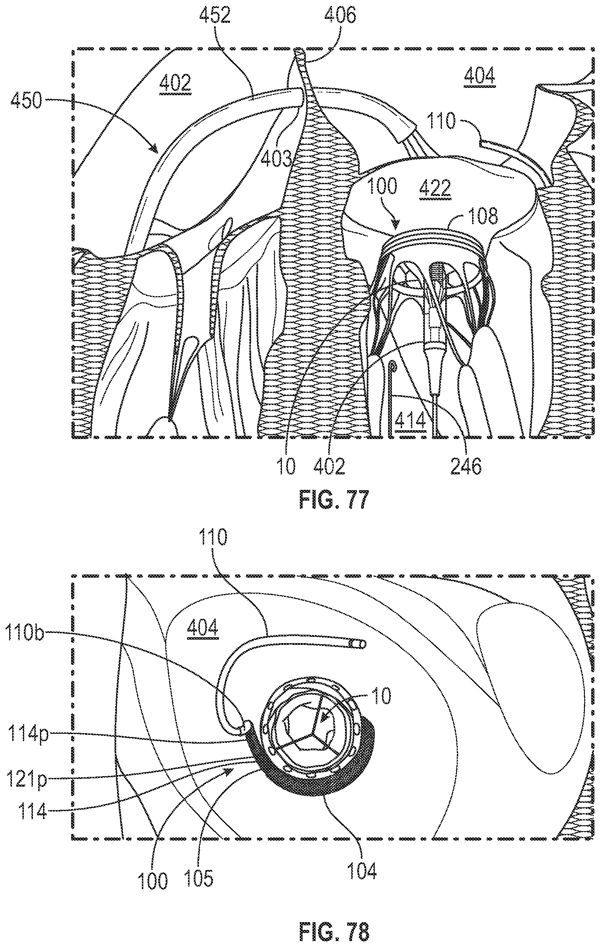

FIGS. 1A-1G show a docking device 100, according to one example. The docking device 100 can, for example, be implanted within a native valve annulus (see, e.g., FIG. 67). As depicted in FIGS. 3A-3B and 78, the docking device can be configured to receive and secure a prosthetic valve within the docking device, thereby securing the prosthetic valve at the native valve annulus.

Referring to FIGS. 1A-1G, the docking device 100 can comprise a coil 102 and a guard member 104 covering at least a portion of the coil 102. In certain examples, the coil 102 can include a shape memory material (e.g., nickel titanium alloy or "Nitinol") such that the docking device 100 (and the coil 102) can move from a substantially straight configuration (also referred to as "delivery configuration") when disposed within a delivery sheath of a delivery apparatus (as described more fully below) to a helical configuration (also referred to as "deployed configuration," as shown in FIGS. 1A-1B) after being removed from the delivery sheath.

In certain examples, when the guard member 104 is in the deployed configuration, the guard member 104 can extend circumferentially relative to a central longitudinal axis 101 of the docking device 100 from 180 degrees to 400 degrees, or from 210 degrees to 330 degrees, or from 250 degrees to 290 degrees, or from 260 degrees to 280 degrees. In one particular example, when the guard member 104 is in the deployed configuration, the guard member 104 can extend circumferentially 270 degrees relative to the central longitudinal axis 101. In other words, the guard member 104 can extend circumferentially from about one half of a revolution (e.g., 180 degrees) around the central longitudinal axis 101 in some examples to more than a full revolution (e.g., 400 degrees) around the central longitudinal axis 101 in other examples, including various ranges in between. As used herein, a range (e.g., 180-400 degrees, from 180 degrees to 400 degrees, and between 180 degrees and 400 degrees) includes the endpoints of the range (e.g., 180 degrees and 400 degrees).

In some examples, the docking device 100 can also include a retention element 114 surrounding at least a portion of the coil 102 and at least being partially covered by the guard member 104. In some instances, the retention element 114 can comprise a braided material. In addition, the retention element 114 can provide a surface area that encourages or promotes tissue ingrowth and/or adherence, and/or reduce trauma to native tissue. For example, in certain instances, the retention element 114 can have a textured outer surface configured to promote tissue ingrowth. In certain instances, the retention element 114 can be impregnated with growth factors to stimulate or promote tissue ingrowth.

In one example, as illustrated in FIGS. 1A-1B and 3A-3B, at least a proximal end portion of the retention element 114 can extend out of a proximal end of the guard member 104. In another example, the retention element 114 can be completely covered by the guard member 104.

As described further below, the retention element 114 can be designed to interact with the guard member 104 to limit or resist motion of the guard member 104 relative to the coil 102. For example, a proximal end 105 of the guard member 104 can have an inner diameter that is about the same as an outer diameter of the retention element 114. As such, an inner surface of the guard member 104 at the proximal end 105 can frictionally interact or engage with the retention element 114 so that axial movement of the proximal end 105 of the guard member 104 relative to the coil 102 can be impeded by a frictional force exerted by the retention element 114.

The coil 102 has a proximal end 102p and a distal end 102d (which also respectively define the proximal and distal ends of the docking device 100). When being disposed within the delivery sheath (e.g., during delivery of the docking device into the vasculature of a patient), a body of the coil 102 between the proximal end 102p and distal end 102d can form a generally straight delivery configuration (i.e., without any coiled or looped portions, but can be flexed or bent) so as to maintain a small radial profile when moving through a patient's vasculature. After being removed from the delivery sheath and deployed at an implant position, the coil 102 can move from the delivery configuration to the helical deployed configuration and wrap around native tissue adjacent the implant position. For example, when implanting the docking device at the location of a native valve, the coil 102 can be configured to surround native leaflets of the native valve (and the chordae tendineae that connects native leaflets to adjacent papillary muscles, if present), as described further below.

The docking device 100 can be releasably coupled to a delivery apparatus. For example, in certain examples, the docking device 100 can be coupled to a delivery apparatus (as described further below) via a release suture that can be configured to be tied to the docking device 100 and cut for removal. In one example, the release suture can be tied to the docking device 100 through an eyelet or eyehole 103 located adjacent the proximal end 102p of the coil. In another example, the release suture can be tied around a circumferential recess that is located adjacent the proximal end 102p of the coil 102.

In some examples, the docking device 100 in the deployed configuration can be configured to fit at the mitral valve position. In other examples, the docking device can also be shaped and/or adapted for implantation at other native valve positions as well, such as at the tricuspid valve. As described herein, the geometry of the docking device 100 can be configured to engage the native anatomy, which can, for example, provide for increased stability and reduction of relative motion between the docking device 100, the prosthetic valve docked therein, and/or the native anatomy. Reduction of such relative motion can, among other things, prevent material degradation of components of the docking device 100 and/or the prosthetic valve docked therein and/or prevent damage or trauma to the native tissue.

As shown in FIGS. 1A-1B, the coil 102 in the deployed configuration can include a leading turn 106 (or "leading coil"), a central region 108, and a stabilization turn 110 (or "stabilization coil") around the central longitudinal axis 101. The central region 108 can possess one or more helical turns having substantially equal inner diameters. The leading turn 106 can extend from a distal end of the central region 108 and has a diameter greater than the diameter of the central region 108 (in one or more configurations). The stabilization turn 110 can extend from a proximal end of the central region 108 and has a diameter greater than the diameter of the central region 108 (in one or more configurations).

In certain examples, the central region 108 can include a plurality of helical turns, such as a proximal turn 108p in connection with the stabilization turn 110, a distal turn 108d in connection with the leading turn 106, and one or more intermediate turns 108m disposed between the proximal turn 108p and the distal turn 108d. In the example shown in FIG. 1A, there is only one intermediate turn 108m between the proximal turn 108p and the distal turn 108d. In other examples, there are more than one intermediate turns 108m between the proximal turn 108p and the distal turn 108d. Some of the helical turns in the central region 108 can be full turns (i.e., rotating 360 degrees). In some examples, the proximal turn 108p and/or the distal turn 108d can be partial turns (e.g., rotating less than 360 degrees, such as 180 degrees, 270 degrees, etc.).

A size of the docking device 100 can be generally selected based on the size of the desired prosthetic valve to be implanted into the patient. In certain examples, the central region 108 can be configured to retain a radially expandable prosthetic valve (as shown in FIGS. 3A-3B and described further below). For example, the inner diameter of the helical turns in the central region 108 can be configured to be smaller than an outer diameter of the prosthetic valve when the prosthetic valve is radially expanded so that additional radial force can act between the central region 108 and the prosthetic valve to hold the prosthetic valve in place. As described herein, the helical turns (e.g., 108p, 108m, 108d) in the central region 108 are also referred to herein as "functional turns."

The stabilization turn 110 can be configured to help stabilize the docking device 100 in the desired position. For example, the radial dimension of the stabilization turn 110 can be significantly larger than the radial dimension of the coil in the central region 108, so that the stabilization turn 110 can flare or extend sufficiently outwardly so as to abut or push against the walls of the circulatory system, thereby improving the ability of the docking device 100 to stay in its desired position prior to the implantation of the prosthetic valve. In some examples, the diameter of stabilization turn 110 is desirably larger than the native annulus, native valve plane, and/or native chamber for better stabilization. In some examples, the stabilization turn 110 can be a full turn (i.e., rotating about 360 degrees). In some examples, the stabilization turn 110 can be a partial turn (e.g., rotating between about 180 degrees and about 270 degrees).

In one particular example, when implanting the docking device 100 at the native mitral valve location, the functional turns in the central region 108 can be disposed substantially in the left ventricle and the stabilization turn 110 can be disposed substantially in the left atrium. The stabilization turn 110 can be configured to provide one or more points or regions of contact between the docking device 100 and the left atrial wall, such as at least three points of contact in the left atrium or complete contact on the left atrial wall. In certain examples, the points of contact between the docking device 100 and the left atrial wall can form a plane that is approximately parallel to a plane of the native mitral valve.

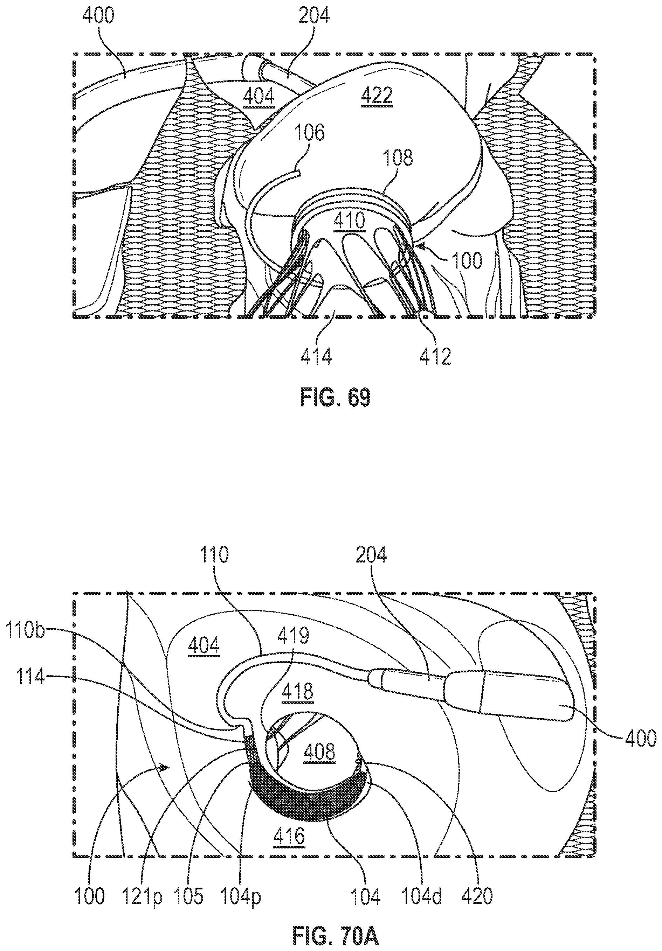

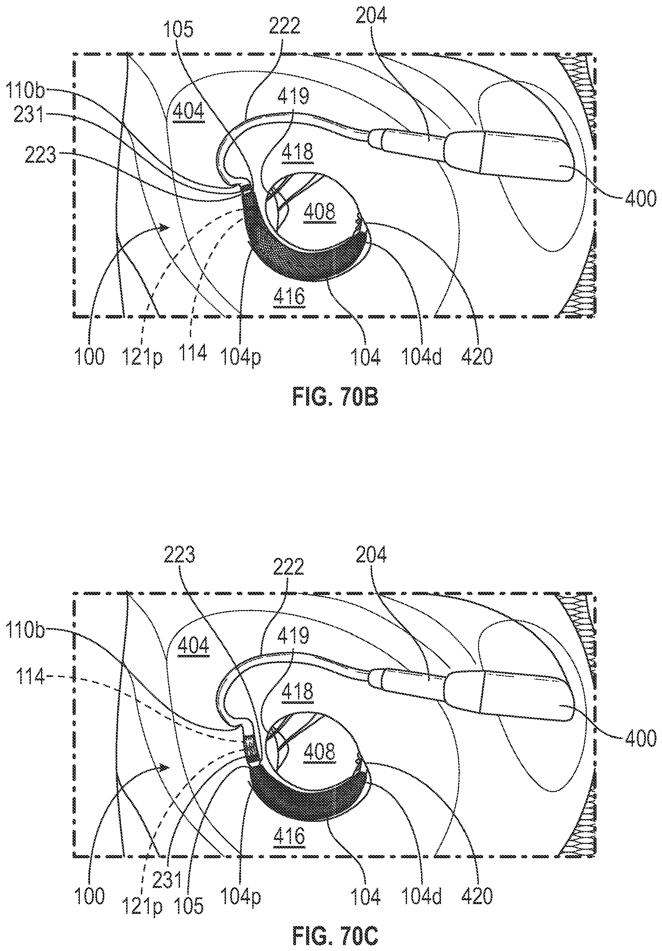

In some examples, the stabilization turn 110 can have an atrial portion 110a in connection with the proximal turn 108p of the central region 108, a stabilization portion 110c adjacent to the proximal end 102p of the coil 102, and an ascending portion 110b located between the atrial portion 110a and the stabilization portion 110c. Both the atrial portion 110a and the stabilization portion 110c can be generally parallel to the helical turns in the central region 108, whereas the ascending portion 110b can be oriented to be angular relative to the atrial portion 110a and the stabilization portion 110c. For example, in certain examples, the ascending portion 110b and the stabilization portion 110c can form an angle from about 45 degrees to about 90 degrees (inclusive). In certain examples, the stabilization portion 110c can define a plane that is substantially parallel to a plane defined by the atrial portion 110a. A boundary 107 (marked by a dashed line in FIG. 1A) between the ascending portion 110b and the stabilization portion 110c can be determined as a location where the ascending portion 110b intersects the plane defined by the stabilization portion 110c. The curvature of the stabilization turn 110 can be configured so that the atrial portion 110a and the stabilization portion 110c are disposed on approximately opposite sides when the docking device 100 is fully expanded. When implanting the docking device 100 at the native mitral valve location, the atrial portion 110a can be configured to abut the posterior wall of the left atrium and the stabilization portion 110c can be configured to flare out and press against the anterior wall of the left atrium (see e.g., FIGS. 70-71 and 78).

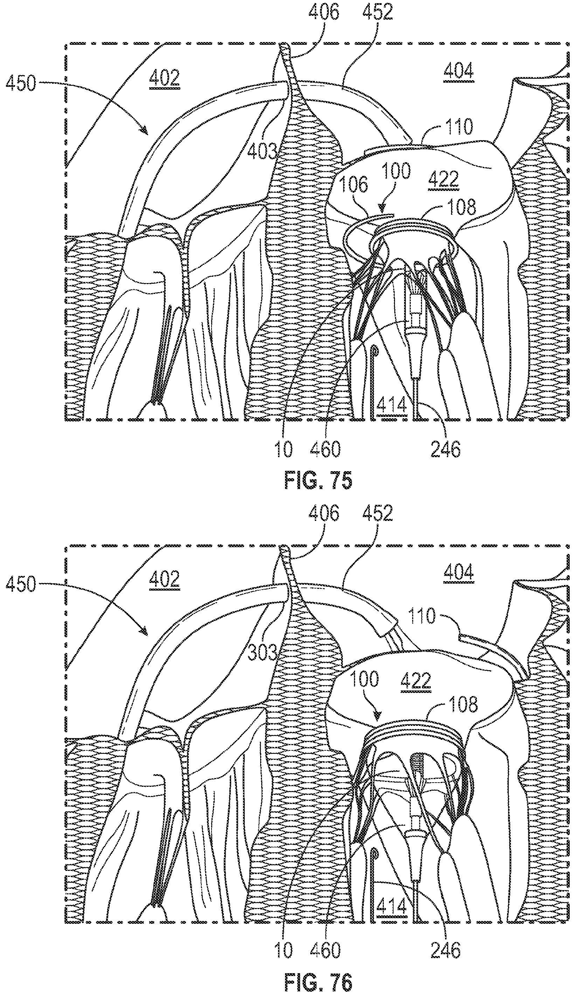

As noted above, the leading turn 106 can have a larger radial dimension than the helical turns in the central region 108. As described herein, the leading turn 106 can help more easily guide the coil 102 around and/or through the chordae tendineae and/or adequately around all native leaflets of the native valve (e.g., the native mitral valve, tricuspid valve, etc.). For example, once the leading turn 106 is navigated around the desired native anatomy, the remaining coil (such as the functional turns) of the docking device 100 can also be guided around the same features. In some examples, the leading turn 106 can be a full turn (i.e., rotating about 360 degrees). In some examples, the leading turn 106 can be a partial turn (e.g., rotating between about 180 degrees and about 270 degrees). As described further below in reference to FIG. 76, when a prosthetic valve is radially expanded within the central region 108 of the coil, the functional turns in the central region 108 can be further radially expanded. As a result, the leading turn 106 can be pulled in the proximal direction and become a part of the functional turn in the central region 108.

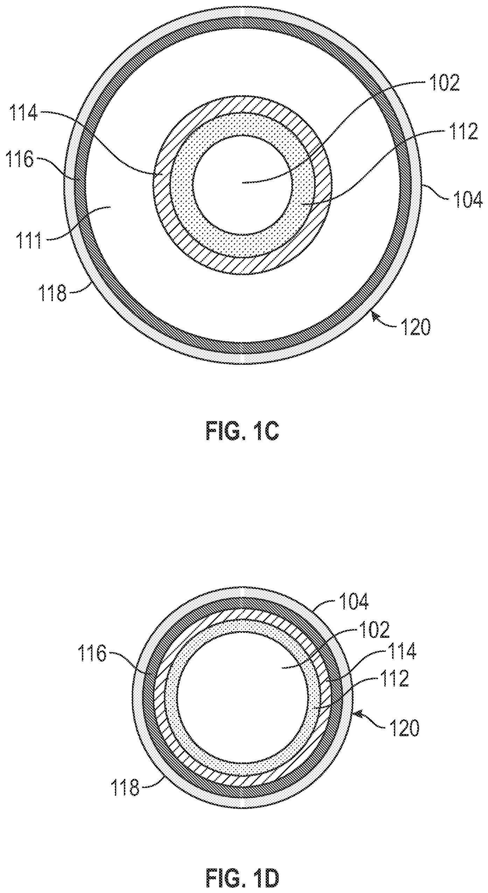

In certain examples, at least a portion of the coil 102 can be surrounded by a first cover 112. As shown in FIGS. 1C-1F, the first cover 112 can have a tubular shape and thus can also be referred to as a "tubular member." In certain examples, the tubular member 112 can cover an entire length of the coil 102. In certain examples, the tubular member 112 covers only selected portion(s) of the coil 102.

In certain examples, the tubular member 112 can be coated on and/or bonded on the coil 102. In certain examples, the tubular member 112 can be a cushioned, padded-type layer protecting the coil. The tubular member 112 can be constructed of various native and/or synthetic materials. In one particular example, the tubular member 112 can include expanded polytetrafluoroethylene (ePTFE). In certain examples, the tubular member 112 is configured to be fixedly attached to the coil 102 (e.g., by means of textured surface resistance, suture, glue, thermal bonding, or any other means) so that relative axial movement between the tubular member 112 and the coil 102 is restricted or prohibited.

In some examples, as illustrated in FIGS. 1C-1D, at least a portion of the tubular member 112 can be surrounded by the retention element 114. In some examples, the tubular member 112 can extend through an entire length of the retention element 114. Exemplary methods of attaching the retention element 114 to the tubular member 112 are described further below.

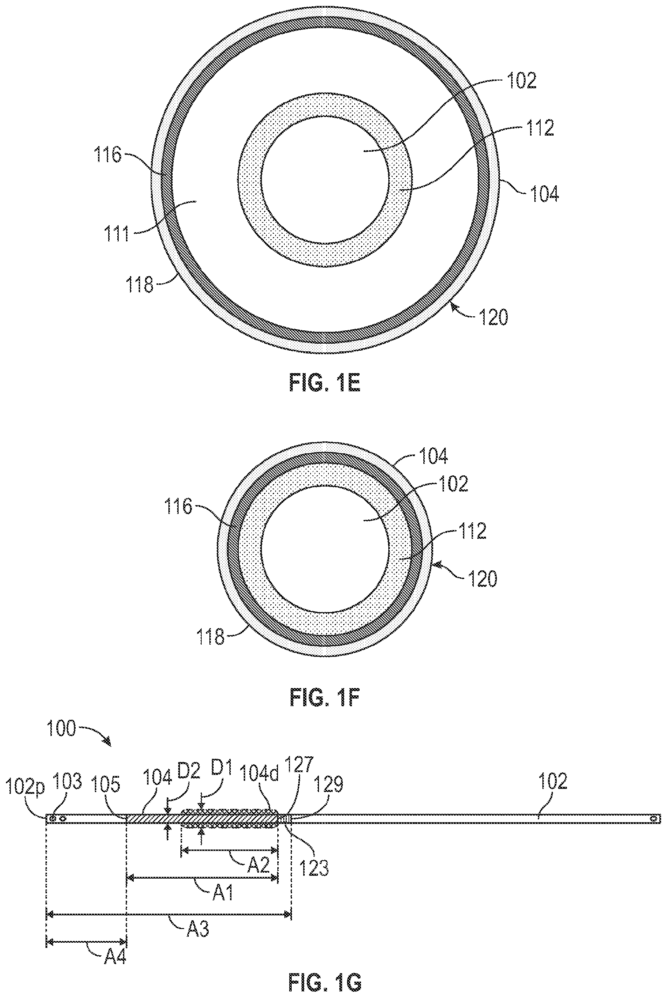

In some examples, a distal end portion of the retention element 114 can extent axially beyond (i.e., positioned distal to) the distal end of the guard member 104, and a proximal end portion of the retention element 114 can extend axially beyond (i.e., positioned proximal to) the proximal end 105 of the guard member 104 to aid retention of prosthetic valve and tissue ingrowth. In one example, a distal end of the retention element 114 can be positioned adjacent the leading turn 106 (e.g., near the location marked by the dashed line 109 in FIG. 1A). In another example, the distal end of the retention element 114 can be disposed at or adjacent a distal end of the coil 102. In one example, a proximal end of the retention element 114 can be disposed at or adjacent the ascending portion 110b of the coil 102. In one example, as illustrated in FIGS. 1E-1F, at least a portion of the tubular member 112 may not be surrounded by the retention element 114.

In certain examples, the docking device 100 can have one or more seating markers. For example, FIGS. 1A-1B show a proximal seating marker 121p and a distal seating marker 121d, wherein the proximal seating marker 121p is positioned proximal relative to the distal seating marker 121d. Both the proximal and distal seating markers 121p, 121d can have predefined locations relative to the coil 102. As shown, both the proximal and distal seating markers 121p, 121d can be disposed distal to the ascending portion 110b, e.g., at the atrial portion 110a, of the coil 102. In addition, a proximal end portion of the retention element 114 can extend to, and/or positioned at, the ascending portion 110b.

In certain examples, both the proximal and distal seating markers 121p, 121d can include a radiopaque material so that these seating markers can be visible under fluoroscopy such as during an implantation procedure. As described further below, the seating markers 121p, 121d can be used to mark the proximal and distal boundaries of a segment of the coil 102 where the proximal end 105 of the guard member 104 can be positioned when deploying the docking device 100.

In certain examples, the seating markers 121p, 121d can be disposed on the tubular member 112 and covered by the retention element 114. In some examples, the seating markers 121p, 121d can be disposed on the atrial portion 110a of the coil 102 and covered by the tubular member 112. In particular examples, the seating markers 121p, 121d can be disposed directly on the retention element 114. In yet alternative examples, the seating markers 121p, 121d can be disposed on different layers relative to each other. For example, one of the seating markers (e.g., 121p) can be disposed outside the tubular member 112 and covered by the retention element 114, whereas another seating marker (e.g., 121d) can be disposed directly on the coil 102 and covered by the tubular member 112.

In certain examples, a segment of the coil 102 located between the proximal seating marker 121p and the distal seating marker 121d can have an axial length between about 2 mm and about 7 mm, or between about 3 mm and about 5 mm. In one specific example, the axial length of the coil segment between the proximal seating marker 121p and the distal seating marker 121d is about 4 mm.

In certain examples, an axial distance between the proximal seating marker 121p and a distal end of the ascending portion 110b is between about 10 mm and about 30 mm, or between about 15 mm and about 25 mm. In one specific example, the axial distance between the proximal seating marker 121p and the distal end of the ascending portion 110b is about 20 mm.

Although two seating markers 121p, 121d are shown in FIGS. 1A-1B, it is to be understood that the number of seating markers can be more than two or less than two. For example, in one example, the docking device 100 can have only one seating marker (e.g., 121p). In another example, one or more additional seating markers can be placed between the proximal and distal seating markers 121p, 121d. As noted above, the proximal end 105 of the guard member can be positioned between the proximal and distal seating markers 121p, 121d when deploying the docking device 100. As such, these additional seating markers can function as a scale to indicate a precise location of the proximal end 105 of the guard member 104 relative to the coil 102.

As described herein, the guard member 104 can constitute a part of a cover assembly 120 for the docking device 100. In some examples, the cover assembly 120 can also include the tubular member 112. In some examples, the cover assembly 120 can further include the retention element 114.

In some examples, as shown in FIGS. 1A-1B, when the docking device 100 is in the deployed configuration, the guard member 104 can be configured to cover a portion (e.g., the atrial portion 110a) of the stabilization turn 110 of the coil 102. In certain examples, the guard member 104 can be configured to cover at least a portion of the central region 108 of the coil 102, such as a portion of the proximal turn 108p. In certain examples, the guard member 104 can extend over the entirety of the coil 102.

As described herein, the guard member 104 can radially expand so as to help preventing and/or reducing paravalvular leakage. Specifically, the guard member 104 can be configured to radially expand such that an improved seal is formed closer to and/or against a prosthetic valve deployed within the docking device 100. In some examples, the guard member 104 can be configured to prevent and/or inhibit leakage at the location where the docking device 100 crosses between leaflets of the native valve (e.g., at the commissures of the native leaflets). For example, without the guard member 104, the docking device 100 may push the native leaflets apart at the point of crossing the native leaflets and allow for leakage at that point (e.g., along the docking device or to its sides). However, the guard member 104 can be configured to expand to cover and/or fill any opening at that point and inhibit leakage along the docking device 100.

In another example, when the docking device 100 is deployed at a native atrioventricular valve, the guard member 104 covers predominantly a portion of the stabilization turn 110 and/or a portion of the central region 108. In one example, the guard member 104 can cover predominantly the atrial portion 110a of the stabilization turn 110 that is located distal to the ascending portion 110b. Thus, the guard member 104 does not extend into the ascending portion 110b (or at least the guard member 104 can terminate before the anterolateral commissure 419 of the native valve, see e.g., FIGS. 70-71) when the docking device 100 is in the deployed configuration. In certain circumstances, the guard member 104 can extend onto the ascending portion 110b. This may cause the guard member 104 to kink, which (in some instances) may reduce the performance and/or durability of the guard member. Thus, the retention member 114 can, among other things, improve the functionality and/or longevity of the guard member 114 by preventing the guard member 104 from extending into the ascending portion 110b of the coil 102.

Yet in alternative examples, the guard member 104 can cover not only the atrial portion 110a but can also extend over the ascending portion 110b of the stabilization turn 110. This can occur, e.g., in circumstances when the docking device is implanted in other anatomical locations and/or the guard member 104 is reinforced to reduce the risk of wire break.

In various examples, the guard member 104 can help covering an atrial side of an atrioventricular valve to prevent and/or inhibit blood from leaking through the native leaflets, commissures, and/or around an outside of the prosthetic valve by blocking blood in the atrium from flowing in an atrial to ventricular direction (i.e., antegrade blood flow)-other than through the prosthetic valve. Positioning the guard member 104 on the atrial side of the valve can additionally or alternatively help reduce blood in the ventricle from flowing in a ventricular to atrial direction (i.e., retrograde blood flow).

In some examples, the guard member 104 can be positioned on a ventricular side of an atrioventricular valve to prevent and/or inhibit blood from leaking through the native leaflets, commissures, and/or around an outside of the prosthetic valve by blocking blood in the ventricle from flowing in a ventricular to atrial direction (i.e., retrograde blood flow). Positioning the guard member 104 on the ventricular side of the valve can additionally or alternatively help reduce blood in the atrium from flowing in the atrial direction to ventricular direction (i.e., antegrade blood flow)-other than through the prosthetic valve.

The guard member 104 can include an expandable member 116 and a cover member 118 (also referred to as a "second cover" or an "outer cover") surrounding an outer surface of the expandable member 116. In certain examples, the expandable member 116 surrounds at least a portion of the tubular member 112. In certain examples, the tubular member 112 can extend (completely or partially) through the expandable member 116.

The expandable member 116 can extend radially outwardly from the coil 102 (and the tubular member 112) and is movable between a radially compressed (and axially elongated) state and a radially expanded (and axially foreshortened) state. That is, the expandable member 116 can axially foreshorten when it moves from the radially compressed state to the radially expanded state and can axially elongate when it moves from the radially expanded state to the radially compressed state.

In certain examples, the expandable member 116 can include a braided structure, such as a braided wire mesh or lattice. In certain examples, the expandable member 116 can include a shape memory material that is shape set and/or pre-configured to expand to a particular shape and/or size when unconstrained (e.g., when deployed at a native valve location). For example, the expandable member 116 can have a braided structure containing a metal alloy with shape memory properties, such as Nitinol or cobalt chromium. The number of wires (or fibers, strands, or the like) forming the braided structure can be selected to achieve a desired elasticity and/or strength of the expandable member 116. In certain examples, the number of wires used to braid the expanding member 116 can range from 16 to 128 (e.g., 48 wires, 64 wires, 96 wires, etc.). In certain examples, the braid density can range from 20 picks per inch (1 pick per inch (PPI) = 39.37 picks per meter) to 70 PPI, or from 25 PPI to 65 PPI. In one specific example, the braid density is about 36 PPI. In another specific example, the braid density is about 40 PPI. In certain examples, the diameter of the wires can range from about 0.002 inch (1 inch = 2,54 cm) to about 0.004 inch. In one particularly example, the diameter of the wires can be about 0.003 inch. In another example, the expandable member 116 can be a combination of braided Nitinol wire and textile (e.g., polyethylene terephthalate (PET), polytetrafluoroethylene (PTFE), etc.) yarns. In yet another example, the expandable member 116 can include a polymeric material, such as a thermoplastic material (e.g., PET, polyether ether ketone (PEEK), thermoplastic polyurethane (TPU), etc.).

In certain examples, the expandable member 116 can include a foam structure. For example, the expandable member can include an expandable memory foam which can expand to a specific shape or specific pre-set shape upon removal of a crimping pressure (e.g., removal of the docking device 100 from the delivery sheath) prior to delivery of the docking device.

As described herein, the cover member 118 can be configured to be so elastic that when the expandable member 116 moves from the radially compressed (and axially elongated) state to the radially expanded (and axially foreshortened) state, the cover member 118 can also radially expand and axially foreshorten together with the expandable member 116. In other words, the guard member 104, as a whole, can move from a radially compressed (and axially elongated) state to a radially expanded (and axially foreshortened) state. As described herein, the radially expanded (and axially foreshortened) state is also referred to as the "relaxed state," and the radially compressed (and axially elongated) state is also referred to as the "collapsed state."

In certain examples, the cover member 118 can be configured to be atraumatic to native tissue and/or promote tissue ingrowth into the cover member 118. For example, the cover member 118 can have pores to encourage tissue ingrowth. In another example, the cover member 118 can be impregnated with growth factors to stimulate or promote tissue ingrowth, such as transforming growth factor alpha (TGF-alpha), transforming growth factor beta (TGF-beta), basic fibroblast growth factor (bFGF), vascular epithelial growth factor (VEGF), and combinations thereof. The cover member 118 can be constructed of any suitable material, including foam, cloth, fabric, and/or polymer, which is flexible to allow for compression and expansion of the cover member 118. In one example, the cover member 118 can include a fabric layer constructed from a thermoplastic polymer material, such as polyethylene terephthalate (PET).

As described more fully below, a distal end portion 104d of the guard member 104 (including a distal end portion of the expandable member 116 and a distal end portion of the cover member 118) can be fixedly coupled to the coil 102 (e.g., via a distal suture), and a proximal end portion 104p of the guard member 104 (including a proximal end portion of the expandable member 116 and a proximal end portion of the cover member 118) can be axially movable relative to the coil 102. Further, the proximal end portion of the expandable member 116 can be fixedly coupled to the proximal end portion of the cover member 118, e.g., via a proximal suture, as described more fully below.

When the docking device 100 is retained within the delivery sheath in the substantially straight configuration, the expandable member 116 can be radially compressed by the delivery sheath and remains in the radially compressed (and axially elongated) state. The radially compressed (and axially elongated) expandable member 116 can contact the retention element 114 (see, e.g., FIG. 1C) or the tubular member 112 (see, e.g., FIG. 1E) so that no gap or cavity exists between the retention element 114 and the expandable member 116 or between the tubular member 112 (and/or the coil 102) and the expandable member 116.

After the docking device 100 is removed from the delivery sheath and changes from the delivery configuration to the deployed configuration, the guard member 104 can also move from a delivery configuration to a deployed configuration. In certain examples, a dock sleeve (which is described more fully below) can be configured to cover and retain the docking device 100 within the delivery sheath when navigating the delivery sheath through the patient's native valve. The docking sleeve can also, for example, help to guide the docking device around the native leaflets and chordae. Retraction of the dock sleeve relative to the docking device 100 can expose the guard member 104 and cause it to move from the delivery configuration to the deployed configuration. Specifically, without the constraint of the delivery sheath and the dock sleeve, the expandable member 116 can radially expand (and axially foreshorten) so that a gap or cavity 111 can be created between the retention element 114 and the expandable member 116 (see, e.g., FIG. 1C) and/or between the tubular member 112 and the expandable member 116 (see, e.g., FIG. 1E). Thus, when the guard member 104 is in the delivery configuration, an outer edge of the guard member 104 can extend along and adjacent the coil 102 (since there is no gap 111, only the retention element 114 and/or the tubular member 112 separate the coil 102 from the expandable member 116, as shown in FIG. 1D and FIG. 1F). When the guard member 104 is in the deployed configuration, the outer edge of the guard member 104 can form a helical shape rotating about the central longitudinal axis 101 (see, e.g., FIGS. 1A-1B and 3A-3B) and at least a segment of the outer edge of guard member can extend radially away from the coil 102 (e.g., due to the creation of the gap 111 between the expandable member 116 and the retention element 114 or the tubular member 112).

Because the distal end portion 104d of the guard member 104 is fixedly coupled to the coil 102 and the proximal end portion 104p of the guard member 104 can be axially moveable relative to the coil 102, the proximal end portion 104p of the guard member 104 can slide axially over the tubular member 112 and toward the distal end 102d of the coil 102 when expandable member 116 moves from the radially compressed state to the radially expanded state. As a result, the proximal end portion 104p of the guard member 104 can be disposed closer to the proximal end 102p of the coil 102 when the expandable member 116 is in the radially compressed state than in the radially expanded state.

In certain examples, the cover member 118 can be configured to engage with the prosthetic valve deployed within the docking device 100 so as to form a seal and reduce paravalvular leakage between the prosthetic valve and the docking device 100 when the expandable member 116 is in the radially expanded state. The cover member 118 can also be configured to engage with the native tissue (e.g., the native annulus and/or native leaflets) to reduce PVL between the docking device and/or the prosthetic valve and the native tissue.

In certain examples, when the expandable member 116 is in the radially expanded state, the proximal end portion 104p of the guard member 104 can have a tapered shape as shown in FIGS. 1A-1B, such that the diameter of the proximal end portion 104p gradually increases from a proximal end 105 of the guard member 104 to a distally located body portion of the guard member 104. This can, for example, help to facilitate loading the docking device into a delivery sheath of the delivery apparatus and/or retrieval and/or repositioning of the docking device into the delivery apparatus during an implantation procedure. In addition, due to its small diameter, the proximal end 105 of the guard member 104 can frictionally engage with the retention element 114 so that the retention element 114 can reduce or prevent axial movement of the proximal end portion 104p of the guard member 104 relative to the coil 102.

In certain examples, the docking device 100 can include at least one radiopaque marker configured to provide visual indication about the location of the docking device 100 relative to its surrounding anatomy, and/or the amount of radial expansion of the docking device 100 (e.g., when a prosthetic valve is subsequently deployed in the docking device 100) under fluoroscopy. For example, one or more radiopaque markers can be placed on the coil 102. In one particular example, a radiopaque marker (which can be larger than the seating markers 121p, 121d) can be disposed at the central region 108 of the coil. In another example, one or more radiopaque markers can be placed on the tubular member 112, the expandable member 116, and/or the cover member 118. As noted above, the docking device 100 can also have one or more radiopaque markers (e.g., 121p and/or 121d) located distal to the ascending portion 110b of the coil 102. The radiopaque marker(s) used to provide visual indication about the location and/or the amount of radial expansion of the docking device 100 can be in addition to the seating markers (e.g., 121p, 121d) described above.

FIG. 1G schematically depicts some example dimensions of the docking device 100 when the coil 102 is in a substantially straight configuration (e.g., compared to the helical configuration depicted in FIG. 1). The guard member 104 surrounding the coil 102 is shown in both the collapsed state (shown in solid contour) and the relaxed state (shown in dashed contour). In certain examples, the guard member 104 in the relaxed state can have a maximum outer diameter (D1) ranging from about 4 mm to about 8 mm (e.g., about 6 mm in one particular example), and the guard member 104 in the collapsed state can have a maximum outer diameter (D2) ranging from about 1 mm to about 3 mm (e.g., about 2 mm in one particular example). The expansion of the guard member 104 from the collapsed state to the relaxed state can be characterized by an expansion ratio defined as D1/D2. In certain examples, the expansion ratio can range from about 1.5 to about 8, or from about 2 to about 6, or from about 2.5 to about 4. In one specific example, the expansion ratio is about 3.

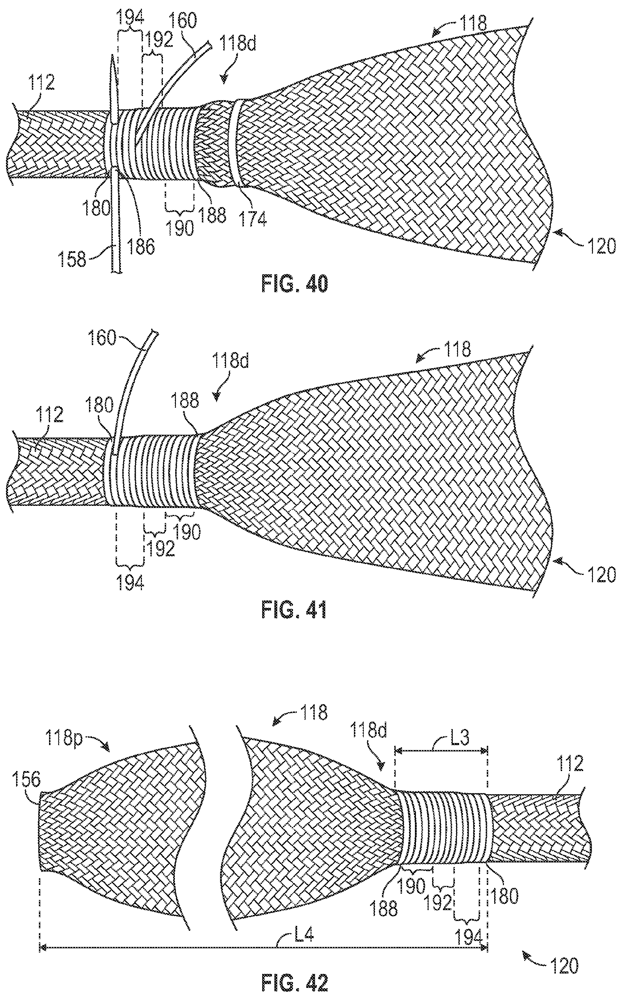

As described more fully below, the distal end portion 104d of the guard member 104 can be fixedly attached to the coil 102 via a distal suture forming a plurality of wraps and knots. Such wraps and knots can define a distal attachment region 123 where the attached portion of the guard member 104 is fixed relative to the coil 102. Thus, only the portion of the guard member 104 that is proximal to the distal attachment region 123 is movable relative to the coil 102. The distal attachment region 123 has a proximal end 127 and a distal end 129. The axial length of the distal attachment region 123, measured between the proximal end 127 and the distal end 129, can approximate L3, which is depicted in FIG. 42 and described further below.

Returning again to FIG. 1G, in certain examples, when the guard member 104 is in the relaxed state, its movable portion (i.e., the portion extending from the proximal end 105 of the guard member 104 to the proximal end 127 of the distal attachment region 123) can have an axial length (A2) ranging from about 30 mm to about 100 mm. In one specific example, A2 is about 51 mm. In another specific example, A2 is about 81 mm. When the guard member 104 is in the collapsed state, its movable portion can have an axial length (A1) ranging from about 50 mm to about 120 mm. In one specific example, A1 is about 72 mm. In another specific example, A1 is between 105 mm and 106.5 mm. The elongation of the guard member 104 from the relaxed state to the collapsed state can be characterized by an elongation ratio defined as A1/A2. In certain examples, the elongation ratio can range from about 1.05 to about 1.7, or from about 1.1 to about 1.6, or from about 1.2 to about 1.5, or from 1.3 to about 1.4. In one specific example, the elongation ratio is about 1.47. In another specific example, the elongation ratio is about 1.31.

In certain examples, an axial length (A3) measured from the proximal end 102p of the coil 102 to the distal end 129 of the distal attachment region 123 can range from about 130 mm to about 200 mm, or from about 140 mm to about 190 mm. In one specific example, A3 is between 133 mm and 135 mm (e.g., 134 mm). In another specific example, A3 is between 178 mm and 180 mm (e.g., 179 mm). In certain examples, when the guard member 104 is in the collapsed state, an axial length (A4) measured from the proximal end 102p of the coil 102 to the proximal end 105 of the guard member 104 can range from about 40 mm to about 90 mm, or from about 50 mm to about 80 mm. In certain examples, A4 is between 60 mm and 70 mm (e.g., 61 mm).

Further details of the docking device and its variants, including various examples of the coil, the first cover (or tubular member), the second cover (or cover member), the expandable member, and other components of the docking device, are described in PCT Patent Application Publication No. WO/2020/247907.

### Exemplary Prosthetic Valves

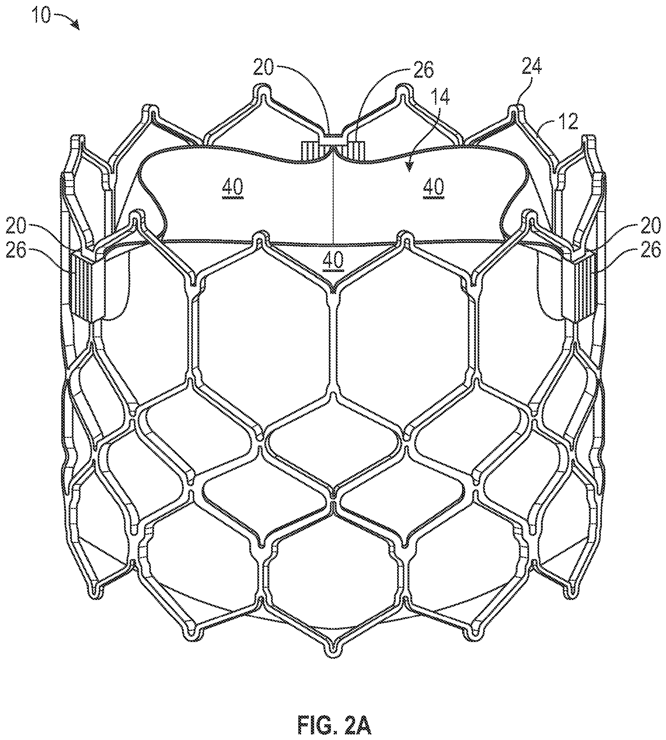

FIGS. 2A-2B show a prosthetic valve 10, according to one example. The prosthetic valve 10 can be adapted to be implanted, with or without a docking device, in a native valve annulus, such as the native mitral valve annulus, native aortic annulus, native pulmonary valve annulus, etc. The prosthetic valve 10 can include a stent, or frame, 12, a valvular structure 14, and a valve cover 16 (the valve cover 16 is removed in FIG. 2A to show the frame structure).

The valvular structure 14 can include three leaflets 40, collectively forming a leaflet structure (although a greater or fewer number of leaflets can be used), which can be arranged to collapse in a tricuspid arrangement. The leaflets 40 are configured to permit the flow of blood from an inflow end 22 to an outflow end 24 of the prosthetic valve 10 and block the flow of blood from the outflow end 24 to the inflow end 22 of the prosthetic valve 10. The leaflets 40 can be secured to one another at their adjacent sides to form commissures 26 of the leaflet structure. The lower edge of valvular structure 14 desirably has an undulating, curved scalloped shape. By forming the leaflets 40 with this scalloped geometry, stresses on the leaflets 40 can be reduced, which in turn can improve durability of the prosthetic valve 10. Moreover, by virtue of the scalloped shape, folds and ripples at the belly of each leaflet 40 (the central region of each leaflet), which can cause early calcification in those areas, can be eliminated or at least minimized. The scalloped geometry can also reduce the amount of tissue material used to form leaflet structure, thereby allowing a smaller, more even crimped profile at the inflow end of the prosthetic valve 10. The leaflets 40 can be formed of pericardial tissue (e.g., bovine pericardial tissue), biocompatible synthetic materials, or various other suitable natural or synthetic materials as known in the art and described in U.S. Patent No. 6,730,118.

The frame 12 can be formed with a plurality of circumferentially spaced slots, or commissure windows 20 (three in the illustrated example) that are adapted to mount the commissures 26 of the valvular structure 14 to the frame. The frame 12 can be made of any of various suitable plastically expandable materials (e.g., stainless steel, etc.) or self-expanding materials (e.g., Nitinol) as known in the art. When constructed of a plastically expandable material, the frame 12 (and thus the prosthetic valve 10) can be crimped to a radially compressed state on a delivery apparatus and then expanded inside a patient by an inflatable balloon or equivalent expansion mechanism. When constructed of a self-expandable material, the frame 12 (and thus the prosthetic valve 10) can be crimped to a radially compressed state and restrained in the compressed state by insertion into a valve sheath or equivalent mechanism of a delivery apparatus. Once inside the body, the prosthetic valve 10 can be advanced from the delivery sheath, which allows the prosthetic valve 10 to expand to its functional size.

Suitable plastically expandable materials that can be used to form the frame 12 include, without limitation, stainless steel, a nickel-based alloy (e.g., a cobalt-chromium or a nickel-cobalt-chromium alloy), polymers, or combinations thereof. In particular examples, frame 12 can be made of a nickel-cobalt-chromium-molybdenum alloy, such as MP35N^{™} (tradename of SPS Technologies), which is equivalent to UNS R30035 (covered by ASTM F562-02). MP35N^{™}/UNS R30035 comprises 35% nickel, 35% cobalt, 20% chromium, and 10% molybdenum, by weight. It has been found that the use of MP35N to form the frame 12 can provide superior structural results over stainless steel. In particular, when MP35N is used as the frame material, less material is needed to achieve the same or better performance in radial and crush force resistance, fatigue resistances, and corrosion resistance. Moreover, since less material is required, the crimped profile of the frame can be reduced, thereby providing a lower profile valve assembly for percutaneous delivery to the treatment location in the body.

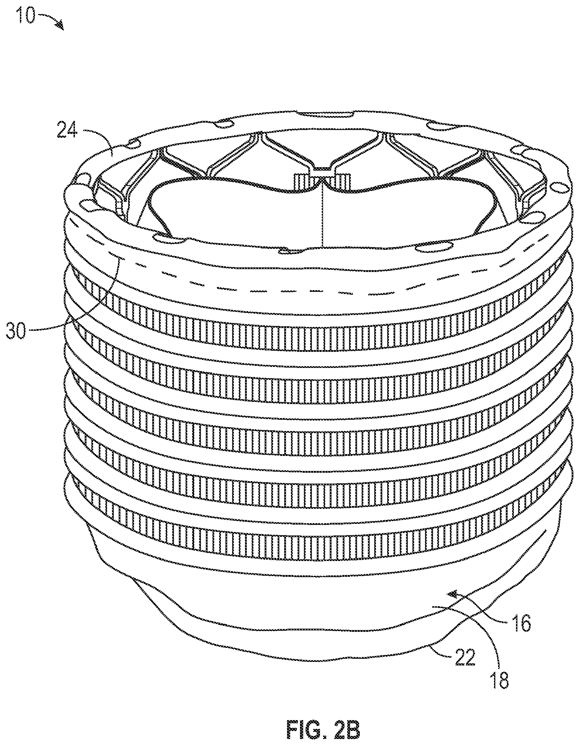

As shown in FIG. 2B, the valve cover 16 can include an outer portion 18 which can cover an entire outer surface of the frame 12. In certain examples, as shown in FIG. 3A, the valve cover 16 can also include an inner portion 28 which can cover an entire inner surface of the frame 12, or alternatively, cover only selected portions of the inner surface of the frame 12. The valve cover 16 can be affixed to the frame 12 by a variety of means, such as via sutures 30.

As described herein, the valve cover 16 can be configured to prevent paravalvular leakage between the prosthetic valve 10 and the native valve, to protect the native anatomy, to promote tissue ingrowth, among some other purposes. For mitral valve replacement, due to the general D-shape of the mitral valve and relatively large annulus compared to the aortic valve, the valve cover 16 can act as a seal around the prosthetic valve 10 (e.g., when the prosthetic valve 10 is sized to be smaller than the annulus) and allows for smooth coaptation of the native leaflets against the prosthetic valve 10.

In various examples, the valve cover 16 can include a material that can be crimped for transcatheter delivery of the prosthetic valve 10 and is expandable to prevent paravalvular leakage around the prosthetic valve 10. Examples of possible materials include foam, cloth, fabric, one or more synthetic polymers (e.g., polyethylene terephthalate (PET), polytetrafluoroethylene (PTFE), expanded polytetrafluoroethylene (ePTFE), etc.), organic tissues (e.g., bovine pericardium, porcine pericardium, equine pericardium, etc.), and/or an encapsulated material (e.g., an encapsulated hydrogel).

In certain examples, the valve cover 16 can be made of a woven cloth or fabric possessing a plurality of floated yarn sections 32 (e.g., protruding or puffing sections, also referred to as "floats" hereinafter). Details of exemplary covered valves with a plurality of floats 32 are further described in U.S. Patent Publication Nos. US2019/0374337, US2019/0192296, and US2019/0046314. In certain examples, the float yarn sections 32 are separated by one or more horizontal bands 34. In some examples, the horizontal bands 34 can be constructed via a leno weave, which can improve the strength of the woven structure. In some examples of the woven cloth, vertical fibers (e.g., running along the longitudinal axis of the prosthetic valve 10) can include a yarn or other fiber possessing a high level of expansion, such as a texturized weft yarn, while horizontal fibers (e.g., running circumferentially around the prosthetic valve 10) in a leno weave can include a low expansion yarn or fiber.

In some examples, the valve cover 16 can include a woven cloth resembling a greige fabric when assembled and under tension (e.g., when stretched longitudinally on a compressed valve prior to delivery of a prosthetic valve 10). When the prosthetic valve 10 is deployed and expanded, tension on floats 32 is relaxed allowing expansion of the floats 32. In some examples, the valve cover 16 can be heat set to allow floats 32 to return to an enlarged, or puffed, space-filling form. In some examples, the number and sizes of floats 32 can be optimized to provide a level of expansion to prevent paravalvular leakage across the mitral plane (e.g., to have a higher level of expansion thickness) and/or a lower crimp profile (e.g., for delivery of the prosthetic valve). Additionally, the horizontal bands 34 can be optimized to allow for attachment of the valve cover 16 to the frame 12 based on the specific size or position of struts or other structural elements on the prosthetic valve 10.

Further details of the prosthetic valve 10 and its components are described, for example, in U.S. Patent Nos. 9,393,110 and 9,339,384. Additional examples of the valve cover are described in PCT Patent Application Publication No. WO/2020/247907.

As described above and illustrated in FIGS. 3A-3B, the prosthetic valve 10 can be radially expanded and securely anchored within the docking device 100.

In certain examples, and as described further below in reference to FIGS. 75-76, the coil 102 of the docking device 100 in the deployed configuration can be movable between a first radially expanded configuration before the prosthetic valve 10 is radially expanded within the coil 102 and a second radially expanded configuration after the prosthetic valve 10 is radially expanded within the coil 102. In the example depicted in FIGS. 3A-3B, the coil 102 is in the second radially expanded configuration since the prosthetic valve 10 is shown in the radially expanded state.

As described herein, at least a portion of the coil 102, such as the central region 108, can have a larger diameter in the second radially expanded configuration than in the first radially expanded configuration (i.e., the central region 108 can be further radially expanded by radially expanding the prosthetic valve 10). As the central region 108 increases in diameter when the coil 102 moves from the first radially expanded configuration to the second radially expanded configuration, the functional turns in the central region 108 and the leading turn 106 can rotate circumferentially (e.g., in clockwise or counter-clockwise direction when viewed from the stabilization turn 110). Circumferential rotation of the functional turns in the central region 108 and the leading turn 106, which can also be referred to as "clocking," can slightly unwind the helical coil in the central region 108. Generally, the unwinding can be less a turn, or less than a half turn (i.e., 180 degrees). For example, the unwinding can be about 60 degrees and may be up to 90 degrees in certain circumstances. As a result, a distance between the proximal end 102p and the distal end 102d of the coil 102 measured along the central longitudinal axis of the coil 102 can be foreshorten.

In the example depicted in FIGS. 3A-3B (and FIG. 78), the proximal end 105 of the guard member 104 is shown to be positioned distal to the proximal seating marker 121p. In other examples, after the prosthetic valve 10 is radially expanded within the coil 102, the proximal end 105 of the guard member 104 can be positioned proximal to the proximal seating marker 121p (i.e., the proximal seating marker 121p is covered by the guard member 104) but remains distal to the ascending portion 110b.

### Overview of Exemplary Cover Assembly

As described above, the docking device 100 can have a cover assembly 120 including the tubular member 112 and the guard member 104, and in some instances the retention element 114. The guard member 104 can further include the expandable member 116 and the cover member 118. As described herein, the cover member 118 can be fixedly coupled to the expandable member 116 so that the cover member 118 can radially expand and axially foreshorten together with the expandable member 116.

In one example, the cover assembly 120 can be assembled by fixedly attaching the distal end portion 104d of the guard member 104 to the coil 102 (and the tubular member 112 surrounding the coil 102) while leaving the proximal end portion 104p of the guard member 104 unattached to the coil 102 (and the tubular member 112 surrounding the coil 102). Thus, the proximal end portion 104p can be axially movable relative to the coil 102 and the tubular member 112. As a result, when the coil 102 moves from the delivery configuration to the deployed configuration (e.g., during the initial deployment of the docking device 100), the proximal end portion 104p of the guard member 104 can slide distally over the coil 102 to cause the guard member 104 to contract axially (i.e., with decrease of axial length) while it expands radially (i.e., with increase in diameter).

On the other hand, the retention element 114, by applying a friction force (e.g., the frictional interaction between the retention element 114 and the proximal end 105 of the guard member 104), can limit the extent of distal movement of the proximal end portion 104p relative to the coil 102. For example, if the proximal end portion 104p of a fully expanded guard member 104 (i.e., expanding to its largest diameter) can slide distally over the coil 102 to a first location in the absence of retention element 114, then the presence of the retention element 114 can cause the proximal end portion 104p to slide distally over the coil 102 to a second location that is proximal to the first location. In other words, the retention element 114 can prevent the guard member 104 to expand to its largest diameter and/or contract to its shortest axial length.

Similarly, the retention element 114, by exerting a friction force (e.g., the frictional interaction between the retention element 114 and the proximal end 105 of the guard member 104), can limit the extent of proximal movement of the proximal end portion 104p relative to the coil 102. As noted above and described further below, the coil 102 of the docking device 100 in the deployed configuration can be further radially expanded (e.g., moving from the first radially expanded configuration to the second radially expanded configuration) when the prosthetic valve 10 is radially expanded within the coil 102, and radial expansion of the coil 102 can cause corresponding circumferential rotation of the coil 102. The radially expanded prosthetic valve 10 can press against the guard member 104, causing the guard member 104 to be radially compressed and axially extended. Because the distal end portion 104d of the guard member 104 is fixedly attached to the coil 102 and the proximal end portion 104p of the guard member 104 is untethered to the coil 102, the proximal end portion 104p of the guard member 104 can have a tendency to move proximally relative to the coil 102 when the prosthetic valve 10 is radially expanded within the coil 102. However, the presence of the retention element 114 can impede the proximal end portion 104p of the guard member 104 to move proximally over the coil 102. In specific examples, the presence of the retention element 114 can prevent the proximal end 105 of the guard member 104 from extending onto the ascending portion 110b of the coil 102. This can, for example, improve the functionality and/or durability of the guard member 104, as discussed above.

The guard member 104 can be coupled to the coil 102 and/or tubular member 112 in various ways such as adhesive, fasteners, welding, and/or other means for coupling. For example, in some cases, attaching the cover member 118 to the expandable member 116 or attaching the distal end portion 104d of the guard member to the coil 102 and the tubular member 112 can be achieved by using one or more sutures. However, there are several technical challenges when using the sutures. First, when the expandable member 116 has a meshed wire frame made of certain metal or metal alloy (e.g., Nitinol), sewing the sutures with a needle may scratch the surface of the metal or metal alloy and increase the risk of corrosion for the wire frame when exposed to the bodily fluid, especially if the needle is also made of metal. Sewing the sutures with a non-metal needle (e.g., a plastic needle) has its own disadvantages because the non-metal needle typically has less strength compared to a metal needle, thus making it difficult to thread through various layers of the cover assembly 120. Further, even a non-metal needle can damage the surface of the metal or metal alloy of the wire frame. Second, the routing of sutures can be challenging because the sutures not only must ensure secure attachment between components of the cover assembly 120, but also not significantly increase the radial profile of the guard member 104 so that the docking device 100 can be retained in a delivery sheath of a delivery apparatus for transcatheter implantation.

An example method of assembling the cover assembly is described below. The method describes herein overcomes the challenges described above by forming a plurality of knots and wraps with sutures at both the proximal end portion 104p and distal end portion 104d of the guard member 104. This exemplary method can, for example, secure the guard member to the coil while reducing or eliminating damage to the expandable member and/or maintaining a crimp profile that allows the docking device to fit and be deployed from within the delivery apparatus.

### Exemplary Method of Assembling Proximal End Portion of Guard Member

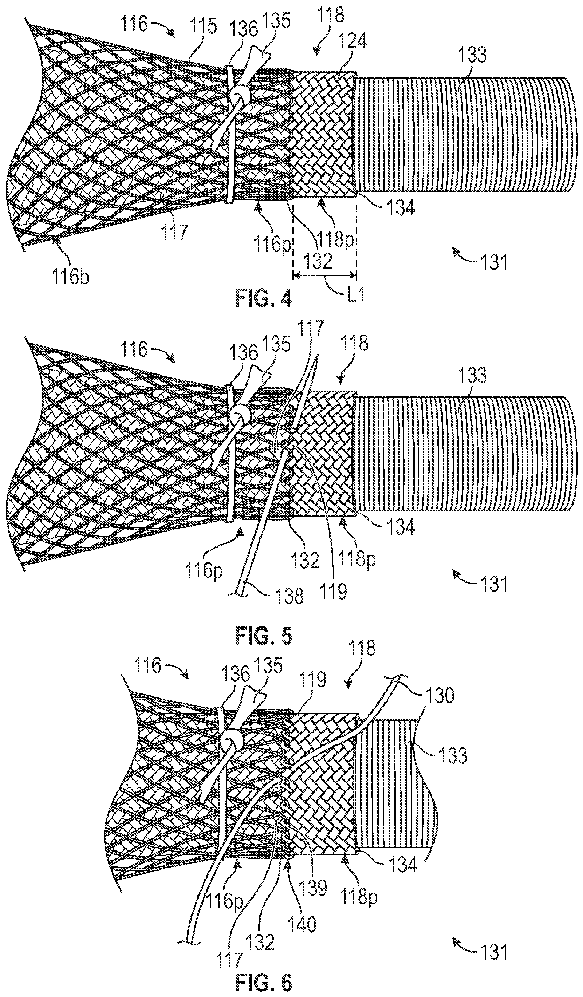

FIGS. 4-19 show a method of fixedly attaching a proximal end portion 116p of the expandable member 116 to a proximal end portion 118p of the cover member 118 via a proximal suture 130, according to one example. As described herein, the proximal suture 130 can comprise an ultra-high molecular weight polyethylene (UHMWPE) material to give it a high strength and durability. In one example, the proximal suture 130 can be a Force Fiber^{®} suture. In other examples, the proximal suture 130 can comprise PTFE and/or other materials. Although described herein as a single suture, one or more sutures can be used.

FIG. 4 shows the cover member 118 being initially positioned within a lumen of the expandable member 116. As shown, the expandable member 116 can have a meshed wire frame, where a plurality of wires 115 are braided together to form a plurality of cells 117. The proximal end portion 116p of the expandable member 116 can, in some examples, taper radially inwardly from a body portion 116b of the expandable member to a terminal proximal end 132 of the expandable member. This can be accomplished via shape-setting. The cover member 118 can be configured to have an annular shape. At least a segment 124 of the proximal end portion 118p of the cover member can extend outside the expandable member 116. In some examples, the terminal proximal end 132 of the expandable member 116 can be spaced apart from a terminal proximal edge 134 of the cover member 118 by a distance L1 (i.e., width of the segment 124) ranging from about 0.5mm to about 3mm. In certain examples, the distance L1 can range from about 1mm to about 2mm. In one example, the distance L1 is about 1.5mm. The terminal proximal edge 134 of the cover member 118 can be sealed (e.g., via heating, sealant, or other means) to prevent it from fraying and/or unraveling. A mandrel 133 can be configured to extend through the cover member 118 in order to provide support for the cover member 118 during the attachment process.

Optionally, a temporary suture 136 can be wrapped around the proximal end portion 116p of the expandable member 116 and a temporary knot 135 can be formed therein. The temporary suture 136 retains the proximal end portion 116p of the expandable member and the proximal end portion 118p of the cover member against the mandrel 133 and prevents relative motion therebetween. In other examples, the temporary suture 136 can be replaced by an O-ring, a tube, a sleeve, and/or other type of restraining member. Thus, a relatively stable fixture 131 comprising the expandable member 116, the cover member 118, and the mandrel 133 can be created.

As described below, the proximal suture 130 can include a plurality of stitches which connect wires 115 at the terminal proximal end 132 of the expandable member 116 to adjacent filaments at the proximal end portion 118p of the cover member.

For example, as shown in FIG. 5, a needle 138 can be used to stitch together the expandable member 116 and the cover member 118 by passing the needle 138 through a cell 117 located at the terminal proximal end 132 of the expandable member and through one or more adjacent strands of yarns or filaments 119 of the cover member 118. Although not shown in FIG. 5, it is to be understood that a first tail 130a of the proximal suture 130 can be connected to the needle 138 and a second tail 130b of the proximal suture 130 can be a free end.

As shown in FIG. 6, the needle 138 can create a plurality of in-and-out stitches 139 that connect wires 115 at the terminal proximal end 132 and the adjacent strands of yarns or filaments 119 of the cover member. The plurality of in-and-out stitches 139 can loop around the terminal proximal end 132 of the expandable member to form a stitch loop 140. As a result, the proximal end portion 118p of the cover member can be connected to the expandable member 116 by the proximal suture 130 via the stitch loop 140. In one example, the stitch loop 140 can extend through every cell 117 around the terminal proximal end 132. In another example, the stitch loop 140 can extend through some of the cells 117 while skipping other cells (e.g., skip one or more cells after stitching through one cell).

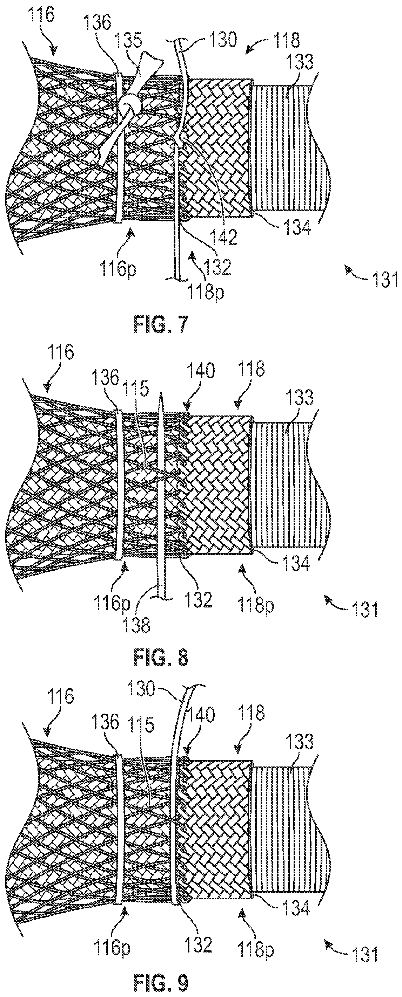

In certain examples, after forming the stitch loop 140, the proximal suture 130 can be tied at the terminal proximal end 132 of the expandable member to create a first knot 142, as shown in FIG. 7. In one example, the first knot 142 can be a square knot tied directly on the terminal proximal end 132 of the wire frame.

In certain examples, a second knot 144 can be tied on opposite side of the first knot 142. For example, as shown in FIG. 8, after creating the first knot 142, the fixture 131 can be rotated about a half turn (i.e., about 180 degrees). Then, the needle 138 (which is connected to the proximal suture 130) can be inserted underneath two (or more) adjacent wires 115 located at the terminal proximal end 132 of the expandable member.

As shown in FIG. 9, pulling the needle 138 out of the expandable member 116 can cause at least a portion of the proximal suture 130 to stay underneath and pick up the two (or more) adjacent wires 115 under which the needle 138 extended through.

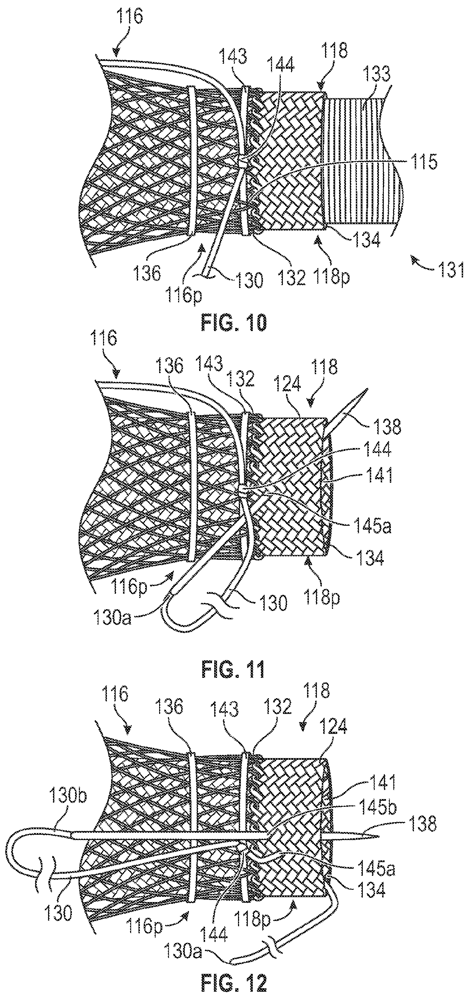

As shown in FIG. 10, the proximal suture 130 can wrap around the terminal proximal end 132 of the expandable member and create the second knot 144. In the depicted example, the second knot 144 is tied at a location that is slightly spaced away (e.g., by one or two cells) from the two (or more) adjacent wires 115 picked up the by the proximal suture 130. In one example, the second knot 144 can be a square knot tied directly on the terminal end of the wire frame.

In the depicted example, the first knot 142 and the second knot 144 are connected to each other by one wrap 143 and are disposed on opposite sides of the annular terminal proximal end 132 of expandable member (i.e., 180 degrees apart). In another example, the first knot 142 and the second knot 144 can be connected by more than one wrap. In some examples, the first knot 142 and/or the second knot 144 can be other types of knot, such as single knot, double knot, etc. In some examples, the first knot 142 or the second knot 144 can be optional. In some examples, there can be more knots (in addition to the first knot 142 and the second knot 144) tied on the terminal proximal end 132 of the expandable member.

As shown in FIG. 11, the mandrel 133 can be removed out of (or retracted partially from) the cover member 118. The needle 138 can then extend through the segment 124 of the cover member 118 extending outside the expandable member 116. In the depicted example, the needle 138 extends through the annular wall of the segment 124 at a first exit location 145a that is adjacent the second knot 144 and the terminal proximal end 132 of the expandable member. The needle 138 can be further pulled out of an annular opening 141 at the terminal proximal edge 134 of the cover member 118. Thus, the first tail 130a of the proximal suture 130 (which is connected to the needle 138) can also extend through the first exit location 145a and further extend out of the annular opening 141 of the cover member 118.

Then, the needle 138 can be disconnected from the first tail 130a and connected to the second tail 130b of the proximal suture 130 (or a second needle can be coupled to the second tail 130b). As shown in FIG. 12, the needle 138 can further extend through the annular wall of the segment 124 at a second exit location 145b that is slightly spaced away from the first exit location 145a (e.g., separated by one, two, or more strands of yarns or filaments of the cover member). Pulling the needle 138 again out of the annular opening 141 can cause the second tail 130b of the proximal suture 130 to extend through the second exit location 145b and further extend out of the annular opening 141 of the cover member 118.

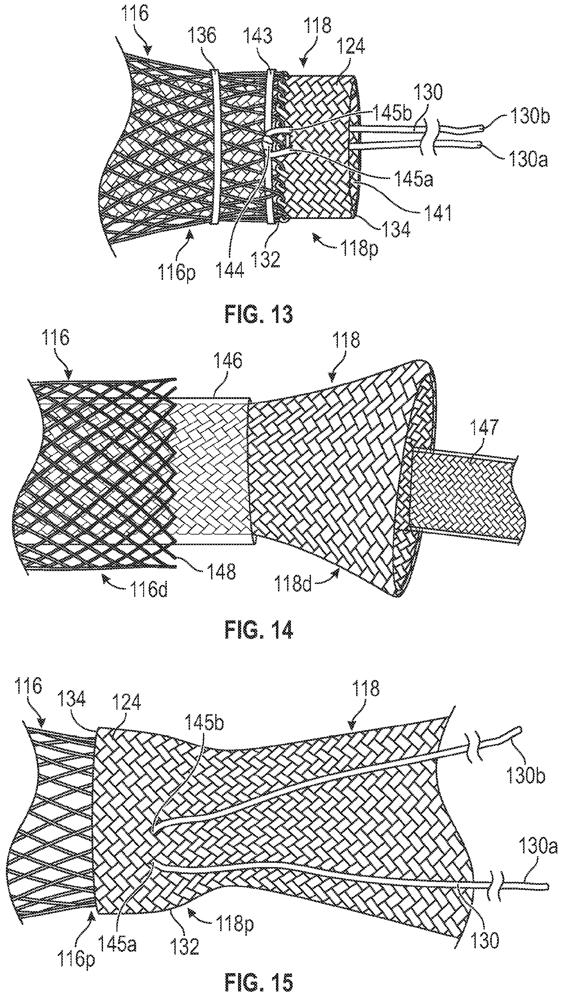

Thus, as shown in FIG. 13, both the first tail 130a and the second tail 130b of the proximal suture can extend from the second knot 144, through the annular wall of the cover member at the respective first exit location 145a and second exit location 145b, and out of the annular opening 141 at the terminal proximal edge 134 of the cover member. As a result, both the first and second tails 130a, 130b of the proximal suture are separated from the expandable member 116 by the cover member 118.

Then, the cover member 118 can be inverted from inside the expandable member 116 to outside the expandable member 116. As shown in FIG. 14, to prevent the cover member 118 from snagging on the expandable member 116 during the inversion, a tubular member 146 having a smooth surface can be inserted into the expandable member 116 from a terminal distal end 148 of the expandable member 116. The tubular member 146 can slide over the cover member 118 so as to separate the cover member 118 from the inner surface of the expandable member 116, as shown in FIG. 14. Optionally, a pusher 147 can press against a distal end portion 118d of the cover member in the proximal direction and push it through a lumen of the tubular member 146.

FIG. 15 shows the proximal end portion 116p of the expandable member and the inverted cover member 118. At this stage, the distal end portion 118d (not shown) of the cover member has been removed out of the expandable member 116 through the terminal proximal end 132 of the expandable member and is located proximal to the terminal proximal end 132. In addition, after inverting the cover member 118, the first tail 130a and the second tail 130b of the proximal suture are also reverted inside-out but they remain separated from the expandable member 116 by the cover member 118. Also, the temporary knot 135 can be untied and the temporary suture 136 can be removed.

As shown in FIG. 15, after inverting the cover member 118, the segment 124 of the proximal end portion 118p, which previously extends outside the expandable member 116, can be folded distally (i.e., to the left in the orientation depicted in FIG. 15) so as to cover at least a portion of the proximal end portion 116p of the expandable member. As a result, the terminal proximal edge 134 of the cover member surrounds an outer surface of the expandable member 116 at a location distal to the terminal proximal end 132 of the expandable member. By folding the segment 124 backwardly, the segment 124 can also cover the stitch loop 140, the first knot 142, the second knot 144, and the wrap 143 located at the terminal proximal end 132 of the expandable member. Thus, the wrap 143 can also be referred to as an "inner wrap," and the first and second knots 142, 144 can also be referred to as "inner knots."

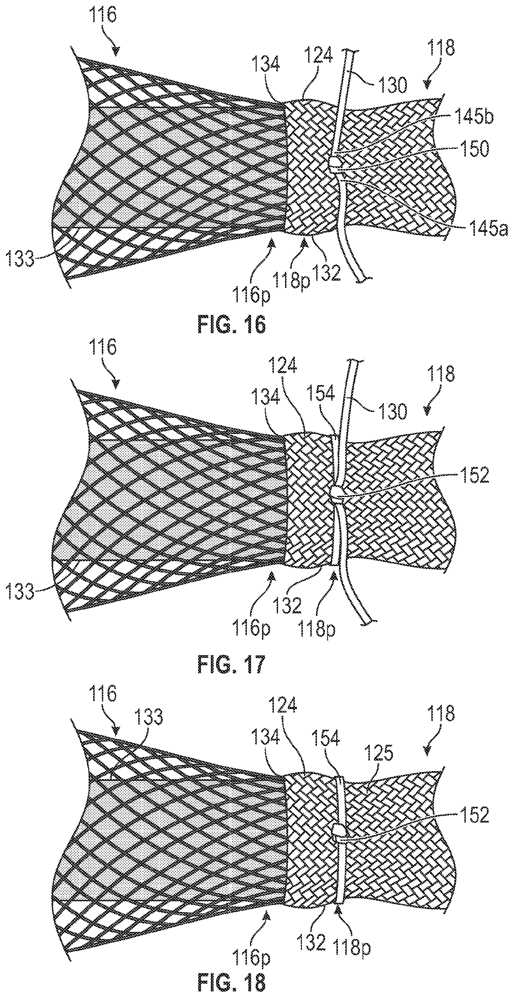

As shown in FIG. 16, a third knot 150 can be tied on the cover member 118 using the proximal suture 130. To ensure the tightness of the third knot 150, the mandrel 133 (or a different mandrel) can be inserted into the lumen of the expandable member 116 and extends through its terminal proximal end 132. Because the first and second tails 130a, 130b of the proximal suture respectively exit the cover member 118 at the first and second exit locations 145a, 145b, the third knot 150 can be located between the first and second exit locations 145a, 145b, both of which are adjacent to the terminal proximal end 132 of the expandable member. In the depicted example, the third knot 150 is a square knot. In other examples, the third knot 150 can be a single knot, a double knot, or other type of knot.

Referring to FIG. 17, the proximal suture 130 can be wrapped around the cover member 118 and then tie one or more knots 152 on opposite side of the third knot 150. In one example, the one or more knots 152 include one single knot and one double knot. In another example, the one or more knots 152 include only one single knot or one double knot. In other examples, the one or more knots 152 can include more than two knots and/or other types of knot. In the depicted example, the one or more knots 152 are connected to the third knot 150 by one wrap 154. In other examples, the one or more knots 152 can be connected to the third knot 150 by more than one wrap.

The knots 150, 152 and the wrap 154 are all disposed on the cover member 118 at locations adjacent and slightly distal to the terminal proximal end 132 of the expandable member. Thus, the wrap 154 can be tied to a slightly smaller diameter than the diameter of the annular segment 124 which is folded over the proximal end portion 116p of the expandable member. The diameter of the annular segment 124 is about the diameter of the terminal proximal end 132 of the expandable frame plus two times the thickness of the segment 124. In one example, the diameter of the wrap 154 can be about the same as, or slightly smaller than, the diameter of the terminal proximal end 132 of the expandable frame.

As shown in FIG. 18, both the first tail 130a and the second tail 130b of the proximal suture can be trimmed (e.g., with scissors or a blade), leaving each tail about 2.5mm-3mm from the knots 152. As shown, the knots 150, 152 and the wrap 154 are all disposed on the cover member 118 and do not directly contact the expandable member 116. Thus, the knots 150 and 152 can also be referred to as "outer knots," and the wrap 154 can also be referred to as an "outer wrap." In other words, the segment 124 of the cover member 118 can separate the outer knots 150, 152 and the outer wrap 154 from the inner knots 142, 144 and the inner wrap 143.

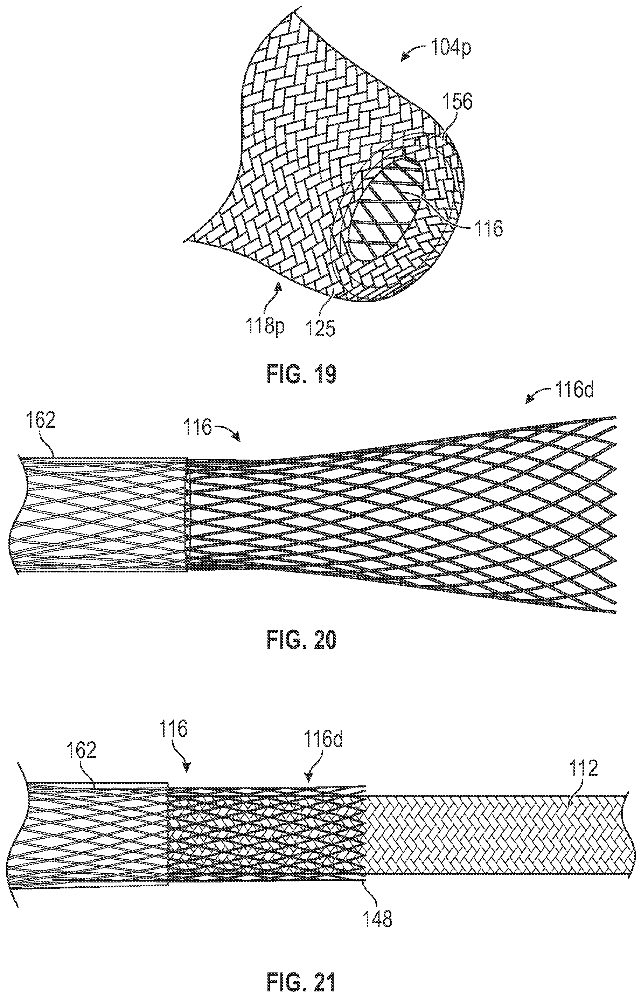

Then, the cover member 118 can be folded distally (i.e., the distal end portion 118d of the cover member can be folded to the left in the orientation depicted in FIG. 18) to cover the outer surface of the expandable member 116. Folding the cover member 118 backwardly can create a fold 156 at the terminal proximal end 132 of the expandable member 116. FIG. 19 shows the attached proximal end portion 104p of the guard member 104. In the depicted example, the outer knots 150, 152 and the outer wrap 154 are disposed within the fold 156. In some examples, the fold 156 can have about the same diameter as the terminal proximal end 132 of the expandable member. In certain examples, the fold 156 and the outer wrap 154 disposed therein can abut the terminal proximal end 132 of the expandable member. Thus, as shown in FIG. 19, the fold 156 formed at the proximal end portion 118p of the cover member can cover the terminal proximal end 132 of the expandable member. In this manner, the outer wrap 154 can, for example, act as a protective barrier between the cover member 118 and the terminal proximal end of the expandable member 116.

By folding the cover member 118 backwardly, the segment 124 of the cover member is covered by another segment 125 of the cover member. Thus, the proximal end portion 118p of the cover member can form a double layer over the proximal end portion 116p of the expandable member. The segment 124 can be referred to as an "inner layer" or "inner portion" and the segment 125 can be referred to as an "outer layer" or "outer portion." The segments 124 and 125 are joined at the terminal proximal end 132 of the expandable member to define the fold 156. As described above, the inner knots 142, 144 and the inner wrap 143 directly contact the expandable member 116 and are disposed inside the segment 124, whereas the outer knots 150, 152 and the outer wrap 154 are disposed between the double layer formed by the segments 124, 125. Thus, the proximal suture 130 can be fully covered by the cover member 118 and disposed underneath by the segment 125.

After folding the cover member 118 backwardly, the distal end portion 118d of the cover member can be aligned with the distal end portion 116d of the expandable member so that an entire outer surface of the expandable member is covered by the cover member 118. The axial length of the cover member 118 can be configured to be about the same as the axial length of the expandable member 116. The distal end portion 118d of the cover member can then be attached to the distal end portion 116d of the expandable member as well as the tubular member 112, as described more fully below.

While the method described above for assembling proximal end portion of the guard member involves particular steps, it should be understood that some of the steps may be optional and can be omitted, different number of wraps and/or knots can be used, and the sequence of the some of the steps may be changed.

### Exemplary Method of Assembling Distal End Portion of Guard Member

FIGS. 20-42 show a method of fixedly attaching the distal end portion 116d of the expandable member 116 and the distal end portion 118d of the cover member 118 to the tubular member 112 via a distal suture 160, according to one example. Because the tubular member 112 can be fixedly attached to the coil 102, the method described herein can also fixedly couple the distal end portion 116d of the expandable member 116 and the distal end portion 118d of the cover member 118 to the coil 102. In certain examples, the tubular member 112 can be at least partially surrounded by the retention element 114, and the method described herein can be similarly used to fixedly attaching the distal end portion 116d of the expandable member 116 and the distal end portion 118d of the cover member 118 to both the tubular member 112 and the retention element 114 via the distal suture 160. As described herein, the distal suture 160 can comprise an UHMWPE material to give it a high strength and durability. In other examples, the distal suture 160 can comprise PTFE and/or other materials. In one example, the distal suture 160 can be a Force Fiber^{®} suture.

As shown in FIG. 20, a tubular member 162 can slide over the expandable member 116 so that at least a body portion of the expandable member 116 can be restrained within a lumen of the tubular member 162 while the distal end portion 116d of the expandable member can extend outside the tubular member 162. In alternative examples, the tubular member 162 can be replaced by an O-ring, or a temporary suture wrap, and/or other type of restraining member.

Referring to FIG. 21, the expandable member 116 can slide over the tubular member 112 so that the tubular member 112 extends through a lumen of the expandable member 116.

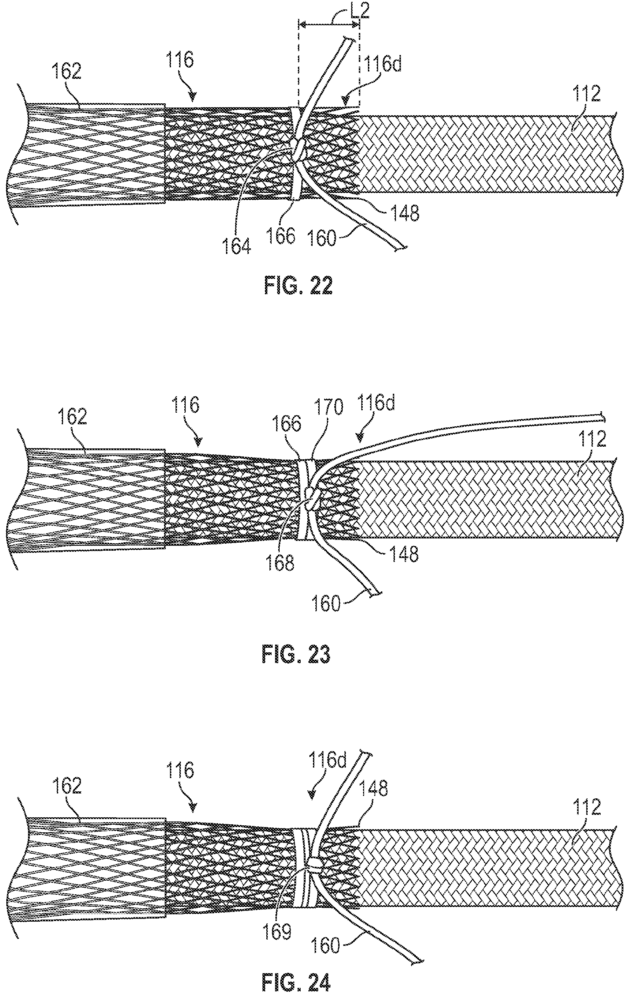

As shown in FIG. 22, the distal suture 160 can be wrapped around the distal end portion 116d of the expandable member and create a first inner knot 164 and a first inner wrap 166, which can tie together the expandable member 116 and the tubular member 112 extending therethrough. In the depicted example, the first inner knot 164 is a double knot. In alternative examples, the first inner knot 164 can be other types of knot, such as a single knot, a square knot, etc. In some examples, the first inner knot 164 is spaced away from the terminal distal end 148 of the expandable member by a distance L2 ranging from about 1mm to about 4mm. In certain examples, the distance L2 is between about 2mm and about 3mm. In one example, the distance L2 is about 2.5mm.

In certain examples, a second inner knot 168 can be tied on opposite side of the first inner knot 164. For example, as shown in FIG. 23, the expandable member 116 and the tubular member 112 extending therethrough can be rotated about a half turn (i.e., about 180 degrees). Then, the distal suture 160 can be wrapped around the distal end portion 116d of the expandable member to create a second inner wrap 170 and tie the second inner knot 168. The second inner wrap 170 can be placed parallel to and in direct contact with the first inner wrap 166. In the depicted example, the second inner knot 168 is a double knot. In alternative examples, the second inner knot 168 can be other types of knot, such as a single knot, a square knot, etc.

In the depicted example, the first and second inner knots 164, 168 are connected to each other by two inner wraps 166, 170 and disposed on opposite sides of the distal end portion 116d of expandable member. In some examples, the second inner knot 168 and the second inner wrap 170 can be optional.

In some examples, there can be more than two (e.g., 3-6) inner wraps (in additional to the inner wraps 166, 170) between the two inner knots 164, 168. In some examples, there can be more inner knots (in addition to the inner knots 164, 168) tied on the distal end portion 116d of the expandable member. For example, FIG. 24 shows that a third inner knot 169 can be tied on opposite side of the second inner knot 168. In the depicted example, the third inner knot 169 is a square knot. In alternative examples, the third inner knot 169 can be other types of knot, such as a single knot, a double knot, etc. In some examples, the third inner knot 169 can be optional.

As described herein, the inner knots 164, 168, 169 and the inner wraps 166 and 170 can secure the distal end portion 116d of the expandable member to the tubular member 112 extending therethrough. The tubular member 162 can thus be removed.

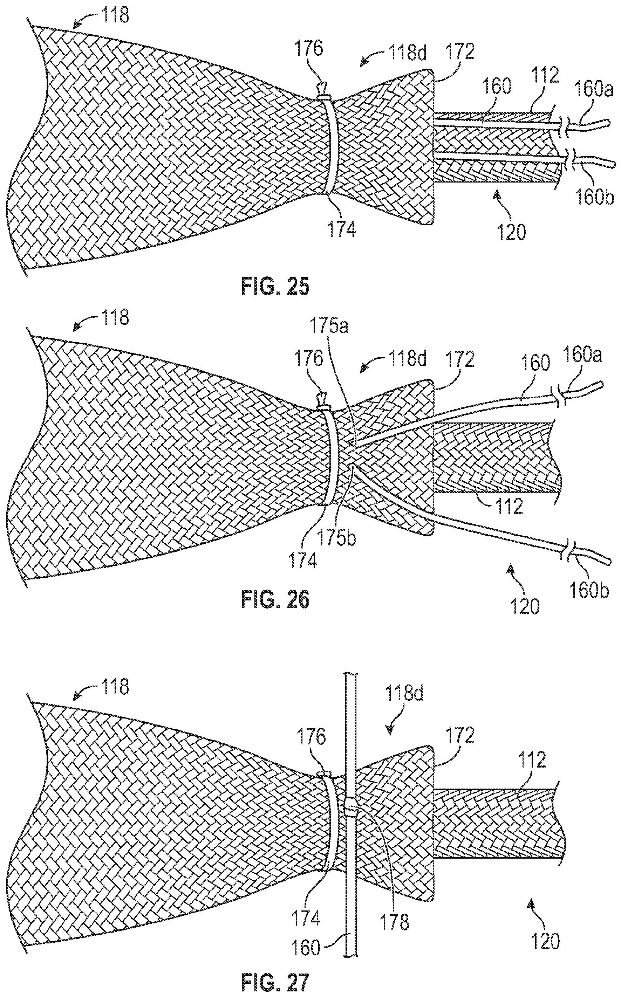

As shown in FIG. 25, the distal end portion 118d of the cover member (which has been folded backwardly as described above) can be aligned with the distal end portion 116d of the expandable member. For example, a terminal distal edge 172 of the cover member can be aligned with the terminal distal end 148 of the expandable member. The terminal distal edge 172 of the cover member can be sealed (e.g., via heating, sealant, or other means) to prevent it from fraying and/or unraveling.

A temporary suture 174 can be wrapped around the distal end portion 118d of the cover member and create a temporary knot 176 that tightens the distal end portion 118d of the cover member and the distal end portion 116d of the expandable member to the tubular member 112 extending through the expandable member. The temporary knot 176 can be positioned adjacent to the first and second inner wraps 166, 170. As described herein, the knots 164, 168, 169 are referred to as "inner knots" and the wraps 166, 170 are referred to as "inner wraps" because they are all covered by the cover member 118. Specifically, the inner knots 164, 168, 169 and the inner wraps 166, 170 are in direct contact with the expandable member 116 and an inner surface of the cover member.

The distal suture 160 can have a first tail 160a and a second tail 160b. As shown in FIG. 26, both tails 160a, 160b can be transferred or routed from inside of the cover member 118 to outside of the cover member 118. For example, the first tail 160a can be connected to a needle (such as the needle 158 described below, which can be the same as or different from the needle 138 described above). The needle and the first tail 160a connected thereto can then extend through the distal end portion 118d of the cover member at a first exit point 175a. The needle can then be disconnected from the first tail 160a and connected to the second tail 160b (alternatively, a different needle can be connected to the second tail 160b). Then the needle and the second tail 160b connected thereto can extend through the distal end portion 118d of the cover member at a second exit point 175b. The first and second exit points 175a, 175b can be slightly spaced away from each other (e.g., separated by one, two, or more strands of yarns or filaments of the cover member).

As shown in FIG. 27, an outer knot 178 can be tied on the distal end portion 118d of the cover member. Because the first and second tails 160a, 160b of the distal suture respectively exit the cover member 118 at the first and second exit points 175a, 175b, the outer knot 178 can be located between the first and second exit points 175a, 175b, both of which are adjacent to the temporary suture 174 wrapped around the cover member. In the depicted example, the outer knot 178 is a single knot. In alternative examples, the outer knot 178 can be a double knot, a square knot, or other types of knot. The outer knot 178 can be referred to as an "outer knot" because it is located outside the cover member 118 and does not directly contact the expandable member 116.

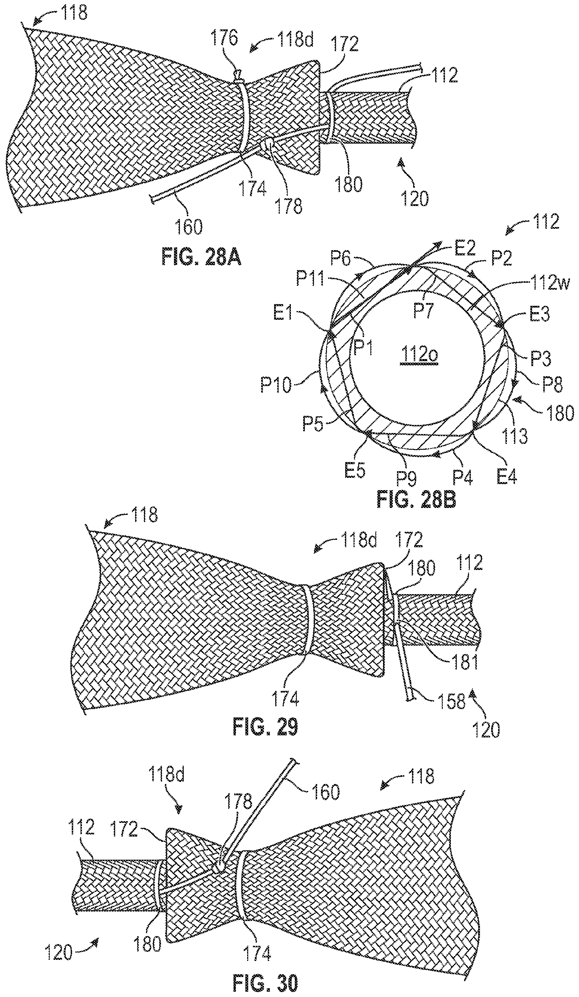

Referring to FIG. 28A, a locking loop 180 can be secured to the tubular member 112 at a location that is adjacent and slightly distal to the terminal distal edge 172 of the cover member. In certain examples, a distance between the locking loop 180 and the terminal distal end 148 of the expandable member can range from about 0.5mm to about 2mm. In one example, the distance between the locking loop 180 and the terminal distal end 148 of the expandable member is about 1mm. As described herein, the locking loop 180 can be secured to the tubular member 112 by operating only one tail (e.g., 160a or 160b) of the distal suture 160 while leaving the other tail (e.g., 160b or 160a) of the distal suture 160 free.

In the depicted example, the locking loop 180 can be formed by creating a lasso stitch around the tubular member 112, as illustrated in FIG. 28B. As shown, the tubular member 112 can have a tubular shape, comprising a lumen 112o and a circumferential wall 112w surrounding the lumen 112o. The lasso stitch can include a plurality of in-and-out stitches around the circumferential wall 112w of the tubular member 112. Specifically, the free end of the distal suture 160 can be stitched into and out of an outer surface 113 of the tubular member 112 following the paths indicated by the arrows P1-P11: the free end of the distal suture 160 can enter the wall 112w at a first location E1; extend along the path P1; exit the wall 112w at a second location E2; wrap around the outer surface 113 along the path P2; enter the wall 112w at a third location E3; extend along the path P3; exit the wall 112w at a fourth location E4; wrap around the outer surface 113 along the path P4; enter the wall 112w at a fifth location E5; extend along the path P5; exit the wall 112w adjacent the first location E1; wrap around the outer surface 113 along the path P6; enter the wall 112w adjacent the second location E2; extend along the path P7; exit the wall 112w adjacent the third location E3; wrap around the outer surface 113 along the path P8; enter the wall 112w adjacent the fourth location E4; extend along the path P9; exits the wall 112w adjacent the fifth location E5; wrap around the outer surface 113 along the path P10; enter the wall 112w adjacent the first location E1; extend along the path P11; and finally exit the wall 112w adjacent the second location E2.

Following the above stitching sequence, the distal suture 160 can have some inner segments (e.g., along the paths P1, P3, P5, P7, P9, and P11) embedded within the wall 112w and some outer segments (e.g., along the paths P2, P4, P6, P8, and P10) disposed on the outer surface 113 of the tubular member 112. In addition, the distal suture 160 can form two loops around the circumferential wall 112w of the tubular member 112: the first loop is formed by suture paths P1-P5 and the second loop is formed by suture paths P6-P10. The first and second loops are so interlaced that that an inner segment in the first loop is paired with an outer segment in the second loop and vice versa (e.g., pairs P1-P6, P2-P7, P3-P8, P4-P9, and P5-P10). Further, the outer segments of the two loops (e.g., P2, P4, P6, P8, and P10) can jointly form a complete wrap (i.e., about 360 degrees circumferentially) over the outer surface 113 of the tubular member 112.

It is to be understood that the lasso stitch shown in FIG. 28B is merely illustrative, and many variants can be used to create the lasso stitch based on the same principle disclosed herein. For example, the number of enter/exit locations (e.g., E1-E5) can be more or less than 5, and the number of paths (e.g., P1-P11) can be more or less than 11, resulting in more or less inner and/or outer segments.

In alternative examples, the locking loop 180 can be created in the form of a lasso knot or other types of knot (e.g., hitch knot, noose knot, etc.) that wraps around the tubular member 112. It is to be understood that any other locking mechanisms can be used to create the locking loop 180. For example, the locking loop 180 can be simply created by wrapping the distal suture 160 around the tubular member 112 to form a wrapped loop, and then stitch or glue the wrapped loop together with the tubular member 112."

Referring to FIG. 29, the tail forming the locking loop 180 can be embedded within the locking loop 180. For example, the tail forming the locking loop 180 can be connected to the needle 158. The needle 158 can then stitch through the locking loop 180 so that a tail portion of the distal suture 160 can be threaded through the locking loop 180. In the depicted example, one tail (e.g., 160a) is connected to the needle 158, which creates a first stitch 181 splitting strands of yarns or filaments of the distal suture 160 forming the locking loop 180, through the tubular member 112, and exiting on a proximal side of the locking loop 180. In another example, the stitch 181 can be the last in-and-out stitch forming the lasso stitch (e.g., the stitch entering the wall 112w adjacent the first location E1, extending along the path P11, and exiting the wall 112w adjacent the second location E2, as shown in FIG. 28B). The tail (e.g., 160a) can then be trimmed (e.g., with scissors or a blade) at the exiting site so that it is embedded within and hidden inside the locking loop 180.

Then, a plurality of wraps can be formed between the outer knot 178 and the locking loop 180, as described below. These wraps can also be referred to as "outer wraps" because they are not hidden underneath the cover member 118. Note that in FIGS. 30-41, the cover assembly 120 (including the cover member 118, the expandable member 116, and the tubular member 112) is shown in a horizontally flipped position compared to FIG. 29.

FIG. 30 shows the cover assembly 120 after forming the locking loop 180 around the tubular member 112 at a location adjacent the terminal distal edge 172 of the cover member. As described above, one tail (e.g., 160a) can be hidden inside the locking loop 180 and the other tail (e.g., 160b) remains free.

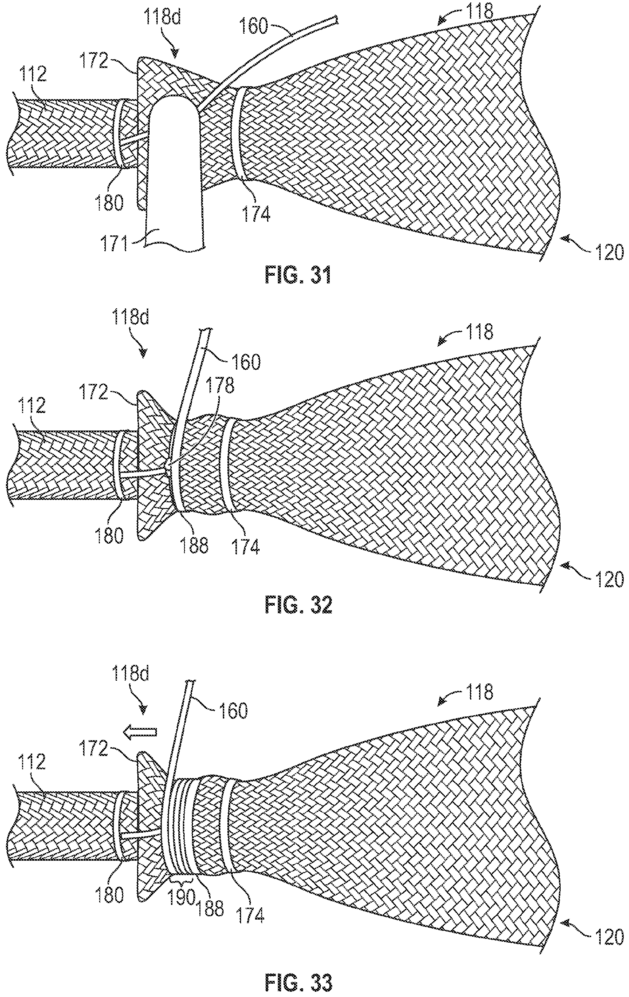

FIG. 31 shows an optional step where the outer knot 178 can be smoothed by pressing it against the cover member, for example, by using flat forceps 171 and/or another tool configured for compressing.

Referring to FIG. 32, by holding the free tail (e.g., 160b), the distal suture 160 can be wrapped around the cover member 118 to create a first outer wrap 188 adjacent the outer knot 178. In the depicted example, the first outer wrap 188 is located on the proximal side of the outer knot 178. In alternative example, the first outer wrap 188 can be located on the distal side of the outer knot 178. In yet another example, the first outer wrap 188 can be optional.

Referring to FIG. 33, after creating the first outer wrap 188, the distal suture 160 can be wrapped around the cover member 118 to create a first set of distal wraps 190 traveling in the distal direction as indicated by the left-pointed arrow (i.e., traveling from the outer knot 178 toward the locking loop 180). In the depicted example, the first set of distal wraps 190 crosses over the outer knot 178 and are positioned distal to the outer knot 178. The first set of distal wraps 190 can be tightly wound such that there is no gap between any two adjacent wraps. In certain examples, the first set of distal wraps 190 can contain between about 3 and about 12 wraps. In the depicted example, the first set of distal wraps 190 contains about 5-7 wraps.

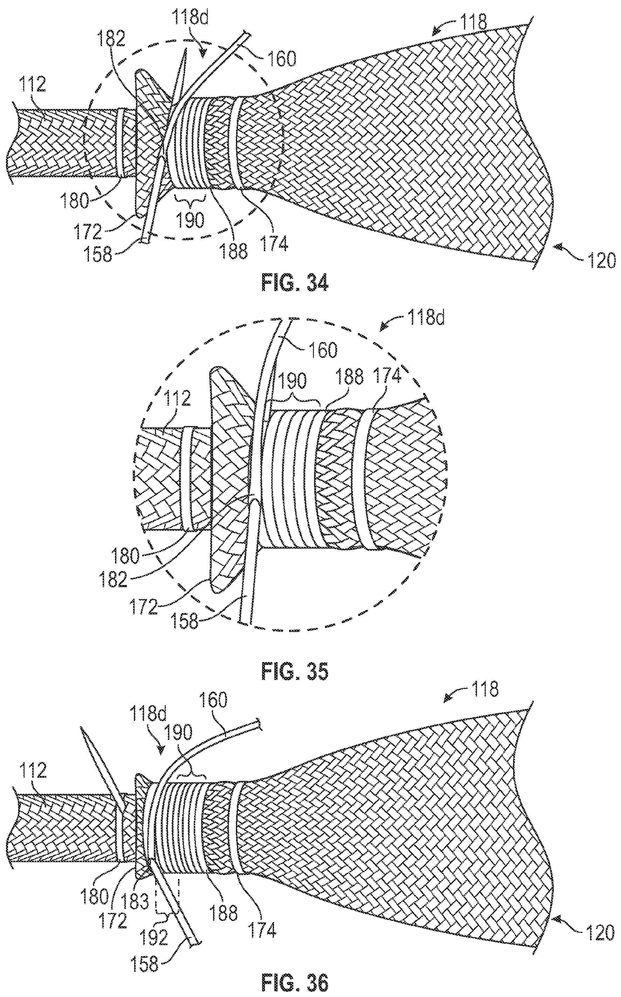

Referring to FIGS. 34-35, after creating the first set of distal wraps 190, a second stitch 182 can be created by threading the free tail (e.g., 160b) of the distal suture 160 through the first set of distal wraps 190 using the needle 158. In the depicted example, the second stitch 182 splits between two strands of yarns or filaments of the distal suture 160 at the most distal one of the first set of distal wraps 190. In certain examples, the second stitch 182 does not split between strands of yarns or filaments of the distal suture 160. Instead, the second stitch 182 can split between two adjacent wraps of the first set of distal wraps 190. The second stitch 182 can extend through the cover member 118 and fixedly couple the first set of distal wraps 190 to the cover member 118. In certain examples, the second stitch 182 can also extend through the expandable member 116 and/or the tubular member 112. In the depicted example, the second stitch 182 extends through the tubular member 112, the expandable member 116, as well as the cover member 118. In certain examples, the second stitch 182 can be optional.

Referring to FIG. 36, after creating the first set of distal wraps 190 and optionally the second stitch 182, the distal suture 160 can be wrapped around the cover member 118 to create a second set of distal wraps 192 traveling in the distal direction (i.e., traveling from the most distal wrap of the first set of distal wraps 190 toward the locking loop 180). As shown, the second set of distal wraps 192 can be positioned distal to the first set of distal wraps 190 and there is no gap between the first and set sets of distal wraps 190, 192. Similarly, the second set of distal wraps 192 can be tightly wound such that there is no gap between any two adjacent wraps. In certain examples, the second set of distal wraps 192 can contain between about 2 and about 6 wraps. In the depicted example, the second set of distal wraps 192 contains about 4 wraps.

Still referring to FIG. 36, after creating the first set of distal wraps 190, a third stitch 183 can be created by threading the free tail (e.g., 160b) of the distal suture 160 through the second set of distal wraps 192 and the locking loop 180 using the needle 158. In the depicted example, the third stitch 183 extends diagonally relative to the axial axis of the tubular member 112. Specifically, the third stitch 183 can first extend through the most distal one of the second set of distal wraps 192 and then exits from the locking loop 180 (e.g., by splitting between strands of yarns or filaments of the distal suture forming the locking loop 180). In certain examples, the third stitch 183 can exit at the proximal or distal side of the locking loop 180. Similar to the second stitch 182, the third stitch 183 can split between strands of yarns or filaments of the distal suture at one of the second set of distal wraps 192, or split between two adjacent wraps of the second set of distal wraps 192. The third stitch 183 can extend through the cover member 118 and fixedly couple the second set of distal wraps 192 to the cover member 118. In certain examples, the third stitch 183 can also extend through the expandable member 116 and/or the tubular member 112. In the depicted example, the third stitch 183 extends through the tubular member 112, the expandable member 116, as well as the cover member 118. In certain examples, the second set of distal wraps 192 and/or the third stitch 183 can be optional.

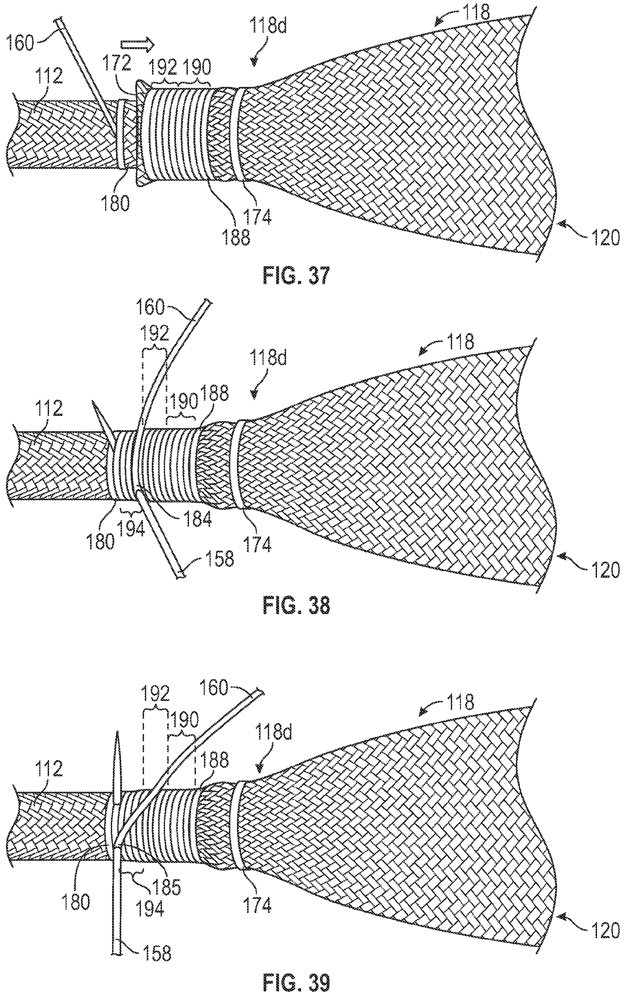

As shown in FIG. 37, after creating the second set of distal wraps 192 and the third stitch 183, the distal suture 160 can be wrapped around the tubular member 112 and the cover member 118 to create a third set of distal wraps 194 traveling in the proximal direction as indicated by the right-pointed arrow (i.e., traveling from the locking loop 180 toward the second set of distal wraps 192). As noted above, the locking loop 180 is secured around the tubular member 112 at a location that is adjacent and slightly distal to the terminal distal edge 172 of the cover member. Thus, the third set of distal wraps 194 may be initially only wrapped around the tubular member 112 until they reach the terminal distal edge 172 of the cover member, from where the third set of distal wraps 194 are wrapped around the cover member 118.

As shown in FIG. 38, the third set of distal wraps 194 can be positioned distal to the second set of distal wraps 192. Specifically, the third set of distal wraps 194 can extend from the locking loop 180 to a distal end of the second set of distal wraps 192 and there is no gap between the second and third sets of distal wraps 192, 194. Similarly, the third set of distal wraps 194 can be tightly wound such that there is no gap between any two adjacent wraps. In certain examples, the third set of distal wraps 194 can contain between about 2 and about 6 wraps. In the depicted example, the third set of distal wraps 194 contains about 4-5 wraps (though fewer (e.g., 1-3) or more (e.g., 6-12) wraps can be used in other examples).

Still referring to FIG. 38, after creating the third set of distal wraps 194, a fourth stitch 184 can be created by threading the free tail (e.g., 160b) of the distal suture through the third set of distal wraps 194 using the needle 158. In the depicted example, the fourth stitch 184 extends diagonally relative to the axial axis of the tubular member 112. Specifically, the fourth stitch 184 can first extend from a proximal end portion of the third set of distal wraps 194 and then exits at a distal end portion of the third set of distal wraps 194. In some examples, the fourth stitch 184 can exit from the third set of distal wraps 194 at a location that is about 0.3mm-0.6mm, or specifically about 0.5mm, from the locking loop 180. In certain examples, the fourth stitch 184 can split between strands of yarns or filaments of the distal suture or split between two adjacent wraps of the third set of distal wraps 194. The fourth stitch 184 can extend through the cover member 118 and fixedly couple the third set of distal wraps 194 to the cover member 118. In certain examples, the fourth stitch 184 can also extend through the expandable member 116 and/or the tubular member 112. In the depicted example, the fourth stitch 184 extends through the tubular member 112, the expandable member 116, as well as the cover member 118. In certain examples, the third set of distal wraps 194 and/or the fourth stitch 184 can be optional.

Referring to FIG. 39, after creating the third set of distal wraps 194 and optionally the fourth stitch 184, a fifth stitch 185 can be created by threading the free tail (e.g., 160b) of the distal suture through the third set of distal wraps 194 using the needle 158. In the depicted example, the fifth stitch 185 extends vertically relative to the axial axis of the tubular member 112 and is located at the distal end portion of the third set of distal wraps 194 (and proximal to the locking loop 180). In some examples, the fifth stitch 185 can be oriented diagonally relative the axial axis of the tubular member 112. In certain examples, the fifth stitch 185 can split between strands of yarns or filaments of the distal suture or split between two adjacent wraps of the third set of distal wraps 194. The fifth stitch 185 can extend through the cover member 118. In certain examples, the fifth stitch 185 can also extend through the expandable member 116 and/or the tubular member 112. In the depicted example, the fifth stitch 185 extends through the tubular member 112, the expandable member 116, and the cover member 118. In certain examples, the fifth stitch 185 can be optional.

Referring to FIG. 40, a sixth stitch 186 can be created by threading the free tail (e.g., 160b) of the distal suture through the third set of distal wraps 194 using the needle 158. The sixth stitch 186 can be parallel to the fifth stitch 185. In one example, the sixth stitch 186 can extend in the same direction as the fifth stitch 185. In another example, the sixth stitch 186 can extend in opposite direction relative to the fifth stitch 185. Similarly, the sixth stitch 186 can split between strands of yarns or filaments of the distal suture or split between two adjacent wraps of the third set of distal wraps 194. The sixth stitch 186 can extend through the cover member 118. In certain examples, the sixth stitch 186 can also extend through the expandable member 116 and/or the tubular member 112. In the depicted example, the sixth stitch 186 extends through the tubular member 112, the expandable member 116, and the cover member 118. In certain examples, the sixth stitch 186 can be optional.

Then, as shown in FIG. 41, the temporary knot 176 and the temporary suture 174 can be removed. The free tail (e.g., 160b) of the distal suture can be trimmed by using scissors or a blade, e.g., at the exit of the sixth stitch. Optionally, the trimmed free tail can be sealed (e.g., by applying a soldering iron thereto).

As noted above, the stitches 182, 183, 184, 185, and 186 can fixedly couple respective wraps to the cover member 118. Thus, these stitches can also be referred to as "locking stitches."

In addition, the combined first and second sets of distal wraps 190, 192 can be referred to as a "first sequence of outer wraps" because both sets of wraps travel in the distal direction and are not hidden under the cover member. The third set of distal wraps 194 can also be referred to as a "second sequence of outer wraps" because they travel in the proximal direction (i.e., opposite the direction of the first sequence of outer wraps) and are not hidden under the cover member. As noted above, a distal end of the first sequence of the outer wraps (i.e., the most distal one of the second set of distal wraps 192) can abut a proximal end of the second sequence of the outer wraps (i.e., the most proximal one of the third set of distal wraps 194).

FIG. 42 shows the attached distal end portion 104d of the guard member 104. As described above, the distal end portion 118d of the cover member, the distal end portion 116d of the expandable member 116, and the tubular member 112 can be securely coupled together by the outer knot (e.g., 178), the locking loop 180, the outer wraps (e.g., 188, 190, 192, and 194), as well as the inner wraps (e.g., 166 and 170) and inner knots (e.g., 164, 168, and 169).

After both the proximal end portion 104p and the distal end portion 104d of the guard member are assembled as respectively shown in FIG. 19 and FIG. 42, the docking device 100 can be assembled by integrating the cover assembly 120 with the coil 102, for example, by inserting the coil 102 through an inner lumen of the tubular member 112.

**In** some examples, the total number of outer wraps (including the first outer wrap 188, the first set of distal wraps 190, the second set of distal wraps, 192, and the third set of distal wraps 194) can range from about 5 to about 25. **In** some examples, the total number of outer wraps ranges from about 10 to about 20. **In** one particular example, the total number of wraps ranges from about 13 to about 16. Optionally, each of the outer wraps can be smoothed by pressing it with flat forceps.

In some examples, the total number of stitches applied to the distal end portion 104d of the guard member can range from about 3 to about 6. In the depicted example, six stitches 181-186 are applied to securely lock the outer knot (e.g., 178) and the outer wraps (e.g., 188, 190, 192, and 194) to the cover member 118, the expandable member 116, and/or the tubular member 112. As described above, all six stitches 181-186 can be confined in a region between the outer knot 178 and the locking loop 180. In other words, none of the stitches 181-186 extends through any portion of the expandable member 116 that is uncovered by the outer wraps (e.g., 188, 190, 192, and 194) and/or the outer knot (e.g., 178). Thus, any stitch that potentially scratches the surface of the metal or metal alloy of the wire frame of the expandable member 116 is limited to that region, where the wire frame is tightly constrained and protected by the outer wraps (e.g., 188, 190, 192 and 194) and/or the outer knot (e.g., 178) and not exposed to the bodily fluid. Since none of the stitches extends proximal to the outer knot 178, no scratching damage can occur to a body portion of the wire frame extending from the outer knot 178 to the terminal proximal end 132 of the expandable member. Thus, the risk of corrosion of the wire frame of the expandable member can be reduced.

In some examples, a distance L3 between the outer knot 178 and the locking loop 180 can range from about 1mm to about 5mm. In some examples, the distance L3 is between about 2mm and about 4mm. In the depicted example, the distance L3 is between about 3mm and about 3.5mm.

In some examples, when the expandable member 116 is in the radially expanded state, a length (L4) of the guard member 104, measured between the terminal proximal end 132 or fold 156 of the expandable member (see e.g., FIG. 19) and the locking loop 180, can range from about 30mm to about 160mm. In some examples, the length L4 is between about 140mm and about 150mm. In some examples, the length L4 is between about 40mm and about 60mm. In the depicted example, the length L4 is between about 50mm and about 52mm.

In some examples, the maximum outer diameter (OD) of the outer knot (e.g., 178) and the outer wraps (including the wraps 188, 190, 192, and 194) is smaller than about 4mm. In certain examples, the maximum OD of the outer knot and the outer wraps is smaller than about 3mm. In the depicted example, the maximum OD of the outer knot and the outer wraps is between about 2.4mm and about 2.5mm.

### Exemplary Method of Attaching Retention Element to Tubular Member

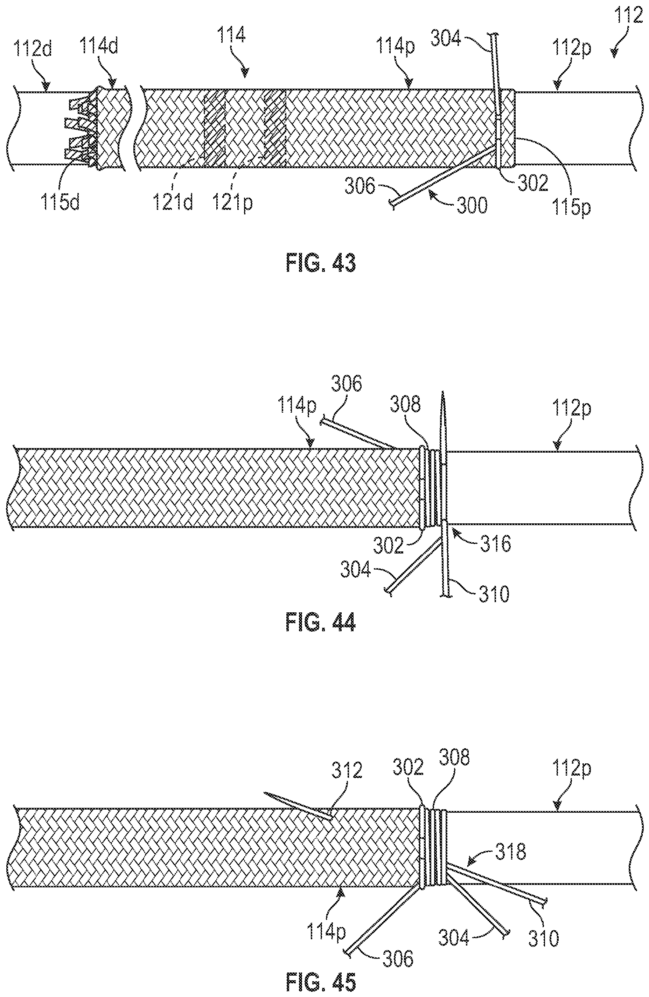

FIGS. 43-59 illustrate a method of attaching the retention element 114 to the tubular member 112, according to one example. In the depicted examples, the retention element 114 comprises braided fibers.

Generally, a proximal end portion 114p of the retention element 114 can be fixedly attached to a proximal portion 112p of the tubular member 112 and a distal end portion 114d of the retention element 114 can be fixedly attached to a distal portion 112d of the tubular member 112. Specifically, the proximal end portion 114p of the retention element 114 can be fixedly attached to the proximal portion 112p of the tubular member 112 via a proximal suture 300 and the distal end portion 114d of the retention element 114 can be fixedly attached to the distal portion 112d of the tubular member 112 via a distal suture 330. In certain examples, the proximal suture 300 and/or the distal suture 330 can be Force Fiber^{®} sutures. In other examples, the proximal suture 300 and/or the distal suture 330 can comprise PTFE and/or other materials.

As depicted in FIG. 43, the retention element 114 can slide over the tubular member 112. In certain examples, a mandrel can be inserted into a lumen of the tubular member 112 before placing the retention element 114 over the tubular member 112. In some instances, one end of the retention element 114 can be trimmed (e.g., via scissors) and sealed (e.g., via soldering iron, or a sealant, or other means) to prevent fraying of the braided fibers, whereas another end of the retention element 114 can remain unsealed. For example, a proximal end 115p of the retention element 114 can be sealed and a distal end 115d of the retention element 114 can be unsealed.

In the depicted example, a plurality of radiopaque markers are disposed on the tubular member 112. For example, two radiopaque seating markers 121p, 121d can be disposed on the proximal portion of the tubular member 112 and function as the proximal and distal seating markers, respectively, as described above. Additional radiopaque markers can be placed between the proximal and distal seating markers 121p, 121d, and/or in other portions (e.g., the middle) of the tubular member 112. In the depicted example, the proximal and distal seating markers 121p, 121d are covered by the retention element 114.

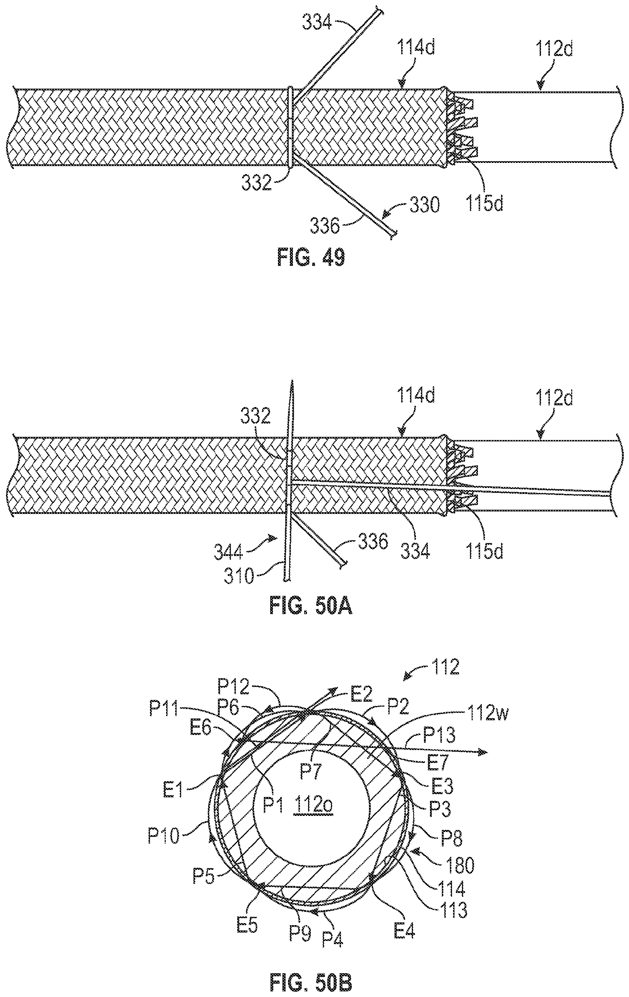

Still referring to FIG. 43, the proximal suture 300 can fixedly attach the proximal end portion 114p of the retention element 114 to the proximal portion 112p of the tubular member 112 via a proximal lasso stitch 302. The proximal lasso stitch 302 can be formed in a similar manner as illustrated in FIG. 28B, except the retention element 114 forms an outer layer surrounding the tubular member 112. Specifically, FIG. 50B illustrates stitching patterns for the proximal lasso stitch 302 and distal lasso stitch 332 (described below), which are similar to the stitching patterns depicted in FIG. 28B, except that (i) the outer surface 113 of the tubular member in FIG. 28B is covered by the retention element 114 in FIG. 50B, and (ii) two additional paths P12 and P13 are added to illustrate a "back stitch" associated with the distal lasso stitch 332 (described below). As shown, the proximal lasso stitch 302 can include a plurality of in-and-out stitches through both the circumferential wall 112w of the tubular member 112 and the retention element 114. As a result, the proximal suture 300 can have some inner segments (e.g., along the paths P1, P3, P5, P7, P9, and P11) embedded within the circumferential wall 112w of the tubular member 112 and some outer segments (e.g., along the paths P2, P4, P6, P8, and P10) disposed on the outer surface of the retention element 114.

In some examples, the proximal lasso stitch 302 can be spaced away from the proximal end 115p of the retention element 114 by a distance ranging from about 0.5 mm to about 2.5 mm. In one specific example, the distance between the proximal lasso stitch 302 and the proximal end 115p is about 1.5 mm.

The proximal lasso stitch 302 can have a first tail 304 and a second tail 306. The first tail 304 can be formed by an end portion of the proximal suture 300 leaving outside the retention element 114 after creating the first in-and-out stitch (e.g., along the path P1 in FIG. 28B). The second tail 306 can be formed by another end portion of the proximal suture 300 exiting the last in-and-out stitch (e.g., along the path P11 in FIG. 28B). In one example, the first tail 304 is shorter than the second tail 306. In another example, the first tail 304 is longer than the second tail 306.

As shown in FIG. 44, a plurality of wraps 308 can be created on the proximal side of the proximal lasso stitch 302 by wrapping around the retention element 114 and the tubular member 112 using the first tail 304 (or alternatively, using the second tail 306) until the proximal end 115p of the retention element 114 is covered by the wraps 308.

Still referring to FIG. 44, one or more locking stitches 316 (similar to 182-186 described above) can be created by threading the first tail 304 through the last wrap (i.e., the most proximal wrap of the plurality of wraps 308) using a needle 310. In some examples, the locking stitches 316 can split between strands of yarns or filaments of the proximal suture 300 when piecing through the last wrap.

As shown in FIGS. 45-46, the needle 310 can create a diagonal stitch 318 to thread the first tail 304 underneath the retention element 114 (and possibly within the circumferential wall 112w of the tubular member 112). In the depicted example, the needle 310 (connect to the first tail 304) is inserted from the last wrap and exits from the retention element 114 at an exit location 312 that is distal to the proximal lasso stitch 302. A distance between the exit location 312 and the proximal lasso stitch 302 can range from about 1 mm and about 3 mm, e.g., about 2 mm. The first tail 304 can then be pulled taut and trimmed closed to its exit location 312, e.g., using scissors. As a result, the first tail 304 can be hidden underneath the retention element 114. The plurality of wraps 308 can be smoothed, e.g., using forceps.

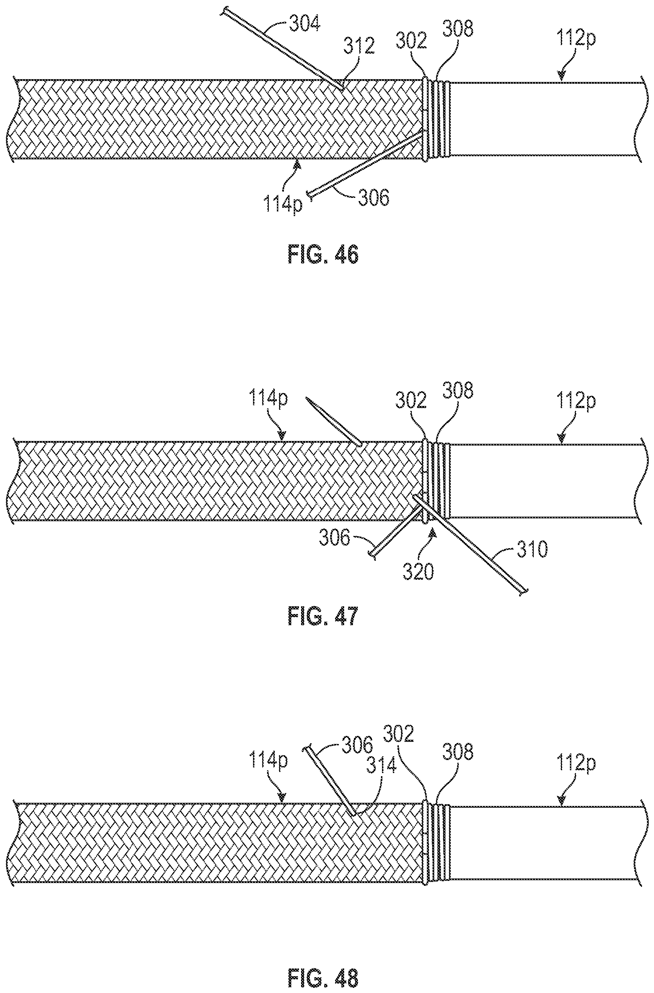

Referring to FIGS. 47-48, a first spiral stitch 320 can be created using the second tail 306 (or alternatively, using the first tail 304 if the second tail 306 is used to create the wraps 308). As shown, the needle 310 (connected to the second tail 306) can be inserted at the base of the second tail 306, i.e., the first entry point (e.g., E1 in FIG. 50B) of the proximal lasso stitch 302, and exit diagonally from the retention element 114 at an exit location 314 that is distal to the proximal lasso stitch 302. A distance between the exit location 314 and the proximal lasso stitch 302 can range from about 0.5 mm and about 1 mm, e.g., about 1 mm. The second tail 306 of the proximal suture 300 can then be used to create additional spiral stitches 320, as described below.

Referring to FIG. 49, the distal suture 330 can fixedly attach the distal end portion 114d of the retention element 114 to the distal portion 112d of the tubular member 112 via a distal lasso stitch 332. The distal lasso stitch 332 can be formed in the same manner as the proximal lasso stitch 302 described above. For example, as shown in FIG. 50B, the distal lasso stitch 332 can include a plurality of in-and-out stitches through both the circumferential wall 112w of the tubular member 112 and the retention element 114 which forms an outer layer surrounding the tubular member 112. As a result, the distal suture 330 can have some inner segments (e.g., along the paths P1, P3, P5, P7, P9, and P11) embedded within the circumferential wall 112w of the tubular member 112 and some outer segments (e.g., along the paths P2, P4, P6, P8, and P10) disposed on the outer surface of the retention element 114. Similar to the proximal lasso stitch 302, the distal lasso stitch 332 can also have a first tail 334 and a second tail 336.

Referring to FIG. 50A, a "back stitch" 344 can be created using the needle 310 after creating the distal lasso stitch 332. As illustrated in FIG. 50B, the back stitch 344 can be created by following two additional sewing paths P12 and P13. Specifically, after the distal suture 330 creates the distal lasso stitch 332, i.e., a free end of the distal suture 330 exits near the second location E2, the free end of the distal suture 330 can be wrapped backward in direction P12 (which is opposite to the direction P11) and overlay a portion of the outer segment along the path P6. Then, the free end of the distal suture 330 can enter the wall 112w at a sixth location E6 located between E1 and E2, extend along the path P13, and exit the wall 112w at a seventh location E7 that is adjacent to E3. As shown, the path P13 can intersect the paths P1, P7, and P11.

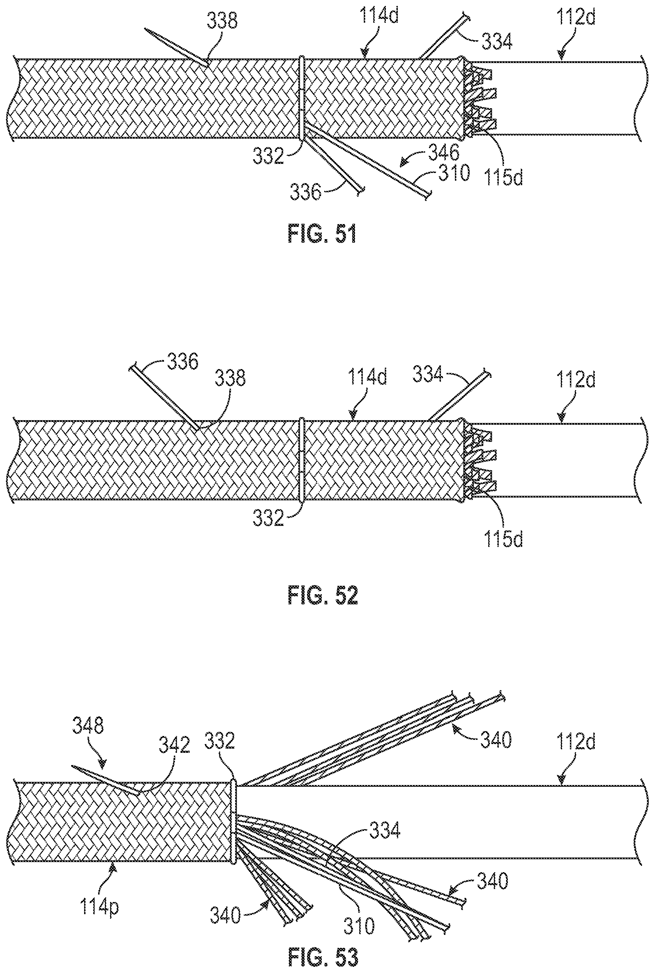

As described herein, the free end used to the create the back stitch 344 can be the first tail 334 or the second tail 336. Assuming the second tail 336 is used to create the back stitch 344, FIGS. 51-52 show a method of hiding the second tail 336 after creating the back stitch 344. As shown, the needle 310 can create a diagonal stitch 346 to thread the second tail 336 underneath the retention element 114 (and possibly within the circumferential wall 112w of the tubular member 112). In the depicted example, the needle 310 (connected to the second tail 336) is inserted from the base of the second tail 336 (i.e., near the last exist location E7 in FIG. 50B) and exits from the retention element 114 at an exit location 338 that is proximal to the distal lasso stitch 332. A distance between the exit location 338 and the distal lasso stitch 332 can range from about 2 mm and about 3 mm, e.g., about 2.5 mm. The second tail 336 can then be pulled taut and trimmed closed to its exit location 338, e.g., using scissors. As a result, the second tail 336 can be hidden underneath the retention element 114.

Referring to FIG. 53, the portion of the retention element 114 located distal to the distal lasso stitch 332 can be unraveled to form a plurality of braid groups 340 (e.g., three braid groups are shown in the depicted example). The first tail 334 can be combined with any of the braid groups 340. In certain examples, a small loop (or hole) can be created on the first tail 334 by splitting strands of yarns or filaments of the first tail 334. The braid group 340 combined with the first tail 334 can be inserted through such small loop (or hole). Thus, by pulling the first tail 334 taut, the small loop (or hole) can be reduced in size so that the braid group 340 and the first tail 334 can be tied together.

Still referring to FIG. 53, the combined first tail 334 and the braid group 340 can be connected to the needle 310, which can create a diagonal stitch 348 to thread the first tail 334 and the combined braid group 340 underneath the retention element 114 (and possibly within the circumferential wall 112w of the tubular member 112). As shown, the needle 310 can be inserted from a distal edge of the distal lasso stitch 332 and exit from the retention element 114 at an exit location 342 that is proximal to the distal lasso stitch 332. A distance between the exit location 342 and the distal lasso stitch 332 can range from about 2 mm and about 3 mm, e.g., about 2.5 mm.

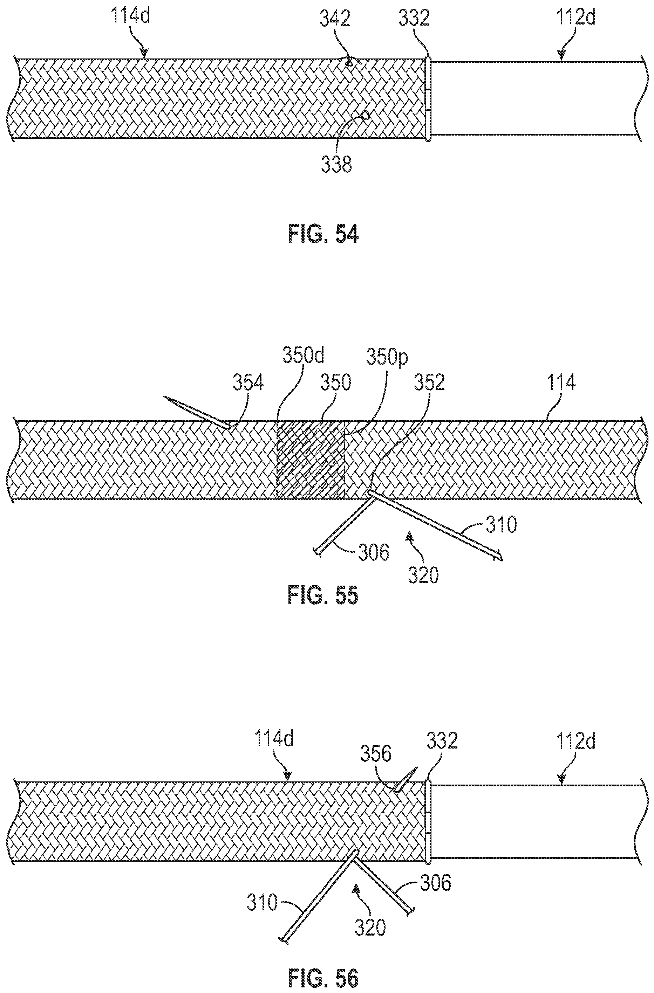

Then, the first tail 334 can be combined with another braid group 340 and repeat the above process until all braid groups 340 are threaded underneath the retention element 114. The first tail 334 and all braid groups 340 can then be trimmed close their exit locations (e.g., 342), e.g., using scissors. As shown in FIG. 54, the end result is that the distal lasso stitch 332 can define the distal end 115d of the retention element 114, and both tails 334, 336 of the distal lasso stitch 332 are hidden underneath the retention element 114.

In certain examples, the second tail 306 of the proximal suture 300 can be used to create additional spiral stitches 320 that extend axially between the proximal end portion 114p and the distal end portion 114d of the retention element 114 and circumferentially around the tubular member 112. Each of the spiral stitches 320 can piece through the retention element 114 and extend within the circumferential wall 112w of the tubular member 112. Through the spiral stitches 320, the retention element 114 can be prevented from sliding or sagging relative to the tubular member 112.

For each additional spiral stitch 320, the needle 310 (connected to the second tail 306 of the proximal suture 300) can be inserted from an entry point adjacent to (e.g., about 0.5 mm apart from) the previous exit location (e.g., the exit location 314 of the first spiral stitch in FIG. 48 is the previous exit location when creating the second spiral stitch), and exit from another exit location that is about 4-6 mm (e.g., about 5 mm) diagonally from and distally relative to the entry point. The tubular member 112 (and the retention element 114) can then be rotated slightly (e.g., a quarter turn, a third turn, a half turn, etc.) before creating the next diagonal stitch 320.

FIG. 55 illustrates threading a spiral stitch 320 across a radiopaque marker 350 (which can be any one of the seating marker 121p, 121d, and any other radiopaque markers) located on the tubular member 112. As shown, the spiral stitch 320 can enter the retention element 114 and the tubular member 112 from an entry point 352 that is proximal to a proximal end 350p of the radiopaque marker 350 and exit the tubular member 112 and the retention element 114 from an exit point 354 that is distal to a distal end 350d of the radiopaque marker 350. As noted above, the distance between the entry point 352 and the exit point 354 can be about 4-6 mm. Depending on the width of the radiopaque marker 350, a distance between the entry point 352 and the proximal end 350p and a distance between the exit point 354 and the distal end 350d can vary. In certain examples, the distance between the entry point 352 and the proximal end 350p can range from about 0.5 mm to about 1 mm. In certain examples, the distance between the exit point 354 and the distal end 350d can range from about 0.5 mm to about 1 mm.

The steps of creating spiral stitches 320 described above can be continued until an exit location 356 of a spiral stitch 320 is adjacent to (e.g., about 0.5 mm) from the distal lasso stitch 332, as illustrated in FIG. 56.

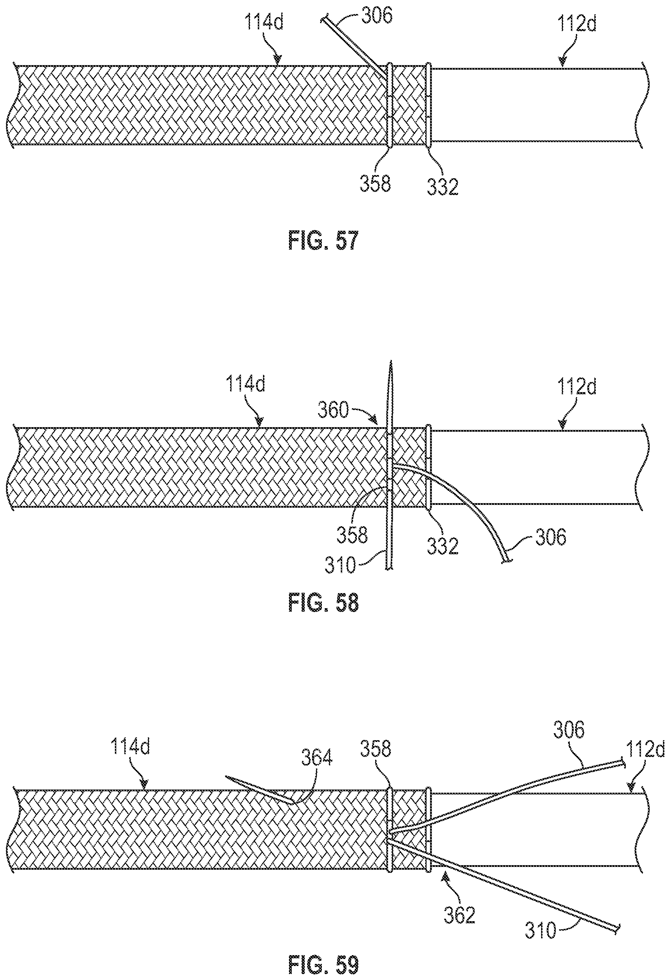

As shown in FIG. 57, another lasso stitch 358 can be created using the second tail 306 of the proximal suture 300 at or adjacent to the exit location 356 of the last spiral stitch 320. The lasso stitch 358 can be formed in the same manner as the distal lasso stitch 332 described above. In certain examples, a distance between the lasso stitch 358 and the distal lasso stitch 332 is less than 0.5 mm.

Referring to FIG. 58, a back stitch 360 can be created using the needle 310 (connected to the second tail 306 of the proximal suture 300) after creating the lasso stitch 358. The back stitch 360 can be created in the same way as the back stitch 344 illustrated in FIG. 50B (e.g., following the sewing paths P12 and P13).

Finally, FIG. 59 illustrates a method of hiding the second tail 306 of the proximal suture 300 using a diagonal stitch 362 to thread the second tail 306 underneath the retention element 114 (and possibly within the circumferential wall 112w of the tubular member 112). In the depicted example, the needle 310 (connected to the second tail 306) is inserted from the base of the second tail 306 (i.e., near the last exist location E7 in FIG. 50B) and exits from the retention element 114 at an exit location 364 that is proximal to the lasso stitch 358. A distance between the exit location 364 and the lasso stitch 358 can range from about 2 mm and about 3 mm, e.g., about 2.5 mm. The second tail 306 can then be pulled taut and trimmed closed to its exit location 364, e.g., using scissors. As a result, the second tail 306 can be hidden underneath the retention element 114.

### Exemplary Delivery Apparatus

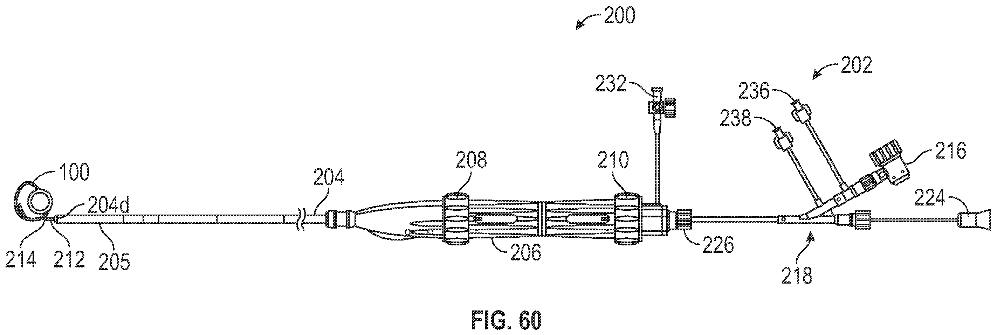

FIG. 60 shows a delivery apparatus 200 configured to implant a docking device, such as the docking device 100 described above or other docking devices, to a target implantation site in a patient, according to one example. Thus, the delivery apparatus 200 can also be referred to as a "dock delivery catheter" or "dock delivery system."

As shown, the delivery apparatus 200 can include a handle assembly 202 and a delivery sheath 204 (also referred to as the "delivery shaft" or "outer shaft" or "outer sheath") extending distally from the handle assembly 202. The handle assembly 202 can include a handle 206 including one or more knobs, buttons, wheels, and/or other means for controlling and/or actuating one or more components of the delivery apparatus 200. For example, in some examples, as shown in FIG. 60, the handle 206 can include knobs 208 and 210 which can be configured to steer or control flexing of the delivery apparatus 200 such as the delivery sheath 204 and/or the sleeve shaft 220 described below.

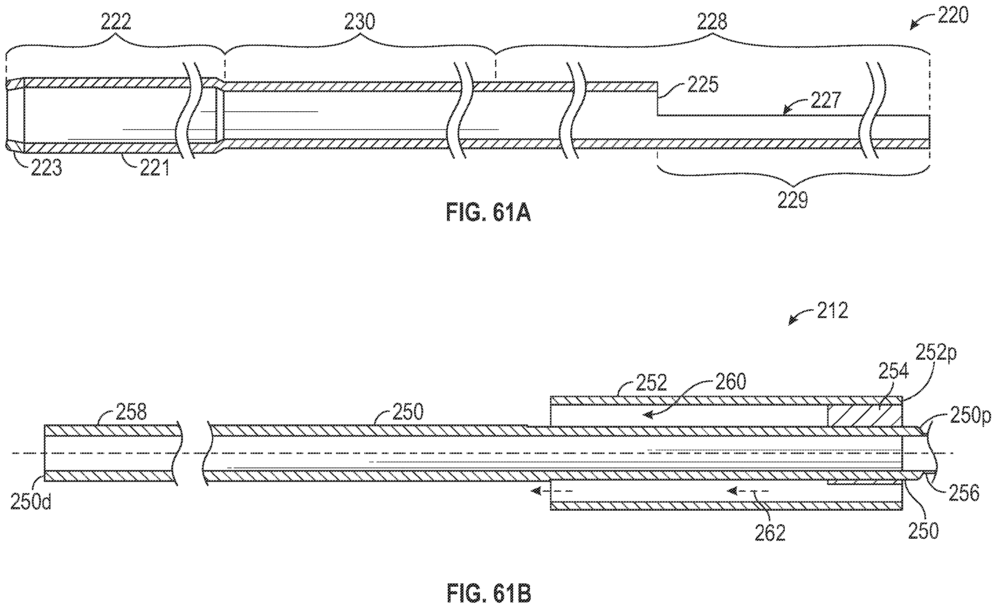

In certain examples, the delivery apparatus 200 can also include a pusher shaft 212 (see e.g., FIG. 61B) and a sleeve shaft 220 (see e.g., FIG. 61A), both of which can extend through an inner lumen of the delivery sheath 204 and have respective proximal end portions extending into the handle assembly 202.

As described below, a distal end portion (also referred to as "distal section") of the sleeve shaft 220 can include a lubricous dock sleeve 222 configured to cover (e.g., surround) the docking device 100. For example, the docking device 100 (including the guard member 104) can be retained inside the dock sleeve 222, which is further retained by a distal end portion 205 of the delivery sheath 204, when navigating through a patient's vasculature. As noted above, the docking device 100 retained within the delivery sheath 204 can remain in the delivery configuration. Similarly, the guard member 104 retained within the dock sleeve 222 can also remain in the delivery configuration.

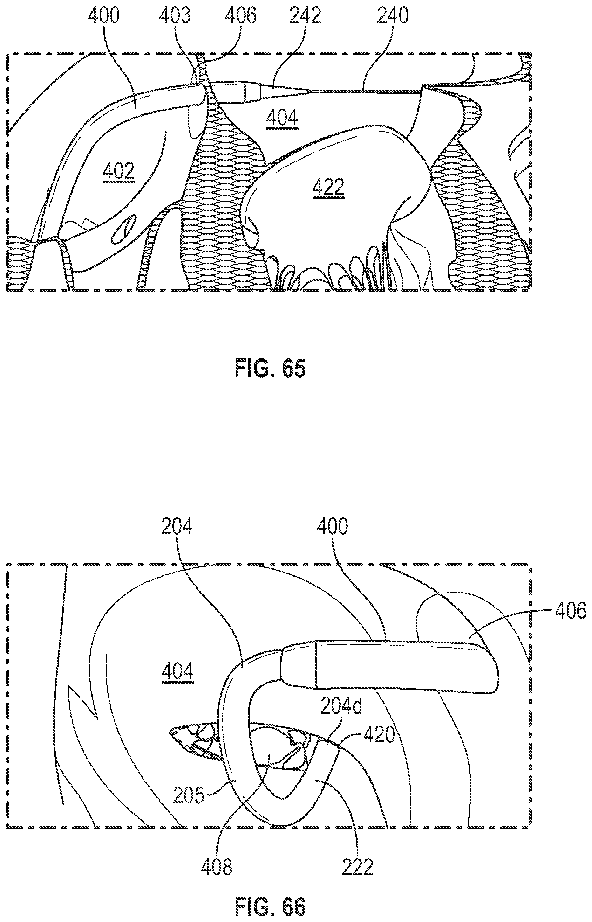

Additionally, the distal end portion 205 of the delivery sheath 204 can be configured to be steerable. In one example, by rotating a knob (e.g., 208 or 210) on the handle 206, a curvature of the distal end portion 205 can be adjusted so that the distal end portion 205 of the delivery sheath 204 can be oriented in a desired angle. For example, as shown in FIG. 66 and described below, to implant the docking device 100 at the native mitral valve location, the distal end portion 205 of the delivery sheath 204 can be steered in the left atrium so that the dock sleeve 222 and the docking device 100 retained therein can extend through the native mitral valve annulus at a location adjacent the posteromedial commissure.

In certain examples, the pusher shaft 212 and the sleeve shaft 220 can be coaxial with one another, at least within the delivery sheath 204. In addition, the delivery sheath 204 can be configured to be axially movable relative to the sleeve shaft 220 and the pusher shaft 212. As described further below, a distal end of the pusher shaft 212 can be inserted into a lumen of the sleeve shaft 220 and press against the proximal end (e.g., 102d) of the docking device 100 retained inside the dock sleeve 222.

After reaching a target implantation site, the docking device 100 can be deployed from the delivery sheath 204 by manipulating the pusher shaft 212 and sleeve shaft 220 using a hub assembly 218, as described further below. For example, by pushing the pusher shaft 212 in the distal direction while holding the delivery sheath 204 in place or retracting the delivery sheath 204 in the proximal direction while holding the pusher shaft 212 in place, or pushing the pusher shaft 212 in the distal direction while simultaneously retracting the delivery sheath 204 in the proximal direction, the docking device 100 can be pushed out of a distal end 204d of the delivery sheath 204, thus changing from the delivery configuration to the deployed configuration. In certain examples, the pusher shaft 212 and the sleeve shaft 220 can be actuated independently of each other.

In certain examples, when deploying the docking device 100 from the delivery sheath 204, the pusher shaft 212 and the sleeve shaft 220 can be configured to move together, in the axial direction, with the docking device 100. For example, actuation of the pusher shaft 212, to push against the docking device 100 and move it out of the delivery sheath 204 can also cause the sleeve shaft 220 to move along with the pusher shaft 212 and the docking device 100. As such, the docking device 100 can remain being covered by the dock sleeve 222 of the sleeve shaft 220 during the procedure of pushing the docking device 100 into position at the target implantation site via the pusher shaft 212. Thus, when the docking device 100 is initially deployed at the target implantation site, the lubricous dock sleeve 222 can facilitate the covered docking device 100 to encircle the native anatomy.

During delivery, the docking device 100 can be coupled to the delivery apparatus 200 via a release suture 214 (or other retrieval line comprising a string, yarn, or other material that can be configured to be tied around the docking device 100 and cut for removal) that extends through the pusher shaft 212. In one specific example, the release suture 214 can extend through the delivery apparatus 200, e.g., through an inner lumen of the pusher shaft 212, to a suture lock assembly 216 of the delivery apparatus 200.

The handle assembly 202 can further include a hub assembly 218 to which the suture lock assembly 216 and a sleeve handle 224 are attached. The hub assembly 218 can be configured to independently control the pusher shaft 212 and the sleeve shaft 220 while the sleeve handle 224 can control an axial position of the sleeve shaft 220 relative to the pusher shaft 212. In this way, operation of the various components of the handle assembly 202 can actuate and control operation of the components arranged within the delivery sheath 204. In some examples, the hub assembly 218 can be coupled to the handle 206 via a connector 226.

The handle assembly 202 can further include one or more flushing ports (e.g., three flushing ports 232, 236, 238 are shown in FIG. 60) to supply flush fluid to one or more lumens arranged within the delivery apparatus 200 (e.g., annular lumens arranged between coaxial components of the delivery apparatus 200), as described below.

Further details on delivery apparatus/catheters/systems (including various examples of the handle assembly) that are configured to deliver a docking device to a target implantation site can be found in U.S. Patent Publication Nos. 2018/0318079 and 2018/0263764.

### Exemplary Sleeve Shaft

FIG. 61A shows a sleeve shaft 220, according to one example. In some examples, the sleeve shaft 220 can have a lubricous distal section 222 (also referred to as the "dock sleeve" herein) configured to cover a docking device (e.g., 100) during deployment, a proximal section 228 used to manipulate or actuate position of the distal section 222, and a middle section 230 connecting the distal section 222 and the proximal section 228.

In some examples, the dock sleeve 222 can be configured to be flexible, have a lower durometer than the remainder of the sleeve shaft 220, and have a hydrophilic coating, which can act as a lubricous surface to improve the ease of encircling the native anatomy and reduce risk of damage to the native tissue. In some examples, the dock sleeve 222 can form a tubular structure which has an inner diameter sufficient to surround the docking device 100 and an outer diameter that is small enough to be retained within and axially movable within the delivery sheath 204. In some examples, the outer diameter of the dock sleeve 222 can be slightly larger than the outer diameter of the middle section 230. In some examples, the length of the dock sleeve 222 is sufficient to cover or longer than the full length of the docking device 100 when it is retained inside the dock sleeve 222.

The dock sleeve 222 can have a body portion 221 and a tip portion 223 located at a distal end of the body portion 221. In some examples, the tip portion 223 can extend about 1-4 mm (e.g., about 2 mm) distally from the distal end of the body portion 221. In some examples, the tip portion 223 can taper radially inwardly such that it has a smaller diameter than the body portion 221. In some examples, during delivery, the tip portion 223 can extend past the distal end (e.g., 102d) of the docking device, thereby providing the dock sleeve 222 with a more atraumatic tip that can bend, squeeze, deform, or the like, as it is navigated around the native architecture of the implantation site for the docking device.

Additional examples of the dock sleeve, including various features of the body portion and tip portion of the dock sleeve, are described further in Provisional U.S. Application No. 63/138,910.

In some examples, the middle section 230 of the sleeve shaft 220 can be configured to provide a sufficient column strength so as to push the dock sleeve 222 (with the docking device 100) out of a distal end 204d of the delivery sheath 204, and/or retract the dock sleeve 222 after the docking device 100 is deployed at the target implantation site. The middle section 230 can also be configured to have an enough flexibility so as to facilitate navigating the anatomy of a patient from the point of insertion of the delivery apparatus 200 to the heart. In certain examples, the dock sleeve 222 and the middle section 230 can be formed as a single, continuous unit with varying properties (e.g., dimensions, polymers, braids, etc.) along the length of the singular unit.

In some examples, a proximal portion of the proximal section 228 can be arranged in the handle assembly 202. The proximal section 228 of the sleeve shaft 220 can be configured to be more rigid and provide column strength to actuate the position of the dock sleeve 222 by pushing the middle section 230 and dock sleeve 222 with the docking device 100 and retracting the dock sleeve 222 after the docking device 100 is deployed at the target implantation site.

In some examples, the proximal portion of the proximal section 228 can include a cut portion 229 which has a cross-section (in a plane normal to a central longitudinal axis of the sleeve shaft 220) that is not a complete circle (e.g., is open and does not form a closed tube). An end surface 225 can be formed between the cut portion 229 and the remainder of the proximal section 228. The end surface 225 can be configured normal to a central longitudinal axis of the sleeve shaft 220 and can be configured to come into contact with a stop element (e.g., plug 254) of the pusher shaft 212, as explained further below.

The cut portion 229 can extend into the hub assembly 218 of the handle assembly 202. As described below, a proximal extension 256 of the pusher shaft 212 can extend along an inner surface of the cut portion 229. The cut (e.g., open) profile of the cut portion 229 can allow the proximal extension 256 of the pusher shaft 212 to extend out of a void space 227 formed in the cut portion 229 and branch off, at an angle relative to the cut portion 229, into the suture lock assembly 216 of the hub assembly 218 (see e.g., FIG. 60). As such, the pusher shaft 212 and sleeve shaft 220 can be operated in parallel with one another and an overall length of the delivery apparatus 200 in which the sleeve shaft 220 and pusher shaft 212 are incorporated can be maintained similar to or only minimally longer than a delivery system that does not incorporate the sleeve shaft 220.

Additional examples of the sleeve shaft are described further in PCT Patent Application Publication No. WO/2020/247907.

### Exemplary Pusher Shaft

FIG. 61B shows a pusher shaft 212, according to one example. As shown, the pusher shaft 212 can include a main tube 250, a shell 252 surrounding a proximal end portion of the main tube 250, a plug 254 connecting the main tube 250 to the shell 252, and a proximal extension 256 extending from a proximal end of the main tube 250.

The main tube 250 can be configured for advancing and retracting a docking device (such as one of the docking devices described herein) and housing the release suture (e.g., 214) that secures the docking device to the pusher shaft 212. The main tube 250 can extend from the distal end 204d of the delivery sheath 204 into the handle assembly 202 of the delivery apparatus 200. For example, in certain examples, a proximal end portion of the pusher shaft 212, which includes an interface between the main tube 250, the shell 252, the plug 254, and the proximal extension 256, can be arranged within or proximate to the hub assembly 218 of the handle assembly 202. Thus, the main tube 250 can be an elongate tube that extends along a majority of the delivery apparatus 200.

The main tube 250 can be a relatively rigid tube that provides column strength for actuating deployment of a docking device. In some examples, the main tube 250 can be a hypo tube. In some examples, the main tube 250 can comprise a biocompatible metal, such as stainless steel. The main tube 250 can have a distal end 250d configured to interface with a docking device and a proximal end 250p, where the proximal extension 256 is attached. In some examples, a distal section 258 of the main tube 250 can be relatively more flexible (e.g., via one or more cuts into an outer surface of the main tube and/or having a durometer material) than the remaining part of the main tube 250. Thus, the distal section 258 can flex and/or bend along with the delivery sheath 204 of the delivery apparatus 200, as it is navigated through a vasculature of a patient, to the target implantation site.

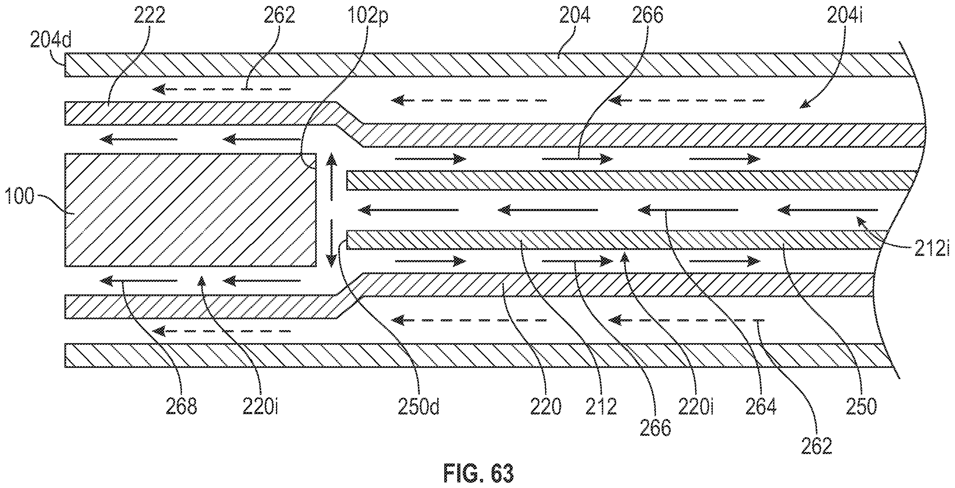

In some examples, the shell 252 can be configured to lock the main tube 250 and provide a hemostatic seal on the pusher shaft 212 without interfering with movement of the sleeve shaft 220. As shown in FIG. 61B, an inner diameter of the shell 252 can be larger than an outer diameter of the main tube 250, thereby forming an annular cavity 260 between the main tube 250 and the shell 252. As such, the proximal section 228 of the sleeve shaft 220 can slide within the annular cavity 260, as described further below. Further, flush fluid provided to a lumen on an exterior of the proximal extension 256, in the hub assembly 218, can flow through the annular cavity 260 and exit at a distal end of the shell 252 (as shown by arrows 262) to enter a lumen between the sleeve shaft 220 and delivery sheath 204 of the delivery apparatus, as discussed further below with reference to FIG. 63.

The plug 254 can be configured to be arranged within the annular cavity 260, at a proximal end 252p of the shell 252. In some examples, the plug 254 can be configured to "plug" or fill a portion of the annular cavity 260 located at the proximal end 252p of the shell 252, while leaving the remaining portion of the annular cavity 260 open to receive the cut portion 229 of the sleeve shaft 220 therein. In some examples, the shell 252 and the plug 254 can be fixedly coupled to the main tube 250 (e.g., via welding) to allow the cut portion 229 of the sleeve shaft 220 to slide between the main tube 250 and the shell 252. As described below, the plug 254 can also act as a stop for the sleeve shaft 220.

As noted above, the proximal extension 256 can extend from the proximal end 250p of the main tube 250 and the shell 252. The proximal extension 256 can provide the pusher shaft 212 with certain flexibility such that it may be routed from the inside of the sleeve shaft 220 (e.g., the cut portion 229) to the outside of the sleeve shaft 220, thereby allowing the pusher shaft 212 and the sleeve shaft 220 to be actuated in parallel and reducing an overall length of the delivery apparatus. In certain examples, the proximal extension 256 can be made of a flexible polymer.

Additional examples of the pusher shaft are described further in PCT Patent Application Publication No. WO/2020/247907.

### Exemplary Sleeve Shaft and Pusher Shaft Assembly

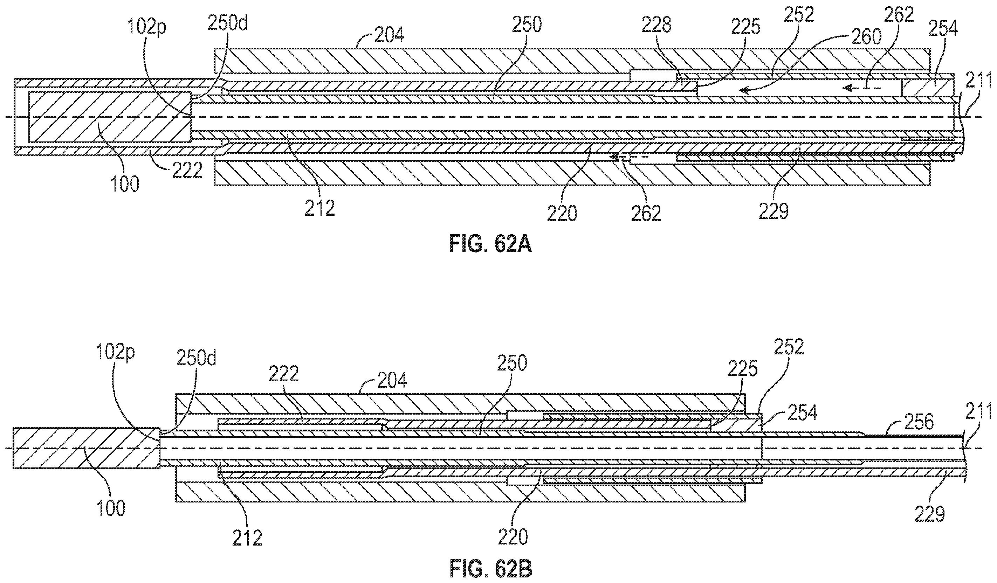

FIGS. 62A-62B illustrate example arrangements of the pusher shaft 212 and sleeve shaft 220 in the delivery sheath 204 of the delivery apparatus 200, before and after deployment of a docking device such as 100. As shown, the main tube 250 of the pusher shaft 212 can extend through a lumen of the sleeve shaft 220, which can extend through a lumen of the delivery sheath 204. The pusher shaft 212 and the sleeve shaft 220 can share a central longitudinal axis 211 of the delivery sheath 204.

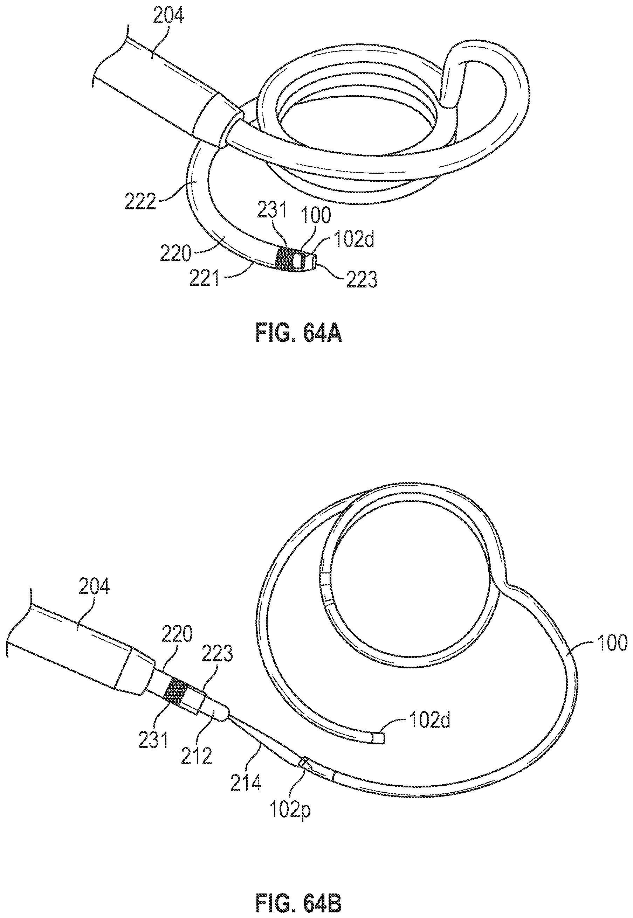

FIG. 63 shows various lumens configured to receive flush fluid during a delivery and implantation procedure can be formed between the docking device 100, the pusher shaft 212, the sleeve shaft 220, and the delivery sheath 204. Additionally, FIG. 64A shows a first configuration where the docking device 100 has been deployed from the delivery sheath 204 while still being covered by the dock sleeve 222 of the sleeve shaft 220. The dock sleeve 222 in the first configuration is also referred to be in a "covered state." When the dock sleeve 222 is in the covered state, the guard member 104 (not shown for clarity purposes) can remain in the delivery configuration (i.e., radially compressed by and retained within the dock sleeve 222). FIG. 64B shows a second configuration where the docking device 100 is uncovered by the dock sleeve 222 after the sleeve shaft 220 has been retracted back into the delivery sheath 204. The dock sleeve 222 in the second configuration is also referred to be in an "uncovered state." When the dock sleeve 222 is in the uncovered state, the guard member 104 (not shown for clarity purposes) can radially expand and move to the deployed configuration.

Specifically, FIG. 62A illustrates a first configuration of the pusher shaft 212 and sleeve shaft 220 assembly, pre-deployment or during deployment of the docking device 100, according to one example. As shown, the dock sleeve 222 can be configured to cover the docking device 100 while the end surface 225 of the sleeve shaft 220 is positioned away from the plug 254. In addition, the distal end 250d of the pusher shaft 212 can extend into the dock sleeve 222 and come into contact with the proximal end 102p of the docking device 100.

During deploying the docking device 100 from the delivery sheath 204, the pusher shaft 212 and the sleeve shaft 220 can be configured to move together, in the axial direction, with the docking device 100. For example, actuation of the pusher shaft 212, to push against the docking device 100 and move it out of the delivery sheath 204 can also cause the sleeve shaft 220 to move along with the pusher shaft 212 and the docking device 100. As such, the docking device 100 can remain being covered by the dock sleeve 222 of the sleeve shaft 220 during the procedure of pushing the docking device 100 into position at the target implantation site via the pusher shaft 212, as illustrated in FIG. 64A.

Additionally, as shown in FIG. 64A, during delivery and implantation of the covered docking device 100 at the target implantation site, the tip portion 223 of the sleeve shaft 220 can extend distal to the distal end 102d of the docking device 100, thereby providing the dock sleeve 222 with a more atraumatic tip.

In some examples, one or more radiopaque markers 231, can be placed at the dock sleeve 222 to increase the ability to visualize the dock sleeve 222 during deployment of a docking device (e.g., 100). In certain examples, at least one radiopaque marker 231 can be placed at the intersection between the body portion 221 and the tip portion 223. In certain examples, at least one radiopaque marker 231 can be placed on the tip portion 223. In some examples, the distal end 102d of the docking device 100 can be arranged proximate to or just distal to the radiopaque markers 231 of the dock sleeve 222.

In some examples, the radiopaque markers 231 can include a radiopaque material such as platinum-iridium. In other examples, the radiopaque material included in the radiopaque markers 231 can be Barium Sulphate (BaSO4), Bismuth Subcarbonate ((BiO)□CO□), Bismuth Oxychloride (BiOCl), or the like.

In some examples, the tip portion 223 of the dock sleeve 222 can be made from a polymeric material loaded with any one of the radiopaque material described above so as to enable the most distal edge of the tip portion 223 to be visible under fluoroscopy.

FIG. 62B illustrates a second configuration of the pusher shaft 212 and sleeve shaft 220 assembly, after deploying the docking device 100 from the delivery sheath 204 at the target implantation site and removing the dock sleeve 222 from the implanted docking device 100, according to one example. As shown, after implanting the docking device 100 at the target implantation site, in its desired position, the sleeve shaft 220 can be pulled off the docking device 100 and retracted back into delivery sheath 204 while holding the pusher shaft 212 steady so that its distal end 250d presses against the proximal end 102p of the docking device 100. Alternatively, the docking device 100 can be exposed by pushing the pusher shaft 212 in the distal direction while holding the sleeve shaft 220 steady. In some examples, as shown in FIG. 62B, the sleeve shaft 220 can be stopped from further retraction into the delivery apparatus upon the end surface 225 coming into contact with the plug 254.

FIG. 64B shows the sleeve shaft 220 removed from the docking device 100, leaving the docking device 100 uncovered by the dock sleeve 222. As shown, the tip portion 223 of the sleeve shaft 220 can be arranged proximal to (e.g., retracted past) the distal end of the pusher shaft 212 which can still be connected to the proximal end 102p of the docking device 100 via the release suture 214. As explained further below, after implanting the docking device 100 at the target implantation site and removing the dock sleeve 222 from covering the docking device 100, the docking device 100 can be disconnected from the delivery apparatus by cutting the release suture 214, e.g., by using the suture lock assembly 216 of the delivery apparatus 200.

As shown in FIG. 63, a first, pusher shaft lumen 212i can be formed within an interior of the pusher shaft 212 (e.g., within an interior of the main tube 250). The pusher shaft lumen 212i can receive a flush fluid from a first fluid source, which may be fluidly coupled to a portion of the handle assembly 202. The flush fluid flow 264 through the pusher shaft lumen 212i can travel along a length of the main tube 250 of the pusher shaft 212, to the distal end 250d of the main tube 250 of the pusher shaft 212. In some examples, the distal end 250d of the main tube 250 can be spaced away from the proximal end 102p of the docking device 100. Thus, at least a portion of the flush fluid flow 264 can flow into a distal portion of a second, sleeve shaft lumen 220i, which is arranged between an outer surface of the docking device 100 and an inner surface of the dock sleeve 222 of the sleeve shaft 220, as flush fluid flow 268. Further, in some examples, a portion of the flush fluid flow 264 can also flow back into a proximal portion of the sleeve shaft lumen 220i, which is arranged between an outer surface of the pusher shaft 212 and an inner surface of the sleeve shaft 220 that is proximal to the dock sleeve 222, as flush fluid flow 266. Thus, the same, first fluid source may provide flush fluid to the pusher shaft lumen 212i the sleeve shaft lumen 220i (including both the distal portion outside the dock sleeve 222 and the proximal portion that is proximal to the dock sleeve 222), via the pusher shaft lumen 212i.

FIG. 63 also shows a third, delivery sheath lumen 204i, which is arranged between an inner surface of the delivery sheath 204 and an outer surface of the sleeve shaft 220. The delivery sheath lumen 204i can receive a flush fluid from one or more second fluid sources, which may be fluidly coupled to a portion of the handle assembly 202, and which may result in flush fluid flow (as shown by arrows 262) flowing through the delivery sheath lumen 204i, to the distal end 204d of the delivery sheath 204.

Flushing the above-described lumens can help prevent or reduce thrombosis on and around the docking device 100 and other concentric parts of the delivery apparatus 200 during deployment of the docking device 100 from the delivery apparatus 200 and implantation of the docking device 100 at a target implantation site. In an example, as shown in FIG. 60, the first and/or the second fluid sources can be connected to one or more flushing ports (e.g., 232, 236, 238) arranged on and/or coupled to the handle assembly 202 of the delivery apparatus 200 to provide the flush fluid to the lumens described above.

Additional examples of the sleeve shaft and pusher shaft assembly are described further in PCT Patent Application Publication No. WO/2020/247907.

### Exemplary Implantation Procedure

An example method of delivering a docking device (such as the docking device 100 described above) and implanting a prosthetic valve (such as the prosthetic valve 10 described above) within the docking device is illustrated in FIGS. 65-78. In this example, the target implantation site is at the native mitral valve 422. Following the same principle described herein, the same method or its variants can also be used for implantation of the docking device and the prosthetic valve at other target implantation sites.

FIG. 65 illustrates introducing a guiding catheter 400 into a patient's heart over a previously inserted guidewire 240. Specifically, the guiding catheter 400 and the guidewire 240 are inserted from the right atrium 402 into the left atrium 404 through the interatrial septum 406 (e.g., via a previously punctured hole 403 in the interatrial septum 406). To facilitate navigation through the patient's vasculature and transseptal insertion, a nosecone 242 having a tapered distal tip can be placed at a distal end of the guiding catheter 400. After the distal end of the guiding catheter 400 enters the left atrium 404, the nosecone 242 and the guidewire 240 can be retracted back into the guiding catheter 400, for example, by operating a handle connected to a proximal end of the guiding catheter 400. The guiding catheter 400 can remain in place (i.e., extending through the interatrial septum 406) so that the distal end of the guiding catheter 400 remains within the left atrium 404.

FIG. 66 illustrates introducing a delivery apparatus (such as the delivery apparatus 200 described above) through the guiding catheter 400. Specifically, the delivery sheath 204 can be inserted through a lumen of the guiding catheter 400 until the distal end portion 205 of the delivery sheath 204 extends distally out of the distal end of the guiding catheter 400 and into the left atrium 404.

As described above, the delivery apparatus 200 can have a sleeve shaft 220 and a pusher shaft 212, both of which can extend through a lumen of the delivery sheath 204. As shown in FIGS. 67-69, the distal end portion of the sleeve shaft 220 can have a dock sleeve 222 which surrounds the docking device 100. As described herein, the dock sleeve 222 can be retained within the distal end portion 205 of the delivery sheath 204.

As described above, the distal end portion 205 of the delivery sheath 204 can be steerable, for example, by operating a knob located on the handle assembly 202. Because the dock sleeve 222 and the docking device 100 are also flexible, flexing of the distal end portion 205 of the delivery sheath 204 can also cause flexing of the dock sleeve 222 and the docking device 100 retained therein. As shown in FIG. 66, the distal end portion 205 of the delivery sheath 204 (along with the dock sleeve 222 retaining the docking device 100) can be flexed in desired angular directions so that the distal end 204d of the delivery sheath 204 can extend through the native mitral valve annulus 408 at a location adjacent the posteromedial commissure 420 and into the left ventricle 414.

FIG. 67 illustrates deployment of the docking device 100 at the mitral valve location. As shown, a distal portion of the docking device 100, which includes the leading turn 106 and the central region 108 of the coil, can be deployed out of the distal end 204d of the delivery sheath 204 and extend into the left ventricle 414. Note that the deployed distal portion of the docking device 100 is still covered by the dock sleeve 222. This can be achieved, for example, by retracting the delivery sheath 204 in the proximal direction while holding both the pusher shaft 212 and the sleeve shaft 220 in place, thus causing the distal portion of the docking device 100 to extend distally out of the delivery sheath 204 while it remains to be covered by the dock sleeve 222. Retraction of the delivery sheath 204 can continue until the delivery sheath 204 is moved to the stabilization turn 110 and proximal to the expandable member 104.

Not being restrained by the distal end portion 205 of the delivery sheath 204, the distal portion of the docking device 100 can move from the delivery configuration to the deployed (i.e., helical) configuration. Specifically, as shown in FIG. 67, the coil of the docking device 100 (covered by the dock sleeve 222) can form the leading turn 106 extending into the left ventricle 414, as well as a plurality of functional turns in the central region 108 that wrap around the native leaflets 410 of the native valve and the chordae tendineae 412.

Because the dock sleeve 222 has a lubricious surface, it can prevent or reduce the likelihood of the tubular member 112 (which surrounds the coil 102 of the docking device) from directly contacting and catching (or getting stuck with) the native tissue and help ensure that the covered docking device 100 encircles the native anatomy. In addition, the soft tip portion 223 (which can have a tapered shape) of the dock sleeve 222 can also facilitate atraumatic encircling around the native tissue. As noted above, a flush fluid (see e.g., 264 in FIG. 63) can flow through the dock sleeve 222 and around the docking device 100 to prevent or reduce thrombosis on and around the docking device 100 and other concentric parts of the delivery apparatus 200 during deployment of the docking device 100.

As shown in FIG. 68, after the functional turns of the docking device 100 successfully wraps round the native leaflets 410 and the chordae tendineae 412, the dock sleeve 222 can be retracted in a proximal direction relative to the docking device 100. This can be achieved, for example, by pulling the sleeve shaft 220 in the proximal direction while holding the pusher shaft 212 steady so that its distal end can press against the proximal end of the docking device 100, as described above with reference to FIG. 62B. As noted above, the dock sleeve 222 can be retracted back into the delivery sheath 204. FIG. 69 shows the docking device 100, which is uncovered by the dock sleeve 222, encircling the native leaflets and chordae tendineae.

FIG. 70A illustrates stabilizing the docking device 100 from the atrial side. As shown, the delivery sheath 204 can be retracted into the guiding catheter 400 so that the atrial side (i.e., the proximal portion) of the docking device 100, including the stabilization turn 110 of the coil can be exposed. The stabilization turn 110 can be configured to provide one or more points or regions of contact between the docking device 100 and the left atrial wall, such as at least three points of contact in the left atrium or complete contact on the left atrial wall. The stabilization turn 110 can be flared out or biased toward both the posterior wall 416 and the anterior wall 418 of the left atrium so as to prevent the docking device 100 from falling into the left ventricle prior to deployment of a prosthetic valve therein.

Without the constraint of the delivery sheath 204 and the dock sleeve 222, the guard member 104 can move to the deployed configuration (due to radial expansion of the expandable member 116). As shown, the guard member 104 of the docking device 100 can be configured to contact the native annulus in the left atrium to create a sealed and atraumatic interface between the docking device 100 and the native tissue. The proximal end portion 104p of the guard member can be configured to be positioned adjacent (but does not reach) the anterolateral commissure 419 of the native valve. In the deployed configuration, the proximal end 105 of the guard member can be configured to be positioned within the atrial portion 110a or the ascending portion 110b of the stabilization turn, but distal to the boundary 107 between the ascending portion 110b and the stabilization portion 110c (see, e.g., FIG. 1A). For example, after the initial deployment of the docking device 100 and before deploying the prosthetic valve (e.g., 10) within the docking device 100, the proximal end 105 of the guard member can be configured to be positioned between the proximal seating marker 121p and the distal seating marker 121d, or slightly distal to the distal seating marker 121d in certain circumstances. In certain examples, the distal end portion 104d of the guard member can be disposed in the left ventricle 414 or at least adjacent a posteromedial commissure 420 of the native valve so that leakage at that location can be prevented or reduced.

In the depicted example, a proximal end portion of the retention element 114 extends into the ascending portion 110b of the coil. In addition, the proximal end 105 of the guard member 104 is located distal to the proximal seating marker 121p, which is located distal to the ascending portion 110b. In certain examples, the proximal end 105 of the guard member 104 is located between the proximal seating marker 121p and the distal seating marker 121d (which is covered by the guard member 104 and not shown in FIG. 70A). As described above, such configuration can advantageously improve the sealing and/or durability of the guard member 104.

In certain instances, after initial deployment of docking device 100, the proximal end 105 of the guard member 104 may incidentally extend onto the ascending portion 110b, as illustrated in FIG. 70B. Under such circumstances, the dock sleeve 222 can be used to "reposition" the proximal end 105 of guard member 104 away from the ascending portion 110b. According to one example, the dock sleeve 222 can be pushed out of the delivery sheath 204 until its tapered tip portion 223 contacts the tapered proximal end 105 of the guard member 104 (see, e.g., FIG. 70B). Location of the tip portion 223 of the dock sleeve 222 can be determined, e.g., based on visualization of the radiopaque marker 231 on the dock sleeve 222 under fluoroscopy. Thus, by further pushing the dock sleeve 222 in the distal direction, the proximal end 105 of the guard member 104 can be moved distally until it is repositioned distal to the proximal seating marker 121p (see e.g., FIG. 70C). Such positioning can be confirmed, e.g., by observing the radiopaque marker 231 on the dock sleeve 222 is located distal to the proximal seating marker 121p. The dock sleeve 222 can then be retracted back into the delivery sheath 204. As described above, the retention element 114, by applying a friction force (e.g., the frictional interaction between the retention element 114 and the proximal end 105 of the guard member 104), can impede the axial movement of the proximal end portion 104p of the guard member 104 relative to the coil. Thus, the retention element 114 can retain the proximal end 105 of the guard member 104 in the repositioned location, which is distal to the ascending portion 110b.

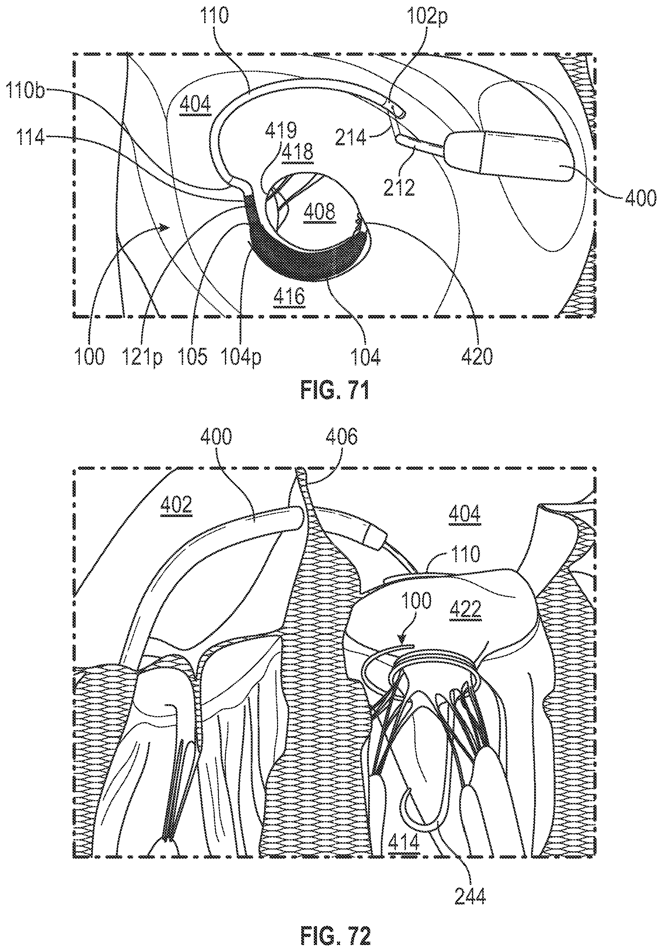

FIG. 71 illustrates the fully deployed docking device 100. The release suture 214, which extends through the pusher shaft 212 and connects the proximal end 102p of the coil to the suture lock assembly 216, can then be cut so that the docking device 100 can be released from the delivery apparatus 200. The delivery apparatus 200 can then be removed from the guiding catheter 400 to prepare for implantation of a prosthetic valve.

FIG. 72 illustrates inserting a guidewire catheter 244 through the guiding catheter 400, across the native mitral valve annulus through the docking device 100, and into the left ventricle 414.

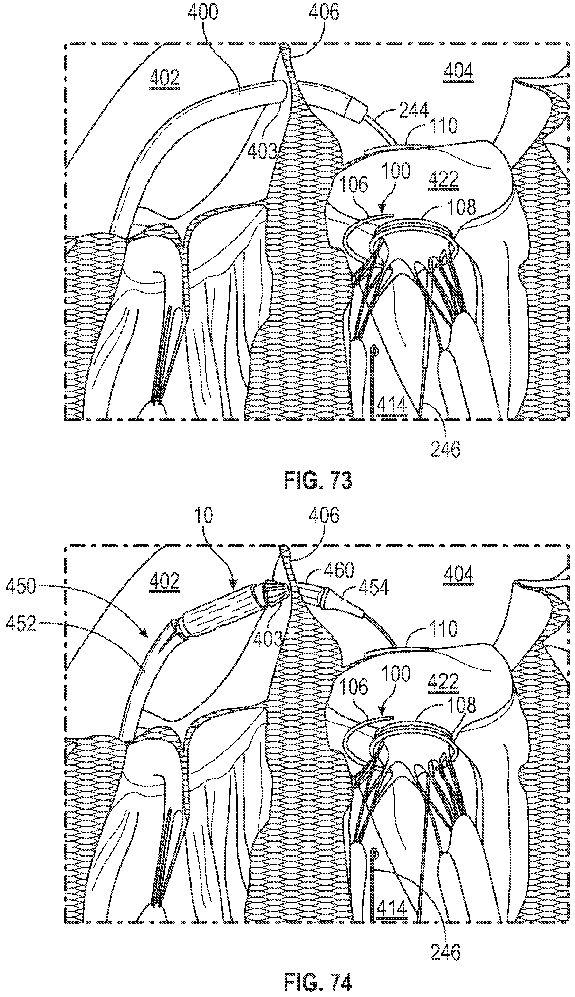

FIG. 73 illustrates inserting a valve guidewire 246 into the left ventricle 414 through an inner lumen of the guidewire catheter 244. The guidewire catheter 244 can then be retracted back into the guiding catheter 400, and the guiding catheter 400 and the guidewire catheter 244 can be removed, leaving the valve guidewire 246 in place.

FIG. 74 illustrates transseptal delivery of a prosthetic valve (such as the prosthetic valve 10) into the left atrium 404. A prosthetic valve delivery apparatus 450 can be introduced over the valve guidewire 246. During delivery, the prosthetic valve 10 can be crimped over a deflated balloon 460 located between a distal end of an outer shaft 452 and a nosecone 454 of the delivery apparatus 450. In some examples, before transseptal delivery of the prosthetic valve 10, the hole 403 on the interatrial septum 406 can be further dilated by inserting a balloon catheter through the hole 403 and radially expanding a balloon mounted on the balloon shaft.

FIG. 75 illustrates placing the prosthetic valve 10 within the docking device 100. Specifically, the prosthetic valve 10 can be positioned within and substantially coaxial with the functional turns in the central region 108 of the docking device 100. In some examples, the outer shaft 452 can be slightly retracted so that the balloon 460 is located outside the outer shaft 452.

FIG. 76 illustrates radial expansion of the prosthetic valve 10 within the docking device 100. Specifically, the balloon 460 can be radially inflated by injecting an inflation fluid into the balloon through the delivery apparatus 450, thereby causing radial expansion of the prosthetic valve 10. As the prosthetic valve 10 is radially expanded within the central region 108 of the coil, the functional turns in the central region 108 can be further radially expanded (i.e., the coil 102 of the docking device can move from the first radially expanded configuration to the second radially expanded configuration, as described above). To compensate for the increased diameter of the function turns, the leading turn 106 can be retracted in the proximal direction and become a part of the functional turn in the central region 108. In other words, the diameter of the leading turn 106 is reduced when the prosthetic valve 10 is expanded.

FIG. 77 illustrates deflating the balloon 460 after radial expansion of the prosthetic valve 10 within the docking device 100. The balloon 460 can be deflated by withdrawing the inflation fluid out of the balloon through the delivery apparatus 450. The delivery apparatus 450 can then be retracted out of the patient's vasculature, and the valve guidewire 246 can also be removed.

FIG. 78 illustrates the final disposition of the docking device 100 at the mitral valve and the prosthetic valve 10 received within the docking device 100. As described above, the radial tension between the prosthetic valve 10 and the central region 108 of the docking device can securely hold the prosthetic valve 10 in place. In addition, the guard member 104 can act as a seal between the docking device 100 and the prosthetic valve 10 disposed therein to prevent or reduce paravalvular leakage around the prosthetic valve 10.

As described above, radially expanding the prosthetic valve 10 within the docking device 100 can cause the guard member 104 to be radially compressed and axially extended, and as a result, the proximal end 105 of the guard member 104 can have a tendency to move proximally relative to the coil. However, the presence of the retention element 114 can frictionally impede the proximal end 105 of the guard member 104 to move proximally over the coil. In addition, the proximal seating marker 121p (which sets the proximal boundary of the proximal end 105 of the guard member 104 after initial deployment of the docking device 100) can be configured to be located far enough from the ascending portion 110b of the coil. Thus, even if the proximal end 105 of the guard member 104 indeed moves proximally due to radial expansion of the prosthetic valve 10 within the docking device 100, such movement can be limited to the extent that the proximal end 105 of the guard member 104 does not extend into the ascending portion 110b of the coil 102.

As the prosthetic heart valve 10 is fully expanded within the docking device 100, the prosthetic heart valve 10 contacts the guard member 104 and urges the guard member 104 against the coil 102, thereby restricting further axial movement of the guard member 104 relative to the native anatomy (e.g., the left atrial wall). In this manner, the retention member 114 can serve to temporarily retain the proximal end of the guard member in the desired position from the time the docking device is deployed until the prosthetic heart valve is expanded therein. After that, the prosthetic heart valve can secure the positioning of the guard member relative to the coil.

Although in the method described above, the prosthetic valve 10 is radially expanded using the inflatable balloon 460, it is to be understood that alternative methods can be used to radially expand the prosthetic valve 10.

For example, in some instances, the prosthetic valve can be configured to be self-expandable. During delivery, the prosthetic valve can be radially compressed and retained within a valve sheath located at a distal end portion of a delivery apparatus. When the valve sheath is disposed within the central region 108 of the docking device, the valve sheath can be retracted to expose the prosthetic valve, which can then self-expand and securely engage with the central region 108 of the docking device. Additional details regarding exemplary self-expandable prosthetic valves and the related delivery apparatus/catheters/systems are described in U.S. Patent Nos. 8,652,202 and 9155,619.

In another example, in certain instances, the prosthetic valve can be mechanically expanded. Specifically, the prosthetic valve can have a frame comprising a plurality of struts that are connected to each other such that an axial force applied to the frame (e.g., pressing an inflow and an outflow end of the frame in toward each other or pulling the inflow end and the outflow end of the frame away from each other) can cause the prosthetic valve to radially expand or compress. Additional details regarding exemplary mechanically-expandable prosthetic valves and the related delivery apparatus/catheters/systems are described in U.S. Patent Application Publication No. 2018/0153689 and PCT Patent Application Publication No. WO/2021/188476.

### Exemplary Foldable PVL Guard

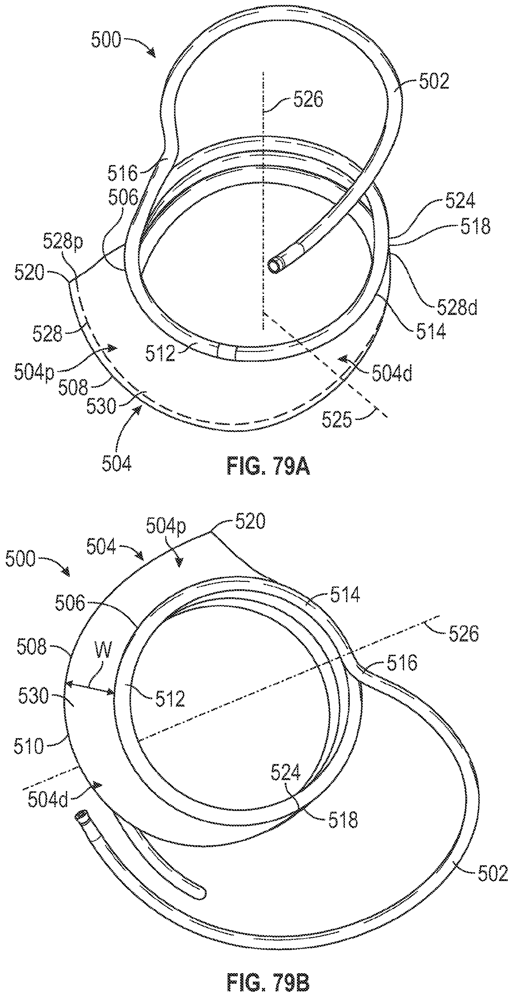

FIGS. 79A-79C show a docking device 500, according to another example. The docking device 500 includes a coil 502 which can move from a substantially straight or delivery configuration to a helical or deployed configuration similar to the coil 102. The docking device 500 also includes a foldable PVL guard 504 attached to the coil 502. As described herein, the foldable PVL guard 504 is also referred to as a "sealing member" or a "skirt," and these terms are used interchangeably hereinafter.

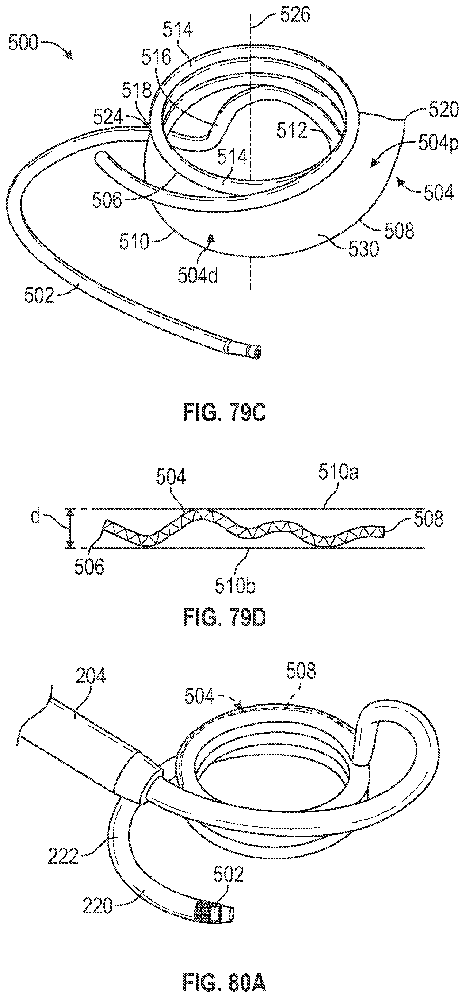

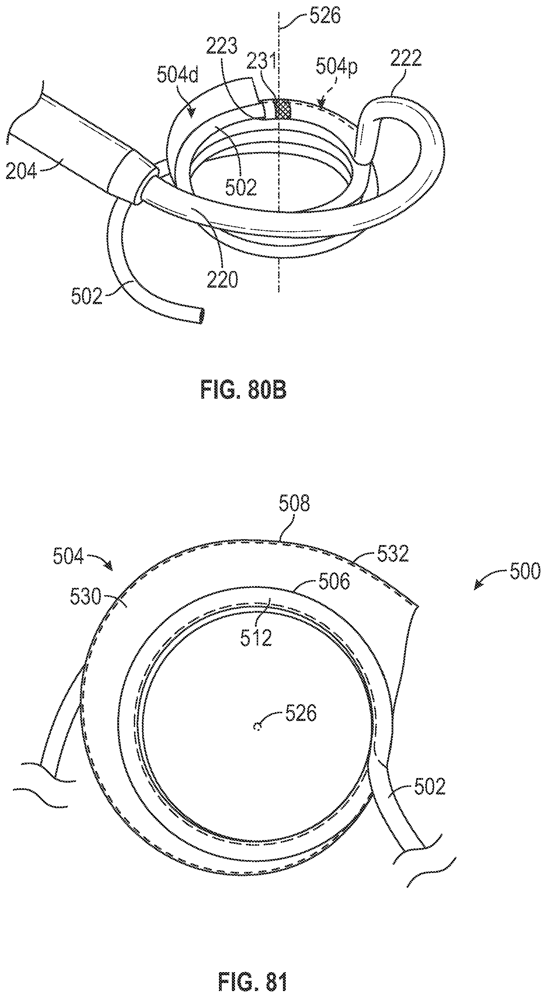

The sealing member 504 can be movable between a delivery configuration (as shown in FIG. 80A) and a deployed configuration (as shown in FIGS. 79A-79C and FIGS. 81-85). In the delivery configuration, the sealing member 504 can be folded and retained within the dock sleeve 222 (which can be a distal end portion of the sleeve shaft 220, as described above). With the coil 502 in the deployed configuration (e.g., FIG. 80A), the sealing member 504 can be exposed (e.g., by retracting the dock sleeve 222 proximally relative to the docking device 500) and extend radially outwardly from the coil 502, as depicted in FIG. 80B. Such radially extended the sealing member 504 can be flat or substantially flat relative to a plane perpendicular to a central longitudinal axis 526 (see FIGS. 79C and 81).

In addition, FIG. 80B shows that the sealing member 504 is partially deployed from the dock sleeve 222. This can happen when the sealing member 504 is partially exposed, e.g., a distal portion 504d of the sealing member is exposed from the dock sleeve 222 but a proximal portion 504p of the sealing member is still covered by the dock sleeve 222. In such circumstances, the distal portion 504d of the sealing member can extend radially outwardly from the coil 502 and form a flat or substantially flat surface, whereas the proximal portion 504p of the sealing member can remain folded and covered by the dock sleeve 222.

FIG. 79D shows a cross-sectional view of the sealing member 504 along a radial axis 525 and depicts an example measurement of flatness of the sealing member 504. For illustrative purposes, the cross-section of the sealing member 504 is depicted as an exaggeratedly rugged or uneven surface. The flatness measure of the sealing member 504 at the cross-section can be defined as a distance between two closest parallel lines 510a, 510b within which the cross-section of the sealing member 504 is bound (e.g., the highest point in the cross-section is located on the line 510a and the lowest point of the cross-section is located on the line 510b).

In certain examples, the flatness measure can be substantially uniform across the sealing member 504 (e.g., the flatness measure can be substantially constant at various cross-sections taken between the proximal end 518 and the distal end 520). In certain examples, the flatness measure can vary across the sealing member 504 (e.g., the flatness measure at a cross-section of the proximal 504p may be different from the flatness measure at a cross-section of the distal portion 504d).

As described here, the sealing member 504 (or a portion of the sealing member) is considered to be flat or substantially flat if a flatness measure at any cross-section of the sealing member 504 (or the portion of the sealing member) is smaller than a predefined threshold value. In certain examples, the predefined threshold value for the flatness measure can range from 0.5 mm to 10 mm, or from 2 mm to 8 mm, or from 3 mm to 6 mm, or from 4 mm to 5 mm.

The sealing member 504 can have an inner edge 506 coupled the coil 502 and an outer edge 508 that is movable between a folded position and an extended position. When the sealing member 504 is in the delivery configuration (see, e.g., FIG. 80A), the outer edge 508 of the sealing member 504 can be in the folded position such that the outer edge 508 can extend along and adjacent the coil 502. When the sealing member 504 is in the deployed configuration, the outer edge 508 can move to the extended position, e.g., at least a segment of the outer edge 508 can extend radially away or spaced apart from the coil 502 (see, e.g., FIG. 79A-79C).

In some examples, as depicted in FIG. 79A-79C, a distal end 518 of the outer edge 508 can be fixedly attached to the coil 502 (and to a distal end 524 of the inner edge 506), e.g., via sutures, glue, and/or any other attachment means, while a proximal end 520 of the outer edge 508 can be radially movable relative to the coil 502. For example, when the sealing member 504 is in the deployed configuration, both the proximal end 520 and a midportion (i.e., the portion between 518 and 520) of the outer edge 508 can extend radially away or spaced apart from the coil 502. As a result, the sealing member 504 in the deployed configuration can have a radially tapered or fan-like shape.

The sealing member 504 in the deployed configuration can have a width (W) defined between the inner edge 506 and the outer edge 508 (see, e.g., FIG. 79B). In certain examples, the width of the sealing member 504 can progressively increase (or in a step-wise manner) from the distal end 518 to the proximal end 520 of the outer edge 508. In certain examples, the width of the sealing member 504 can remain substantially constant along one or more segments of the outer edge 508. For example, the proximal portion 504p of the sealing member can have a substantially constant width such that the outer edge 508 can be parallel or at least substantially parallel to the sealing segment 512 in the proximal portion 504p. In certain examples, the proximal portion 504p of the sealing member can have a width ranging from 3 mm to 8 mm, or from 4 mm to 7 mm, or from 5 mm to 6 mm.

In other examples, the distal end 518 of the outer edge 508 can also be movable relative to the coil 502. In such circumstances, when the sealing member 504 is in the deployed configuration, the complete length of the outer edge 508 (including both the distal end 518 and the proximal end 518) can extend radially away or spaced apart from the coil 502. In such circumstances, the sealing member 504 in the deployed configuration can form a curved band, and the width of the sealing member 504 can be constant or can vary from the distal end 518 to the proximal end 520.

As described herein, the outer edge 508 in the extended position can contact native tissue at an implantation site (e.g., a native valve annulus and/or a wall of a chamber of the heart). Specifically, when the sealing member 504 is in the deployed configuration, the outer edge 508 can create a sealed and atraumatic interface between the docking device 500 and the native tissue so as to reduce or eliminate paravalvular leakage.

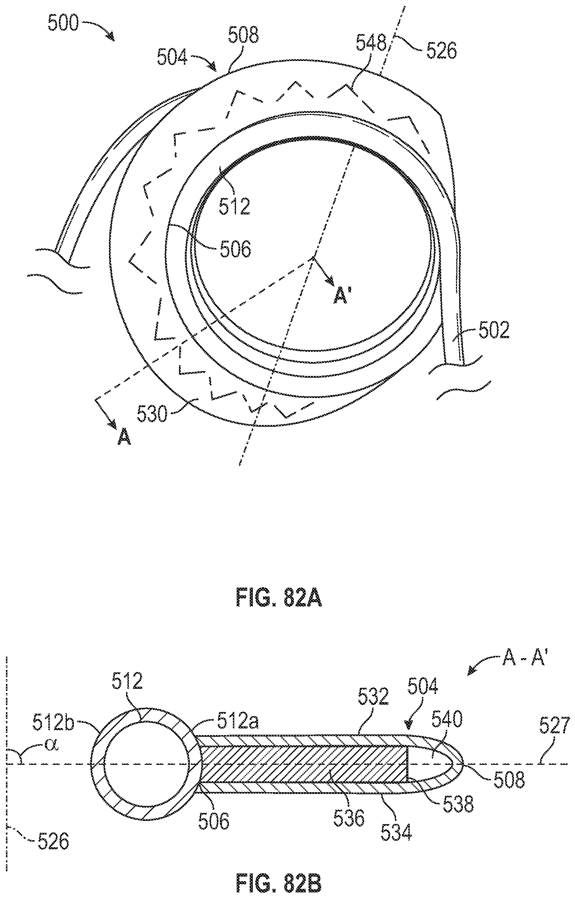

In any of the examples described herein, the inner edge 506 of the sealing member 504 can be fixedly attached (e.g., via sutures, glues, and/or any other attachment means) to a sealing segment 512 of the coil 502. In certain examples, the inner edge 506 can be stitched to the sealing segment 512 via a plurality of in-and-out sutures. As depicted in FIG. 82B, the outer surface of the sealing segment 512 can have an outer part 512a and an inner part 512b, where the inner part 512b is closer to a central longitudinal axis 526 of the docking device 500 than the outer part 512a. In certain examples, a portion of the sealing member 504 adjacent the inner edge 506 can wrap around at least a portion of the sealing segment 512. For example, the sealing member 504 can wrap around the inner part 512b. In certain examples, as depicted in FIG. 82B, the inner edge 506 of the sealing member 504 can be attached to and extend from the outer part 512a of the sealing segment 512. Because the sealing segment 512 is not wrapped around by the sealing member 504, such a configuration can reduce the overall profile of the sealing member 504 when it is folded within the dock sleeve (e.g., in the delivery configuration).

In certain examples, the axial length of the sealing segment 512 can correspond to about the same segment of the coil 102 covered by the guard member 104 in the deployed configuration. For example, when the coil 502 is in the deployed configuration, the sealing segment 512 can extend from one of the functional turns 514 (e.g., similar to 108p) of the coil 502 to a position that is adjacent to (and slightly distal to) an ascending portion 516 (similar to 110b) of the coil 502.

When the sealing member 504 is in the deployed configuration, the outer edge 508 can form a helical shape rotating about the central longitudinal axis 526 of the docking device 500 so that the proximal end 520 of the outer edge 508 is offset from the distal end 518 of the outer edge 508 along the central longitudinal axis 526.

In certain examples, when the sealing member 504 is in the deployed configuration, the outer edge 508 can extend circumferentially relative to the central longitudinal axis 526 from 180 degrees to 400 degrees, or from 210 degrees to 330 degrees, or from 250 degrees to 290 degrees, or from 260 degrees to 280 degrees. In one particular example, when the sealing member 504 is in the deployed configuration, the outer edge 508 can extend circumferentially 270 degrees relative to the central longitudinal axis 526. In other words, the sealing member 504 can extend circumferentially from about one half of a revolution (e.g., 180 degrees) around the central longitudinal axis 526 in some examples to more than a full revolution (e.g., 400 degrees) around the central longitudinal axis 526 in other examples, including various ranges in between. As used herein, a range (e.g., 180-400 degrees, from 180 degrees to 400 degrees, and between 180 degrees and 400 degrees) includes the endpoints of the range (e.g., 180 degrees and 400 degrees).

When the coil 502 is in the deployed configuration, similar to the outer edge 508, the sealing segment 512 of the coil can also form a helical shape rotating about the central longitudinal axis 526 of the docking device 500 such that a proximal end of the sealing segment 512 is offset relative to a distal end of the sealing segment 512 along the central longitudinal axis 526.

As described above, the sealing member 504 in the deployed configuration can be flat or substantially flat. Thus, the outer edge 508 of the sealing member 504 can be generally coplanar with the sealing segment 512 of the coil 502. The flat or substantial flat surface of the sealing member 504 in the deployed configuration can form a right angle or an oblique angle relative to the central longitudinal axis 526 of the docking device 500, when viewed from the top of coil 502 in FIG. 79A. To illustrate, FIG. 82B shows a cross-sectional view of the sealing member 504 along a radial axis 527. The radial axis 527 extends through the width of the sealing member 504 and intersects with the central longitudinal axis 526 for form an angle α. In certain examples, the sealing member 504 can be angled upwardly relative to the sealing segment 512. In other words, the angle α can be less than 90 degrees. For example, the angle α can be between 0 degree and 90 degrees (e.g., 85 degrees), or between 20 degrees and 80 degrees (e.g., 75 degrees), or between 30 degrees and 70 degrees (e.g., 60 degrees). In certain examples, the sealing member 504 can be perpendicular to the central longitudinal axis 526, i.e., the angle α can be 90 degrees. In other examples, the sealing member 504 can be angled downwardly relative to the sealing segment 512. In other words, the angle α can be greater than 90 degrees. For example, the angle α can be between 90 degree and 180 degrees (e.g., 160 degrees), or between 100 degrees and 150 degrees (e.g., 140 degrees), or between 110 degrees and 130 degrees (e.g., 120 degrees).

In any of the examples described above, example measurements of the sealing member 504 (e.g., the width W in FIG. 79B, the angle α in FIG. 82B, the flatness measure, etc.) in its deployed configuration can be obtained when the docking device 500 is deployed outside a patient's body. For example, the docking device 500 retained in a dock sleeve 222 can be deployed at a testing bench station by removing the dock sleeve 222 from the docking device 500, thereby allowing the sealing member 504 to radially extend to the deployed configuration.

As described more fully below, the sealing member 504 can comprise compliant materials. Thus, when deployed at the implantation site, the orientation (e.g., the radial axis 527) of sealing member 504 can adapt to the anatomy of the native tissue. For example, as noted above, the outer edge 508 in the extended position can contact or press against a native wall of a heart chamber. Thus, depending on the anatomy at the implantation site (e.g., the position and/or slope of the native wall contacting the outer edge 508 relative to the implanted docking device 500), the outer edge 508 can be positioned above or below the inner edge 506. As a result, the angle α measured at the implantation site (e.g., due to adaptation of the native anatomy) can be different from the angle α measured outside patient's body (e.g., in a testing bench station).

In certain examples, the docking device 500 can have a tubular member (similar to 112) surrounding at least a portion of the coil 502. For example, the tubular member can surround the sealing segment 512, and a proximal end 522 of the inner edge 506 can be fixedly attached to the tubular member (e.g., via stitching sutures, glues, etc.). In certain examples, the docking device 500 can have a retention element (similar to 114) surrounding at least a portion of the tubular member. For example, the retention element can surround a portion of the tubular member adjacent to a distal end 524 of the inner edge 506. Both the distal end 524 of the inner edge 506 and the distal end 518 of the outer edge 508 can be fixedly attached to the retention element (e.g., via sutures, adhesive, etc.).

### Exemplary Structure of the Foldable PVL Guard

In any of the examples described herein, the sealing member 504 can be assembled separately before being attached to the coil 502.

In certain examples, the sealing member 504 can have a spine 528 (also referred to as an "expansion member" or "support frame") extending along at least a portion of the outer edge 508 and a biocompatible cover 530 (also referred to as a "sealing portion" or a "sealing membrane") extending between the inner edge 506 and the outer edge 508. As noted above, the sealing member 504 in the deployed configuration can have a tapered shape. Thus, a proximal end portion of the cover 530 can have a larger radial width than a distal end portion of the cover 530. Generally, the spine 528 can be stiffer than the cover 530.

The spine 528 can be biased to the deployed configuration and can be moved (e.g., elastically deformed) to the delivery configuration. For example, the spine 528 can include a shape memory material, such as nickel titanium alloy (e.g., nitinol). During delivery, the spine 528 can be retained within the dock sleeve (i.e., the sealing member 504 is in the delivery configuration), extending along and adjacent to the sealing segment 512 of the coil. When the dock sleeve is removed (i.e., the sealing member 504 is in the deployed configuration), the spine 528 can return to its shape set position. In lieu of or in addition to biasing the spine, one or more other mechanisms (e.g., spring, etc.) can be used to move the spine 528 from the delivery position to the deployed position. In some examples, the spine 528 can include one or more alloys such as nitinol, cobalt chromium, and/or stainless steel. In some examples, the spine 528 can include one or more polymeric materials such as polyether ether ketone (PEEK) and/or polyethylene terephthalate (PET) and/or ePTFE/PTFE. In some examples, the spine 528 can include a suture (e.g., a braided surgical suture).

In any of the examples described herein, the cover 530 can include at least one layer of material configured to restrict or prevent blood from passing therethrough, thereby preventing or reducing paravalvular leakage when the sealing member 504 is in the deployed configuration. The cover 530 can include one or more of cloth, PEEK, ePTFE, PET, thermoplastic polyurethane (TPU), and foam. In certain examples, the cover 530 can be monolayer. In certain examples, the cover 530 can have a multi-layer structure, as described below.

In certain examples, the cover 530 can include at least two laminated layers (also referred to as "cover layers"), e.g., a top layer 532 and a bottom layer 534 (see, e.g., FIG. 82B). In certain examples, the cover 530 can include two cloth layers. In certain examples, the cover 530 can include one cloth layer and one layer comprising ePTFE. Respective inner edges of the layers can be fixedly attached to (e.g., via stitches, glue, thermal compression, etc.) to the sealing segment 512 of the coil 502, as noted above. Respective outer edges of the layers can be sealed.

In certain examples, a soldering iron can be used to seal the at least two layers at their respective outer edges to form the outer edge 508 of the sealing member 504. In certain examples, the at least two layers can be sealed along their respective outer edges using a plurality of in-and-out stitches 532, as illustrated in FIG. 81. In certain cases, after stitching two layers along a stitching line adjacent to their respective outer edges, the two layers can be flipped inside-out along the stitching line, which can form the outer edge 508 of the sealing member 504.

In certain examples, the cover 530 can include a third layer 536 inserted between the top and bottom layers 532, 534, as depicted in FIG. 82B. In certain examples, the third layer 536 can include a foam material. In certain examples, the third layer 536 can include TPU. In certain examples, the cover 530 can have a plurality of in-and-out stitches coupling the third layer 536 to the top and bottom layers 532, 534 along an outer edge 538 of the third layer 536. In certain examples, the cover 530 can have a plurality of stitches 548 running in a zigzag pattern (see, e.g., FIG. 82A) to couple the third layer 536 to the top and bottom layers 532, 534.

On one hand, the cover 530 described herein is configured to be sufficiently thin so that it can be folded within the dock sleeve. For example, the thickness of the cover can between 0.02 mm and 0.30 mm, or between 0.05 mm and 0.20 mm, or between 0.06 mm and 0.10 mm. On the other hand, the cover 530 is configured to have a sufficient density so that it will remain stable and not dislocated when deployed in the target location. For example, when the sealing member 504 is in the deployed configuration, the outer edge 508 can remain contact with the native wall and the cover 530 is configured to not move up and/or down with the blood flow, thus forming a stable seal between the docking device 500 and the native wall to reduce paravalvular leakage.

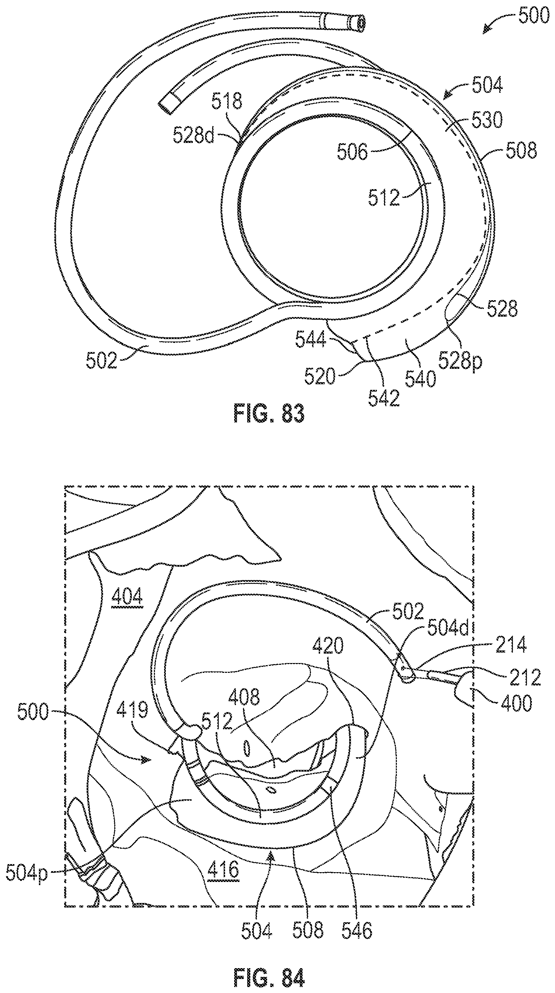

In certain examples, the cover 530 can have a pocket 540 extending along the outer edge 508 and configured to receive the spine 528. If the cover 530 has at least two layers, the pocket 540 can be created by stitching the cover 530 along a line 542 (see, e.g., FIG. 83) that is spaced apart from the outer edge 508. In certain cases (e.g., when the cover 530 has a monolayer structure), the cover 530 can be folded along the outer edge 508, and a seam can be added to the folded cover (e.g., via stitches, glue, thermal compression, etc.) to create the pocket 540.

The spine 528 can be inserted into the pocket 540. In certain examples, a distal end 528d of the spine 528 can be fixedly attached to a distal end portion (e.g., the distal end 518) of the outer edge 508. Thus, when the distal end 518 of the outer edge 508 is fixedly attached to the coil 502, the distal end 528d of the spine 528 can also be fixedly attached to the coil 502.

In certain examples, a proximal end 528p of the spine 528 can also be fixedly attached to a proximal end portion (e.g., the proximal end 520) of the outer edge 508 (see, e.g., FIG. 79A). In such circumstances, the spine 528 is not movable within the pocket 540 (since both the proximal end 528p and the distal end 528d of the spine are fixedly attached to the outer edge 508.

In other examples, the proximal end 528p of the spine 528 is a free end and can move along the outer edge 508 when the outer edge 508 moves between the folded position and the extended position (see, e.g., FIG. 83). In other words, the proximal end 528p of the spine 528 can move or slide within the pocket 540.

In certain examples, the pocket 540 can have a closed proximal end 544. The proximal end 544 of the pocket 540 can be closed or sealed by soldering, stitching, or other means. In certain examples, the proximal end 528p of the spine 528 can have an atraumatic shape configured not piercing through the closed proximal end 544 of the pocket 540. For example, as depicted in FIG. 83, the proximal end 528p of the spine 528 can form a curved loop. In certain examples, the proximal end 528p of the spine 528 can be configured to be retained within and not extend out of the pocket 540. For example, the proximal end 528p of the spine 528 can be spaced away from the proximal end 544 of the pocket 540 (whether the sealing member 504 is in the delivery configuration or deployed configuration) so that it does not apply pressure to the closed proximal end 544 of the pocket 540. In one particular example, when the sealing member 504 is in the deployed configuration, a distance between the proximal end 528p of the spine 528 and the proximal end 544 of the pocket 540 can range from about 10 mm to about 14 mm (e.g., about 12 mm).

### Exemplary Method of Deploying Foldable PVL Guard

The procedure for delivering the docking device 500 to an implantation site and implanting a prosthetic valve (such as the prosthetic valve 10 described above) within the docking device 500 can be generally similar to the procedure described above in reference to FIGS. 65-78, with the exceptions described below.

As noted above, after the functional turns of the docking device successfully wraps round the native leaflets and the chordae tendineae (see, e.g., FIGS. 68-69), the dock sleeve 222 can be retracted in a proximal direction until it is retracted back into the delivery sheath 204. FIG. 84 illustrates the fully deployed docking device 500. As shown, without being constrained by the dock sleeve 222, the sealing member 504 can extend radially outwardly from the coil 502, e.g., as the spine 528 moves from the biased position to the unbiased position. As described above, the release suture 214 can be cut so as to release the docking device 500 from the delivery apparatus 200.

Unlike FIGS. 70A-70C and 71 which show the radially expanded guard member 104 surrounding a portion of the coil 102, FIG. 84 shows that the sealing member 504 in its deployed configuration forms a flat or substantially flat surface extending radially outwardly from the coil 502. In addition, unlike the docking device 100 depicted in FIGS. 70B-70C, where repositioning the proximal end 105 of guard member 104 may be needed (because the proximal end portion 104p can axially move relative to the coil 102), no such repositioning step is needed for the docking device 500 (because the inner edge 506 of the sealing member 504 is fixedly attached to the coil 502).

As shown in FIG. 84, the distal portion 504d of the sealing member can be configured to extend to a location adjacent to the posteromedial commissure 420. In certain examples, the distal portion 504d of the sealing member can be configured to extend through the native mitral valve annulus 408 and into the left ventricle 414. The proximal portion 504p of the sealing member can be configured to be positioned adjacent to the anterolateral commissure 419 of the native valve. As described above, the outer edge 508 of the sealing member 504 can be configured to remain contact with the posterior wall 416 of the left atrium 404. Thus, the sealing member 504 can form a stable seal between the docking device 500 and the native wall of the left atrium to reduce paravalvular leakage.

Positioning of the sealing member 504 relative to the anatomy of the native mitral valve annulus 408 can be examined by visualizing the position of at least one radiopaque marker located on the docking device 500 under fluoroscopy. For example, FIG. 84 shows a radiopaque marker 546 located on the sealing segment 512 of the docking device 500. The radiopaque marker 546 can have a predetermined axial distance to the distal end 524 of the inner edge 506 of the sealing member 504. In the example depicted in FIG. 84, the radiopaque marker 546 is located slightly proximal to the posteromedial commissure 420.

After deploying the docking device 500, a prosthetic valve (e.g., 10) can be delivered into the left atrium 404, placed within the docking device 500, and then radially expanded, following similar steps described above in reference to FIGS. 72-77.

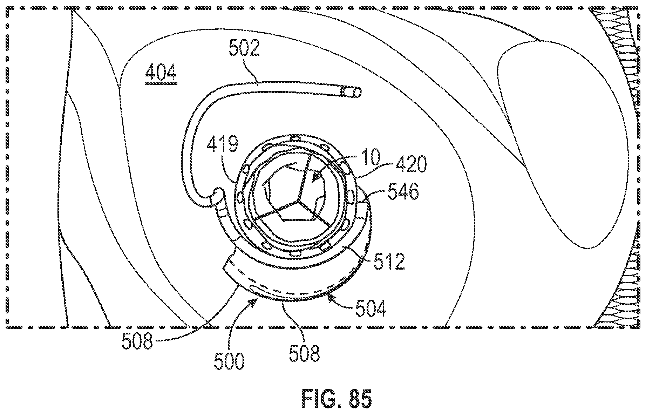

FIG. 85 illustrates the final disposition of the docking device 500 at the mitral valve and the prosthetic valve 10 received within the docking device 500. As noted above, radially expanding the prosthetic valve 10 within the docking device 500 can cause further radial expansion of the coil 502 as well as circumferential rotation of the functional turns and slight unwinding of the helical coil 502 (i.e., clocking). As a result, the sealing segment 512 of the coil 502 can rotate slightly. For example, FIG. 85 shows that the radiopaque marker 546 can move slightly distally (compared to FIG. 84) to the location corresponding to the posteromedial commissure 420. Thus, position of the radiopaque marker 546 relative to posteromedial commissure 420 can be used to confirm final disposition of the docking device 500 and proper expansion of the prosthetic valve 10.

As described above, the radial tension between the prosthetic valve 10 and the central region of the docking device 500 can securely hold the prosthetic valve 10 in place. In addition, the sealing member 504 can act as a seal between the docking device 500 and the native wall to prevent or reduce paravalvular leakage around the prosthetic valve 10.

In view of the many possible examples to which the principles of the disclosed technology may be applied, it should be recognized that the illustrated examples are only preferred examples of the technology and should not be taken as limiting the scope of the disclosure. Rather, the scope of the claimed subject matter is defined by the following claims.

## Claims

1. A docking device (100) for securing a prosthetic valve at a native valve, the docking device (100) comprising:
a coil (102) comprising a proximal end (102p), a distal end (102d) and a plurality of helical turns (108p; 108m; 108d) when deployed at the native valve;
an expandable member (116) extending radially outwardly from the coil (102), the expandable member (116) being movable between a radially-compressed/axially-elongated state and a radially-expanded/axially-foreshortened state; and
a cover member (118) surrounding an outer surface of the expandable member (116),
wherein both a distal end portion (118d) of the cover member (118) and a distal end portion (116d) of the expandable member (116) are fixedly coupled to the coil (102) via a distal suture (160), wherein the distal suture (160) comprises a plurality of knots (164; 168) and a plurality of wraps (166; 170),
wherein a proximal end portion (116p) of the expandable member (116) is fixedly coupled to a proximal end portion (118p) of the cover member (118), wherein the proximal end portion (116p) of the expandable member (116) and the proximal end portion (118p) of the cover member (118) are axially movable relative to the coil (102).

2. The docking device (100) of claim 1, wherein the proximal end portion (116p) of the expandable member (116) and the proximal end portion (118p) of the cover member (118) moves distally when the expandable member (116) changes from the radially-compressed/axially-elongated state to the radially-expanded/axially-foreshortened state; and/or wherein the cover member (118) is configured to engage with the prosthetic valve deployed within the docking device (100) so as to reduce paravalvular leakage between the docking device (100) and the cover member (118) when the expandable member (116) is in the radially-expanded/axially-foreshortened state.

3. The docking device (100) of any one of claims 1 to 2, wherein the coil (102) comprises a shape memory material; and/or wherein the expandable member (116) comprises a braided wire frame; in particular wherein the braided wire frame comprises a metal alloy with shape memory properties.

4. The docking device (100) of any one of claims 1 to 3, wherein the proximal end portion (116p) of the expandable member (116) tapers radially inwardly from a first diameter at a first location to a second diameter at the second location when the expandable member (116) is in the radially-expanded/axially-foreshortened state, wherein the first location is distal to the second location; and/or wherein the cover member (118) comprises a fabric layer.

5. The docking device (100) of any one of claims 1 to 4, wherein the proximal end portion (118p) of the cover member (118) comprises a fold (156) wrapping at a proximal end (116p) of the expandable member (116), wherein the proximal end (116p) of the expandable member (116) is a terminal end (132); and/or wherein the proximal end portion (118p) of the cover member (118) is connected to the expandable member (116) by a proximal suture (300).

6. The docking device (100) of any one of claims 1 to 5, further comprising one or more radiopaque markers (350) disposed along the coil (102) and configured for positioning the expandable member (116) relative to the coil (102); and/or further comprising a retention element (114) disposed radially between the coil (102) and the expandable member (116), wherein the retention element (114) is configured for retaining the position of the proximal end portion (116p) of the expandable member (116) relative to the coil (102).

7. The docking device (100) of any one of claims 1 to 6, further comprising a tubular member (112) surrounding at least a portion of the coil (102); and
a guard member (104) surrounding at least a portion of the tubular member (112), wherein the guard member (104) comprises the expandable member (116) and the cover member (118),
wherein a distal end portion (104d) of the guard member (104) is fixedly attached to the tubular member (112),
wherein a proximal end portion (104p) of the guard member (104) is movable relative to the tubular member (112) and the coil (102),
wherein the guard member (104) is movable between a radially compressed state and a radially expanded state,
wherein the proximal end portion (104p) of the guard member (104) is disposed closer to the proximal end (102p) of the coil (102) when the guard member (104) is in the radially compressed state than in the radially expanded state; in particular wherein in the radially expanded state, the guard member (104) is configured to reduce paravalvular leakage around the prosthetic valve.

8. The docking device (100) of claim 7, wherein the proximal end portion (104p) of the guard member (104) slides axially over the tubular member (112) and toward the distal end (102d) of the coil (102) when the guard member (104) moves from the radially compressed state to the radially expanded state; and/or wherein in the radially expanded state, the proximal end portion (104p) of the guard member (104) tapers radially outwardly from a first diameter at a first location to a second diameter at a second location, the second location being disposed distal to the first location.

9. The docking device (100) of any one of claims 7 to 8, wherein the proximal end portion (118p) of the cover member (118) is fixedly coupled to the proximal end (116p) of the expandable member (116); in particular wherein the proximal end (116p) of the expandable member (116) has a smaller diameter than a body portion of the expandable member (116) when the guard member (104) is in the radially expanded state.

10. The docking device (100) of claim 9, wherein the proximal end portion (118p) of the cover member (118) comprises a fold (156) abutting the proximal end (116p) of the expandable member (116).

11. The docking device (100) of any one of claims 7 to 10, wherein a distal end portion (118d) of the cover member (118) is fixedly coupled to a distal end portion (116d) of the expandable member (116) and the tubular member (112); in particular, wherein the tubular member (112) extends through the distal end portion (116d) of the expandable member (116).

12. The docking device (100) of claim 11, wherein the distal end portion (118d) of the cover member (118) is connected to the distal end portion (116d) of the expandable member (116) and the tubular member (112) by a second suture (160) comprising a plurality of wraps (166; 170) and a plurality of knots (164; 168).

13. The docking device (100) of any one of claims 7 to 12, wherein the guard member (104) has an elongation ration ranging from 1.2 to 1.5.

14. The docking device (100) of anyone of claims 7 to 13, wherein the tubular member (112) comprises at least a first seating marker (121p) and a second seating marker (121d), wherein the first seating marker (121p) is positioned proximal relative to the second seating marker (121d);
wherein when deployed at the native valve, the proximal end portion (104p) of the guard member (104) is positioned between the first seating marker (121p) and the second seating marker (121d).

15. A cover assembly (120) for a docking device (100) configured to receive a prosthetic valve, the cover assembly comprising:
a tubular member (112) configured to cover at least a portion of a helical coil (102) of the docking device (100);
an expandable member (116) surrounding at least a portion of the tubular member (112), the expandable member (116) being changeable between a radially expanded state and a radially compressed state; and
a cover member (118) surrounding an outer surface of the expandable member (116),
wherein a distal end portion (118d) of the cover member (118) is fixedly coupled to a distal end portion (116d) of the expandable member (116) and the tubular member (112),
wherein a proximal end portion (118p) of the cover member (118) comprises a fold (156) covering a proximal end portion (116p) of the expandable member (116),
wherein the proximal end portion (116p) of the expandable member (116) is slidably movable relative to the tubular member (112) and the helical coil (102) of the docking device (100).

16. The cover assembly (120) of claim 15, wherein the expandable member (116) is axially foreshortened when it changes from the radially compressed state to the radially expanded state; in particular wherein the proximal end portion (116p) of the expandable member (116) moves distally relative to the tubular member (112) when the expandable member (116) changes from the radially compressed state to the radially expanded state.

17. The cover assembly (120) of any one of claims 15 to 16, wherein the cover member (118) is configured to engage with the prosthetic valve deployed within the docking device (100) so as to reduce paravalvular leakage between the prosthetic valve and the cover member (118) when the expandable member (116) is in the radially expanded state.

18. The cover assembly (120) of any one of claims 15 to 17, wherein the expandable member (116) comprises a braided Nitinol frame; and/or wherein the tubular member (112) comprises ePTFE; and/or wherein the cover member (118) comprises PET.

## Patentansprüche

1. Andockvorrichtung (100) zur Befestigung einer prothetischen Klappe an einer natürlichen Klappe, wobei die Andockvorrichtung (100) umfasst:
eine Spirale (102), die ein proximales Ende (102p), ein distales Ende (102d) und eine Vielzahl von helixförmigen Windungen (108p; 108; 108d) umfasst, wenn sie an der natürlichen Klappe eingesetzt ist;
ein expandierbares Element (116), das sich radial nach außen von der Spirale (102) erstreckt, wobei das expandierbare Element (116) zwischen einem radial komprimierten/axial gestreckten Zustand und einem radial expandierten/axial verkürzten Zustand beweglich ist; und
ein Abdeckelement (118), das die äußere Oberfläche des expandierbaren Elements (116) umgibt,
wobei sowohl ein distaler Endabschnitt (118d) des Abdeckelements (118) und ein distaler Endabschnitt (116d) des expandierbaren Elements (116) über eine distale Naht (160) fest mit der Spirale (102) verbunden sind, wobei die distale Naht (160) eine Vielzahl von Knoten (164; 168) und eine Vielzahl von Windungen (166; 170) umfasst,
wobei ein proximaler Endabschnitt (116p) des expandierbaren Elements (116) fest mit einem proximalen Endabschnitt (118p) des Abdeckelements (118) verbunden ist, wobei der proximale Endabschnitt (116p) des expandierbaren Elements (116) und der proximale Endabschnitt (118p) des Abdeckelements (118) axial relativ zu der Spirale (102) beweglich sind.

2. Andockvorrichtung (100) gemäß Anspruch 1, wobei der proximale Endabschnitt (116p) des expandierbaren Elements (116) und der proximale Endabschnitt (118p) des Abdeckelements (118) sich in distaler Richtung bewegen, wenn das expandierbare Element (116) von dem radial komprimierten/axial gestreckten Zustand in den radial expandierten/axial verkürzten Zustand wechselt; und/oder wobei das Abdeckelement (118) dazu ausgelegt ist, mit der prothetischen Klappe, die in der Andockvorrichtung (100) eingesetzt ist, in Eingriff zu kommen, um eine paravalvuläre Leckage zwischen der Andockvorrichtung (100) und dem Abdeckelement (118) zu verringern, wenn sich das expandierbare Element (116) im radial expandierten/axial verkürzten Zustand befindet.

3. Andockvorrichtung (100) gemäß einem der Ansprüche 1 oder 2, wobei die Spirale (102) ein Formgedächtnismaterial umfasst; und/oder wobei das expandierbare Element (116) einen geflochtenen Drahtrahmen umfasst; insbesondere wobei der geflochtene Drahtrahmen eine Metalllegierung mit Formgedächtniseigenschaften umfasst.

4. Andockvorrichtung (100) gemäß einem der Ansprüche 1 bis 3, wobei sich der proximale Endabschnitt (116p) des expandierbaren Elements (116) radial nach innen von einem ersten Durchmesser an einer ersten Stelle zu einem zweiten Durchmesser an der zweiten Stelle verjüngt, wenn sich das expandierbare Element (116) im radial expandierten/ axial verkürzten Zustand befindet, wobei die erste Stelle distal zur zweiten Stelle liegt; und/oder wobei das Abdeckelement (118) eine Gewebeschicht umfasst.

5. Andockvorrichtung (100) gemäß einem der Ansprüche 1 bis 4, wobei der proximale Endabschnitt (118p) des Abdeckelements (118) eine Falte (156) aufweist, die sich an einem proximalen Ende (116p) des expandierbaren Elements (116) umschlägt, wobei das proximale Ende (116p) des expandierbaren Elements (116) ein terminales Ende (132) ist; und/oder wobei der proximale Endabschnitt (118) des Abdeckelements (118) mit dem expandierbaren Element (116) über eine proximale Naht (300) verbunden ist.

6. Andockvorrichtung (100) gemäß einem der Ansprüche 1 bis 5, ferner umfassend eine oder mehrere röntgendichte Markierungen (350), die entlang der Spirale (102) angeordnet und dazu ausgelegt sind, das expandierbare Element (116) relativ zu der Spirale (102) zu positionieren; und/oder ferner umfassend ein Halteelement (114), das radial zwischen der Spirale (102) und dem expandierbaren Element (116) angeordnet ist, wobei das Halteelement (114) dazu ausgelegt ist, die Position des proximalen Endabschnitts (116p) des expandierbaren Elements (116) relativ zu der Spirale (102) zu halten.

7. Andockvorrichtung (100) gemäß einem der Ansprüche 1 bis 6, ferner umfassend
ein röhrenförmiges Element (112), dass zumindest einen Abschnitt der Spirale (102) umgibt; und
ein Schutzelement (104), das zumindest einen Abschnitt des röhrenförmigen Elements (112) umgibt, wobei das Schutzelement (104) das expandierbare Element (116) und das Abdeckelement (118) umfasst,
wobei ein distaler Endabschnitt (104d) des Schutzelements (104) fest an dem röhrenförmigen Element (112) befestigt ist,
wobei ein proximaler Endabschnitt (104d) des Schutzelements (104) beweglich relativ zu dem röhrenförmigen Element (112) und der Spirale (102) ist,
wobei das Schutzelement (104) zwischen einem radial komprimierten Zustand und einem radial expandierten Zustand bewegbar ist,
wobei der proximale Endabschnitt (104p) des Schutzelements (104) näher zum proximalen Ende (102p) der Spirale (102) angeordnet ist, wenn das Schutzelement (104) sich im radial komprimierten Zustand befindet als wenn es sich im radial expandierten Zustand befindet; insbesondere wobei das Schutzelement (104) im radial expandierten Zustand dazu ausgelegt ist, paravalvuläre Leckagen um die prothetische Klappe herum zu reduzieren.

8. Andockvorrichtung (100) gemäß Anspruch 7, wobei der proximale Endabschnitt (104p) des Schutzelements (104) axial über das röhrenförmige Element (112) und in Richtung des distalen Endes (102d) der Spirale (102) gleitet, wenn sich das Schutzelement (104) von dem radial komprimierten Zustand in den radial expandierten Zustand bewegt; und/oder wobei sich der proximale Endabschnitt (104p) des Schutzelements (104) im radial expandierten Zustand radial nach außen von einem ersten Durchmesser an einer ersten Stelle zu einem zweiten Durchmesser an einer zweiten Stelle verjüngt, wobei die zweite Stelle distal von der ersten Stelle angeordnet ist.

9. Andockvorrichtung (100) gemäß einem der Ansprüche 7 bis 8, wobei der proximale Endabschnitt (118p) des Abdeckelements (118) fest mit dem proximalen Endabschnitt (116p) des expandierbaren Elements (116) verbunden ist; insbesondere wobei der proximale Endabschnitt (116p) des expandierbaren Elements (116) einen kleineren Durchmesser aufweist als ein Körperabschnitt des expandierbaren Elements (116), wenn das Schuztelement (104) sich im radial expandierten Zustand befindet.

10. Andockvorrichtung (100) gemäß Anspruch 9, wobei der proximale Endabschnitt (118p) des Abdeckelements (118) eine Falte (156) aufweist, die an dem proximalen Ende (116p) des expandierbaren Elements (116) anliegt.

11. Andockvorrichtung (100) gemäß einem der Ansprüche 7 bis 10, wobei ein distaler Endabschnitt (118d) des Abdeckelements (118) fest mit dem distalen Endabschnitt (116d) des expandierbaren Elements (116) und dem röhrenförmigen Element (112) verbunden ist; insbesondere wobei sich das röhrenförmige Element (112) durch den distalen Endabschnitt (116d) des expandierbaren Elements (116) erstreckt.

12. Andockvorrichtung (100) gemäß Anspruch 11, wobei der distale Endabschnitt (118d) des Abdeckelements (118) mit dem distalen Endabschnitt (116d) des expandierbaren Elements (116) und dem röhrenförmigen Element (112) durch eine zweite Naht (160) verbunden ist, die eine Vielzahl von Windungen (166; 170) und eine Vielzahl von Knoten (164; 168) aufweist.

13. Andockvorrichtung (100) gemäß einem der Ansprüche 7 bis 13, wobei das Schutzelement (104) ein Dehnungsverhältnis im Bereich von 1,2 bis 1,5 aufweist.

14. Andockvorrichtung (100) gemäß einem der Ansprüche 7 bis 13, wobei das röhrenförmige Element (112) zumindest eine erste Positionierungsmarkierung (121p) und eine zweite Positionierungsmarkierung (121d) aufweist, wobei die erste Positionierungsmarkierung (121p) proximal relativ zu der zweiten Positionierungsmarkierung (121d) angeordnet ist;
wobei, wenn an der nativen Klappe eingesetzt, der proximale Endabschnitt (104p) des Schutzelements (104) zwischen der ersten Positionierungsmarkierung (121p) und der zweiten Positionierungsmarkierung (121d) angeordnet ist.

15. Abdeckvorrichtung (120) für eine Andockvorrichtung (100), die dazu ausgelegt ist eine prothetische Klappe aufzunehmen, wobei die Abdeckvorrichtung (120) umfasst:
ein röhrenförmiges Element (112) dazu ausgelegt, zumindest einen Abschnitt der helixförmigen Spirale (102) der Andockvorrichtung (100) zu abzudecken;
ein expandierbares Element (116), das zumindest einen Abschnitt des röhrenförmigen Elements (112) umgibt, wobei das expandierbare Element (116) zwischen einem radial expandierten und einem radial komprimierten Zustand veränderbar ist; und
ein Abdeckelement (118), das eine äußere Oberfläche des expandierbaren Elements (116) umgibt,
wobei ein distaler Endabschnitt (118d) des Abdeckelements (118) fest mit einem distalen Endabschnitt (116d) des expandierbaren Elements (116) und dem röhrenförmigen Element (112) verbunden ist,
wobei ein proximaler Endabschnitt (118p) des Abdeckelements (118) eine Falte (156) umfasst, die einen proximalen Endabschnitt (116p) des expandierbaren Elements (116) abdeckt,
wobei der proximale Endabschnitt (116p) des expandierbaren Elements (116) relativ zu dem röhrenförmigen Element (112) und der helixförmigen Spirale (102) der Andockvorrichtung (100) verschiebbar ist.

16. Abdeckvorrichtung (120) gemäß Anspruch 15, wobei das expandierbare Element (116) axial verkürzt ist, wenn es von dem radial komprimierten Zustand in den radial expandierten Zustand wechselt; insbesondere wobei sich der proximale Endabschnitt (116p) des expandierbaren Elements (116) distal relativ zu dem röhrenförmigen Element (112) bewegt, wenn das expandierbare Element (116) von dem radial komprimierten Zustand zu dem radial expandierten Zustand wechselt.

17. Abdeckvorrichtung (120) gemäß einem der Ansprüche 15 bis 16, wobei das Abdeckelement (118) dazu ausgelegt ist, mit der in der Andockvorrichtung (100) eingesetzten prothetischen Klappe in Eingriff zu kommen, um paravalvuläre Leckage zwischen der prothetischen Klappe und dem Abdeckelement (118) zu reduzieren, wenn sich das expandierbare Element (116) im radial expandierten Zustand befindet.

18. Abdeckvorrichtung (120) gemäß einem der Ansprüche 15 bis 17, wobei das expandierbare Element (116) einen geflochtenen Nitinolrahmen umfasst; und/oder wobei das röhrenförmige Element (112) ePTFE umfasst; und/oder wobei das Abdeckelement (118) PET umfasst.

## Revendications

1. Dispositif d'ancrage (100) destiné à fixer de manière sûre une valvule prothétique au niveau d'une valvule native, le dispositif d'ancrage (100) comprenant :
une bobine (102) comprenant une extrémité proximale (102p), une extrémité distale (102d) et une pluralité de spires hélicoïdales (108p ; 108m ; 108d) lorsqu'elle est déployée au niveau de la valvule native ;
un élément extensible (116) s'étendant radialement vers l'extérieur à partir de la bobine (102), l'élément extensible (116) étant mobile entre un état radialement comprimé/axialement allongé et un état radialement étendu/axialement raccourci ; et
un élément de recouvrement (118) entourant une surface externe de l'élément extensible (116),
à la fois une partie d'extrémité distale (118d) de l'élément de recouvrement (118) et une partie d'extrémité distale (116d) de l'élément extensible (116) étant accouplées à demeure à la bobine (102) par l'intermédiaire d'une suture distale (160), la suture distale (160) comprenant une pluralité de noeuds (164 ; 168) et une pluralité d'enroulements (166 ; 170),
une partie d'extrémité proximale (116p) de l'élément extensible (116) étant accouplée à demeure à une partie d'extrémité proximale (118p) de l'élément de recouvrement (118), la partie d'extrémité proximale (116p) de l'élément extensible (116) et la partie d'extrémité proximale (118p) de l'élément de recouvrement (118) étant axialement mobiles par rapport à la bobine (102).

2. Dispositif d'ancrage (100) selon la revendication 1, la partie d'extrémité proximale (116p) de l'élément extensible (116) et la partie d'extrémité proximale (118p) de l'élément de recouvrement (118) se déplaçant de manière distale lorsque l'élément extensible (116) passe de l'état radialement comprimé/axialement allongé vers l'état radialement étendu/axialement raccourci ; et/ou l'élément de recouvrement (118) étant conçu pour venir en prise avec la valvule prothétique déployée à l'intérieur du dispositif d'ancrage (100) de façon à réduire une fuite paravalvulaire entre le dispositif d'ancrage (100) et l'élément de recouvrement (118) lorsque l'élément extensible (116) est dans l'état radialement étendu/axialement raccourci.

3. Dispositif d'ancrage (100) selon l'une quelconque des revendications 1 à 2, la bobine (102) comprenant un matériau à mémoire de forme ; et/ou l'élément extensible (116) comprenant un cadre de fil tressé ; en particulier le cadre de fil tressé comprenant un alliage métallique présentant des propriétés de mémoire de forme.

4. Dispositif d'ancrage (100) selon l'une quelconque des revendications 1 à 3, la partie d'extrémité proximale (116p) de l'élément extensible (116) se rétrécissant radialement vers l'intérieur à partir d'un premier diamètre à un premier emplacement à un second diamètre au second emplacement lorsque l'élément extensible (116) est dans l'état radialement étendu/axialement raccourci, le premier emplacement étant distal par rapport au second emplacement ; et/ou l'élément de recouvrement (118) comprenant une couche de tissu.

5. Dispositif d'ancrage (100) selon l'une quelconque des revendications 1 à 4, la partie d'extrémité proximale (118p) de l'élément de recouvrement (118) comprenant un pli (156) s'enroulant au niveau d'une extrémité proximale (116p) de l'élément extensible (116), l'extrémité proximale (116p) de l'élément extensible (116) étant une extrémité terminale (132) ; et/ou la partie d'extrémité proximale (118p) de l'élément de recouvrement (118) étant reliée à l'élément extensible (116) par une suture proximale (300).

6. Dispositif d'ancrage (100) selon l'une quelconque des revendications 1 à 5, comprenant en outre un ou plusieurs marqueurs radio-opaques (350) disposés le long de la bobine (102) et conçus pour positionner l'élément extensible (116) par rapport à la bobine (102) ; et/ou comprenant en outre un élément de retenue (114) disposé radialement entre la bobine (102) et l'élément extensible (116), l'élément de retenue (114) étant conçu pour retenir la position de la partie d'extrémité proximale (116p) de l'élément extensible (116) par rapport à la bobine (102).

7. Dispositif d'ancrage (100) selon l'une quelconque des revendications 1 à 6, comprenant en outre
un élément tubulaire (112) entourant au moins une partie de la bobine (102) ; et
un élément de protection (104) entourant au moins une partie de l'élément tubulaire (112), l'élément de protection (104) comprenant l'élément extensible (116) et l'élément de recouvrement (118),
une partie d'extrémité distale (104d) de l'élément de protection (104) étant fixée à demeure à l'élément tubulaire (112),
une partie d'extrémité proximale (1 04p) de l'élément de protection (104) étant mobile par rapport à l'élément tubulaire (112) et à la bobine (102),
l'élément de protection (104) étant mobile entre un état radialement comprimé et un état radialement étendu,
la partie d'extrémité proximale (104p) de l'élément de protection (104) étant disposée plus près de l'extrémité proximale (102p) de la bobine (102) lorsque l'élément de protection (104) est dans l'état radialement comprimé qu'elle ne l'est dans l'état radialement étendu de l'élément de protection ; en particulier, dans l'état radialement étendu, l'élément de protection (104) étant conçu pour réduire une fuite paravalvulaire autour de la valvule prothétique.

8. Dispositif d'ancrage (100) selon la revendication 7, la partie d'extrémité proximale (104p) de l'élément de protection (104) coulissant axialement sur l'élément tubulaire (112) et vers l'extrémité distale (102d) de la bobine (102) lorsque l'élément de protection (104) se déplace à partir de l'état radialement comprimé vers l'état radialement étendu ; et/ou dans l'état radialement étendu, la partie d'extrémité proximale (104p) de l'élément de protection (104) se rétrécissant radialement vers l'extérieur à partir d'un premier diamètre à un premier emplacement à un second diamètre à un second emplacement, le second emplacement étant disposé de manière distale par rapport au premier emplacement.

9. Dispositif d'ancrage (100) selon l'une quelconque des revendications 7 à 8, la partie d'extrémité proximale (118p) de l'élément de recouvrement (118) étant accouplée à demeure à l'extrémité proximale (116p) de l'élément extensible (116) ; en particulier l'extrémité proximale (116p) de l'élément extensible (116) présentant un diamètre plus petit que celui d'une partie de corps de l'élément extensible (116) lorsque l'élément de protection (104) est dans l'état radialement étendu.

10. Dispositif d'ancrage (100) selon la revendication 9, la partie d'extrémité proximale (118p) de l'élément de recouvrement (118) comprenant un pli (156) venant en butée contre l'extrémité proximale (116p) de l'élément extensible (116).

11. Dispositif d'ancrage (100) selon l'une quelconque des revendications 7 à 10, une partie d'extrémité distale (118d) de l'élément de recouvrement (118) étant accouplée à demeure à une partie d'extrémité distale (116d) de l'élément extensible (116) et à l'élément tubulaire (112) ; en particulier, l'élément tubulaire (112) s'étendant à travers la partie d'extrémité distale (116d) de l'élément extensible (116).

12. Dispositif d'ancrage (100) selon la revendication 11, la partie d'extrémité distale (118d) de l'élément de recouvrement (118) étant reliée à la partie d'extrémité distale (116d) de l'élément extensible (116) et à l'élément tubulaire (112) par une seconde suture (160) comprenant une pluralité d'enroulements (166 ; 170) et une pluralité de noeuds (164 ; 168).

13. Dispositif d'ancrage (100) selon l'une quelconque des revendications 7 à 12, l'élément de protection (104) présentant un rapport d'allongement situé dans la plage de 1,2 à 1,5.

14. Dispositif d'ancrage (100) selon l'une quelconque des revendications 7 à 13, l'élément tubulaire (112) comprenant au moins un premier marqueur de placement (121p) et un second marqueur de placement (121d), le premier marqueur de placement (121p) étant positionné à proximité du second marqueur de placement (121d) ;
dans lequel, lors du déploiement au niveau de la valvule native, la partie d'extrémité proximale (104p) de l'élément de protection (104) est positionnée entre le premier marqueur de placement (121p) et le second marqueur de placement (121d).

15. Ensemble de recouvrement (120) pour un dispositif d'ancrage (100) conçu pour recevoir une valvule prothétique, l'ensemble de recouvrement comprenant :
un élément tubulaire (112) conçu pour recouvrir au moins une partie d'une bobine hélicoïdale (102) du dispositif d'ancrage (100) ;
un élément extensible (116) entourant au moins une partie de l'élément tubulaire (112), l'élément extensible (116) pouvant passer d'un état radialement étendu vers un état radialement comprimé ; et
un élément de recouvrement (118) entourant une surface externe de l'élément extensible (116),
une partie d'extrémité distale (118d) de l'élément de recouvrement (118) étant accouplée à demeure à une partie d'extrémité distale (116d) de l'élément extensible (116) et à l'élément tubulaire (112),
une partie d'extrémité proximale (118p) de l'élément de recouvrement (118) comprenant un pli (156) recouvrant une partie d'extrémité proximale (116p) de l'élément extensible (116),
la partie d'extrémité proximale (116p) de l'élément extensible (116) étant mobile de manière coulissante par rapport à l'élément tubulaire (112) et à la bobine hélicoïdale (102) du dispositif d'ancrage (100).

16. Ensemble de recouvrement (120) selon la revendication 15, l'élément extensible (116) étant raccourci axialement lorsqu'il passe de l'état radialement comprimé vers l'état radialement étendu ; en particulier la partie d'extrémité proximale (116p) de l'élément extensible (116) se déplaçant de manière distale par rapport à l'élément tubulaire (112) lorsque l'élément extensible (116) passe de l'état radialement comprimé vers l'état radialement étendu.

17. Ensemble de recouvrement (120) selon l'une quelconque des revendications 15 à 16, l'élément de recouvrement (118) étant conçu pour venir en prise avec la valvule prothétique déployée à l'intérieur du dispositif d'ancrage (100) de façon à réduire une fuite paravalvulaire entre la valvule prothétique et l'élément de recouvrement (118) lorsque l'élément extensible (116) est dans l'état radialement étendu.

18. Ensemble de recouvrement (120) selon l'une quelconque des revendications 15 à 17, l'élément extensible (116) comprenant un cadre en Nitinol tressé ; et/ou l'élément tubulaire (112) comprenant de l'ePTFE ; et/ou l'élément de recouvrement (118) comprenant du PET.
